(19) Europäisches Patentamt
European Patent Office
Office européen des brevets

(11) **EP 2 702 150 B1**

(12) **EUROPEAN PATENT SPECIFICATION**

(45) Date of publication and mention
of the grant of the patent:
**10.02.2016 Bulletin 2016/06**

(51) Int Cl.:
*C12N 9/20* (2006.01)     *C12N 1/15* (2006.01)
*C12N 1/21* (2006.01)     *C12P 7/64* (2006.01)
*C11B 3/00* (2006.01)

(21) Application number: **12718322.6**

(22) Date of filing: **27.04.2012**

(86) International application number:
**PCT/GB2012/050936**

(87) International publication number:
**WO 2012/146935 (01.11.2012 Gazette 2012/44)**

(54) **LIPASE VARIANTS HAVING INCREASED ENZYME SPECIFICITY OR ENHANCED TRANS-SELECTIVITY, AND METHODS OF USE**

LIPASEVARIANTEN MIT ERHÖHTER ENZYMSPEZIFITÄT ODER VERBESSERTER TRANSSELEKTIVITÄT UND VERWENDUNGSVERFAHREN

VARIANTS DE LIPASE AYANT UNE SPÉCIFICITÉ ENZYMATIQUE ACCRUE OU UNE TRANS-SÉLECTIVITÉ AMÉLIORÉE, ET LEUR PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.04.2011 US 201161480862 P**
**17.11.2011 US 201161561067 P**

(43) Date of publication of application:
**05.03.2014 Bulletin 2014/10**

(73) Proprietor: **Frito-Lay Trading Company GmbH**
**1211 Geneva 28 (CH)**

(72) Inventors:
• **BORNSCHEUER, Uwe**
**17489 Greifswald (DE)**
• **BRUNDIEK, Henrike**
**49525 Lengerich (DE)**
• **EVITT, Andrew**
**London, Greater London NW2 4EN (GB)**
• **SAß, Stefan**
**17489 Griefswald (DE)**
• **BÖNISCH, Friedericke**
**85354 Freising (DE)**
• **KOURIST, Robert**
**58452 Witten (DE)**

(74) Representative: **Jenkins, Peter David**
**Page White & Farrer**
**Bedford House**
**John Street**
**London WC1N 2BF (GB)**

(56) References cited:
**WO-A1-94/01541     WO-A1-2008/040738**

• **SANDSTROM ANDERS G ET AL: "Directed evolution of Candida antarctica lipase A using an episomaly replicating yeast plasmid dagger", PROTEIN ENGINEERING DESIGN & SELECTION, vol. 22, no. 7, July 2009 (2009-07), pages 413-420, XP002681389, ISSN: 1741-0126**
• **WOOD R ET AL: "Effect of butter, mono- and polyunsaturated fatty acid-enriched butter, trans fatty acid margarine, and zero trans fatty acid margarine on serum lipids and lipoproteins in healthy men", JOURNAL OF LIPID RESEARCH, AMERICAN SOCIETY FOR BIOCHEMISTRY AND MOLECULAR BIOLOGY, INC, US, vol. 34, no. 1, 1 January 1993 (1993-01-01), pages 1-11, XP002436033, ISSN: 0022-2275**

- KASRAYAN A ET AL: "Prediction of the Candida antarctica Lipase A protein structure by comparative modeling and site-directed mutagenisis", CHEMBIOCHEM - A EUROPEAN JOURNAL OF CHEMICAL BIOLOGY, WILEY VCH, WEINHEIM, DE, vol. 8, 13 July 2007 (2007-07-13), pages 1409-1415, XP002446023, ISSN: 1439-4227, DOI: 10.1002/CBIC.200700179
- DOMINGUEZ DE MARIA P ET AL: "Biotechnological applications of Candida antarctica lipase A: State-of-the-art", JOURNAL OF MOLECULAR CATALYSIS. B, ENZYMATIC, ELSEVIER, AMSTERDAM, NL, vol. 37, no. 1-6, 1 December 2005 (2005-12-01), pages 36-46, XP027658783, ISSN: 1381-1177 [retrieved on 2005-12-01]
- ROBERT KOURIST ET AL: "Protein engineering and discovery of lipases", EUROPEAN JOURNAL OF LIPID SCIENCE AND TECHNOLOGY, vol. 112, no. 1, 1 January 2010 (2010-01-01), pages 64-74, XP55034672, ISSN: 1438-7697, DOI: 10.1002/ejlt.200900143
- HENRIKE B. BRUNDIEK ET AL: "Creation of a Lipase Highly Selective for trans Fatty Acids by Protein Engineering", ANGEWANDTE CHEMIE INTERNATIONAL EDITION, vol. 51, no. 2, 23 November 2011 (2011-11-23), pages 412-414, XP55034576, ISSN: 1433-7851, DOI: 10.1002/anie.201106126
- DATABASE UNIPROT [Online] 01 October 2002 'SubName: Full=Lipase {ECO:0000313|EMBL: BAB91331.1}; Flags: Precursor;' Retrieved from EBI, accession no. UNIPROT:Q8NIP2 Database accession no. Q8NIP2

**Description**

**BACKGROUND OF THE INVENTION**

**CROSS-REFERENCE TO RELATED APPLICATIONS:**

The present application claims priority to U.S. Provisional Patent Appl. Nos. 61/561,067 and 61/480,862, filed November 17, 2011 and April 29, 2011, respectively.

**FIELD OF THE INVENTION**

The present invention relates generally to the generation and production of novel lipase variants. More particularly, it concerns lipase compositions, genetic constructs encoding the lipase variants, recombinant host cells capable of expressing the variants, and methods of using such compositions in processes for the catalysis of lipids and fatty acid. In certain embodiments, lipase variants having increased affinity for medium- and long-chain fatty acid moieties are provided. Also disclosed are lipase variants having enhanced *trans*-selectivity as compared to the parent lipases from which the variants are derived. In certain embodiments, lipase variants derived from *Pseudozyma* (formerly *Candida*) *antarctica* lipase A (CAL-A) have been engineered, and expressed in selected bacterial and fungal host cells. The invention also relates to methods for the generation and production of lipase variant enzymes as catalysts for altering the fatty acid composition, or reducing the level of *trans*-fatty acids, of lipids or lipid-containing compositions, foodstuffs, cooking oils, and the like.

**DESCRIPTION OF RELATED ART**

Lipases (triacylglycerol acylhydrolases; EC 3.1.1.3) consist of a genetically diverse and distinctive grouping of water-soluble hydrolytic enzymes that typically act on the ester bonds of lipid substrates. Lipids can include fats, waxes, sterols, fat-soluble vitamins, monoglycerides, diglycerides, fatty acyls, polyketides, and fatty acids, and exist as a number of variations containing different additional chemical structures such as phospholipids, glycerolipids, glycerophospholipids, sphingolipids, sterol lipids, prenol lipids, saccharolipids, *etc.* Lipases have also been used in ester and amide synthesis, kinetic resolutions or asymmetric synthesis to obtain optically pure compounds, and lipid modifications (Bornscheuer and Kazlauskas, 2005). Lipases play an essential role in: (1) the metabolism of dietary lipids, (2) injury and inflammation, (3) cell biology, (4) fermentation, (5) biocatalysis, (6) vitamin absorption, (7) laundering, (8) synthesis of pharmaceuticals and many other biological and chemical processes. Such wide and varying roles have been attributed to lipase stability in organic solvents, high specificity, high enantio-selectivity and regio-selectivity, and a general lack of a need of cofactors for their action. Genes encoding lipases have been found in most, if not all, types of organisms.

Typically, the tertiary structure of lipases includes the alpha/beta ($\alpha/\beta$) hydrolase fold pattern (Ollis *et al.,* 1992), also common in peptidases and esterases (Holmquist, 2000), and can be composed of a core of up to eight beta strands, connected and surrounded by alpha helices. The active sites of lipases are usually formed by at least a catalytic triad consisting of a serine residue as the nucleophile, a histidine residue, and an aspartic or glutamic acid residue. The active site residues are located in a hydrophobic pocket that is covered by a flap or lid structure, usually composed of amphiphilic alpha helices (Anthonsen *et al.,* 1995).

Lipases typically act at the interface generated by a hydrophobic lipid substrate in a hydrophilic aqueous medium. There are typically four basic steps in lipase hydrolysis and/or alcoholysis *(i.e.,* ethanolysis), which involves a conformational change of the lipase itself. First, the lipase is adsorbed and activated by the opening of the hydrophobic pocket by displacement of the lid structure, by the so-called interfacial activation. Once the pocket is opened, the ester bond of the lipid substrate is able to reach and bind to the lipase active site. Second, the nucleophilic oxygen of the serine side chain binds the carbonyl carbon of the ester bond, forming a tetrahedral intermediate, stabilized by hydrogen bonding with amide nitrogen atoms of the amino acid residues nearby. Third, the ester bond is cleaved, which frees an alcohol and produces an acyl-enzyme complex. Last, the acyl-enzyme is hydrolyzed upon entry of a water molecule or alcohol into the active site. This frees the fatty acid (in case of water as nucleophile) or ester (in case of an alcohol as nucleophile) and the lipase is regenerated.

Due, in part, to their diverse functioning and structure, as revealed by sequence analysis and crystallography, lipases belong to different enzyme subclasses or families. *Pseudozyma* (formerly *Candida*) *antartica* is a basidomyceteous yeast strain isolated from Lake Vanda in Antarctica that produces two differently functioning lipases: lipase A

(CAL-A) and lipase B (CAL-B) (Ericsson *et al.,* 2008). These two lipases have been previously characterized and the amino acid and DNA sequences encoding these lipases have been determined (Novo Nordisk A/S; Hoegh *et al.,* 1995). CAL-B is a widely used enzyme in organic synthesis on both the laboratory and commercial scale, especially in the resolution of racemic mixtures.

CAL-A is one representative of a new class of lipases and, due to its properties, including thermostability, has been used as a catalyst in the paper, wax, food and flavor, and biopharmaceutical industries. CAL-A has an unusual lid structure and C-terminal flap, which can accept very bulky substrates like highly branched acyl groups and sterically hindered alcohols and amines (Kirk and Christensen, 2002; Krishna *et al.,* 2002; Schmidt *et al.,* 2005). CAL-A also shows a higher homology to peptidase structures rather than typical lipase structures (Ericsson *et al.,* 2008). CAL-A has shown some selectivity for the esterification of *trans*-fatty acids over the corresponding *cis*-isomers (Borgdorf and Warwel, 1999). The reasons for this selectivity and a model for describing this type of selectivity have not been elucidated. CAL-A has scarcely been used as a biocatalyst (de Maria *et al.,* 2005; Li and Kanerva, 2006).

Fatty acids typically have different lengths and are often classified as short-, medium-, or long-chain fatty acids. Short-chain fatty acids are fatty acids with aliphatic tails fewer than six carbons, medium-chain fatty acids are fatty acids with aliphatic tails of six up to twelve carbons and can form medium-chain triglycerides. Long-chain fatty acids are fatty acids with aliphatic tails greater than or equal to twelve carbons. Medium length fatty acids are typically lower calorie fats, which can be important in dietary intake as well as processing of lipids within the body.

Mono- or polyunsaturated fats with *trans*-isomer fatty acid(s) are commonly called "*trans* fats." *Trans*-isomers contain chains where the carbon atoms next to the double bond are located geometrically opposite, whereas in *cis*-isomers the carbon atoms next to the double bond are geometrically on the same side. In the *cis* configuration, the naturally occurring unsaturated fatty acids have lower melting points than that of saturated fatty acids and thus are found in liquid form. Typically, *trans*-fatty acids are found in food products as a result of a partial hydrogenation process. *Trans*-fatty acids have higher melting points than those of the *cis*- unsaturated fatty acids and are less susceptible to autooxidation and so can form a more stable solid or semi-solid fat. Dietary intake of *trans*-fatty acids has been linked to an increased risk for heart disease, diabetes, obesity, metabolic syndrome, Alzheimer's disease, cancer, liver dysfunction and infertility. For these reasons, attempts have been made to reduce the *trans*-fatty acid content in dietary products (Ratnayake and Cruz-Hernandez, 2009).

Lipases have gained significant commercial importance over the past thirty years; however, their expression levels in native organisms are often much too low to meet increasing commercial and industrial needs. Therefore, numerous attempts have been made to optimize the activity, selectivity, sensitivity and stability of lipases. These methods have included, for example, immobilizing lipases onto solid supports, using nonaqueous solvents, mutant selection, and a variety of recombinant DNA and protein engineering techniques to increase yield, efficiency, stability, and/or specificity. Recent improvements in the understanding of underlying gene expression, protein folding, and polypeptide secretion of lipases has permitted higher levels of production of these biocatalysts in many systems (see *e.g.*, Napolitano and Giuffrida, 2009).

Due to the central importance of lipase function in lipid metabolism and transport, and its implication in serious diseases and conditions such as heart disease, diabetes, obesity, metabolic syndrome, Alzheimer's disease, cancer, liver dysfunction and infertility, it is imperative to know not only how lipases work, but also how to improve the activity, selectivity, sensitivity and stability of lipases. What is desirable, therefore, are compositions and methods for producing novel lipase variants with improved enzymatic properties, increasing the preference or affinity of lipases for particular fatty acid chain lengths, increasing the range and/or number of fatty acid chains that a given type of lipase is able to use as a substrate, and increasing the *trans*-selectivity of lipases to facilitate a reduction or elimination of one or more *trans-fatty* acids from a given lipid substrate. Such compositions and methods find particular utility in a variety of analytical assays, dietary regimens, commercial processes, and manufacturing in the food industry. Sandstrom et al. 2009 (Protein engineering and Design, 22(7) p413-420) discloses a directed evolution method of Candida antarctica lipase A and several mutants generated using said method. WO94/01541 (Novo Nordisk AS, 20.01.1994) discloses Candida antarctica CAL-A lipase mutants, wherein residues have been altered by aromatic residues in order to alter the specificity for certain fatty acids. WO2008/040738 (Novozymes AS, 10.04.2008) discloses CAL-A lipase variants having increased specificity for short chain fatty acids. Database entry UniProt:Q8NIP2 (01.10.2002) discloses a Kurtzmanomyces sp. lipase sequence which has at a residue corresponding with position 246 of present Seq ID NO 11 a substitution of an isoleucine for a leucine.

## BRIEF SUMMARY OF THE INVENTION

The present invention provides new and useful compositions, as well as methods of making and employing them that may advantageously modulate, alter, ameliorate, and/or reduce the amount of one or more *trans-fatty* acid moieties and/or one or more medium- and/or long- chain fatty acid moieties in a substrate containing one or more such compounds.

The present invention provides a lipase variant according to claim 1, an isolated polynucleotide according to claim 9, an expression vector according to claim 10, a microbial host cell according to claim 11, a method according to claim 12 for producing a lipase variant, a method according to claim 13 and a lipase variant according to claim 15. Preferred features are defined in the dependent claims.

EP 2 702 150 B1

Described herein is an isolated lipase variant polypeptide that includes a modified parent lipase amino acid sequence, wherein the lipase variant has altered, enhanced, or improved lipolytic activity as compared to the parent lipase polypeptide from which it was derived. In one embodiment, the parent lipase polypeptide is obtained from, or, alternatively, derived from, one or more lipase enzymes found in a fungal species such as *Candida antarctica, Ustilago maydis, Kurtzmanomyces species. 1-11, Sporisorium relianum,* or a related microbial source.

In some embodiments, the lipase variant polypeptides of the present invention have a preference, an increased selectivity, or enhanced activity, for catalysis of medium- and/or long-chain length fatty acid moieties *(i.e.,* those having a carbon chain length of about 6 carbon atoms or more). In particular, such polypeptides have been shown to possess an increased affinity for fatty acid moieties have a carbon chain length of greater than about 8 or about 10 carbon atoms or more. In other embodiments, the isolated lipase variant polypeptides of the present invention display an increased selectivity for the catalysis of *trans*-fatty acid moieties. In certain embodiments, the isolated lipase variant polypeptides display *both* an increased preference for catalysis of medium- and/or long-chain fatty acids, *and* an increased *trans*-fatty acid selectivity.

In exemplary embodiments, the lipase variant polypeptides of the present disclosure may include an amino acid sequence that is at least about 95% identical to the amino acid sequence of SEQ ID NO:11, or to the complement thereof. Preferably, such polypeptides contain one or more amino acid substitutions at one or more of amino acid residues 170, 171, 239, 242, 243, 254, 258, 262, 269, 322, and 325, wherein each amino acid position is numbered by correspondence to a position in the amino acid sequence of the lipase as set forth in SEQ ID NO:11. More preferably, such lipase variants contain one or more substitutions of one or more amino acid residues of the polypeptide sequence of SEQ ID NO:11, wherein the one or more substitutions confers to the lipase variant polypeptide a) an increased preference for catalysis of medium- and/or long-chain fatty acids; b) an enhanced selectivity for catalysis of *trans*-fatty acids; or c) a combination thereof.

In particular embodiments, the amino acid sequence of the isolated lipase variant is at least about 95% identical, at least about 96% identical, at least about 97% identical, at least about 98% identical, or at least about 99% identical to the full-length polypeptide sequence set forth in SEQ ID NO: 11.

In illustrative embodiments, the one or more amino acid substitutions may include one or more of: a phenylalanine-to-serine substitution, a phenylalanine-to-cysteine substitution, a phenylalanine-to-aspartic acid substitution, a phenylalanine-to-glycine substitution, a phenylalanine-to-methionine substitution, a phenylalanine-to-threonine substitution, a phenylalanine-to-asparagine substitution, a phenylalanine-to-valine substitution, an isoleucine-to-histidine substitution, an isoleucine-to-arginine substitution, an isoleucine-to-alanine substitution, a threonine-to-isoleucine substitution, a threonine-to-histidine substitution, a leucine-to-phenylalanine substitution, a leucine-to-asparagine substitution, a leucine-to-tyrosine substitution, a leucine-to-cysteine substitution, a leucine-to-glutamine substitution, a phenylalanine-to-arginine substitution, a leucine-to-arginine substitution, an alanine-to-asparagine substitution, a glycine-to-alanine substitution, a glycine-to-leucine substitution, a glycine-to-valine substitution, a glycine-to-tyrosine substitution, a glycine-to-arginine substitution, a glycine-to-tryptophan substitution, an isoleucine-to-lysine substitution, an isoleucine-to-threonine substitution, an isoleucine-to-glutamic acid substitution, an isoleucine-to-valine substitution, a leucine-to-alanine substitution, a methionine-to-valine substitution, or a leucine-to-alanine substitution, or any combination thereof.

In exemplary embodiments, the one or more amino acid substitutions includes a phenylalanine-to-serine substitution at amino acid position 243 (denoted "F243S" in standard protein nomenclature), a phenylalanine-to-cysteine substitution at amino acid residue 243 *(i.e.,* an F243C mutation), a phenylalanine-to-aspartic acid substitution at amino acid residue 170 (F170D), a phenylalanine-to-glycine substitution at amino acid residue 170 (F170G), a phenylalanine-to-methionine substitution at amino acid residue 170 (F170M), a phenylalanine-to-threonine substitution at amino acid residue 170 (F170T), a phenylalanine-to-asparagine substitution at amino acid residue 170 (F170N), a phenylalanine-to-valine substitution at amino acid residue 170 (F170V), an isoleucine-to-histidine substitution at amino acid residue 322 (I322H) or at amino acid residue 171 (I171H), an isoleucine-to-arginine substitution at amino acid residue 322 (I322R) or at amino acid residue 171 (I171R), an isoleucine-to-alanine substitution at amino acid residue 322 (I322A), a threonine-to-isoleucine substitution at amino acid residue 242 (T242I), a threonine-to-histidine substitution at amino acid residue 242 (T242H), a leucine-to-phenylalanine substitution at amino acid residue 326 (L326F), a leucine-to-asparagine substitution at amino acid residue 326 (L326N) or at amino acid residue 262 (L262N), a leucine-to-tyrosine substitution at amino acid residue 326 (L326Y), a leucine-to-cysteine substitution at amino acid residue 326 (L326C), a leucine-to-glutamine substitution at amino acid residue 326 (L326Q), a phenylalanine-to-arginine substitution at amino acid residue 244 (F244R), a leucine-to-arginine substitution at amino acid residue 246 (L246R), an alanine-to-asparagine substitution at amino acid residue 149 (A149N), a glycine-to-alanine substitution at amino acid residue 258 (G258A), a glycine-to-leucine substitution at amino acid residue 258 (G258L), a glycine-to-valine substitution at amino acid residue 258 (G258V), a glycine-to-tyrosine substitution at amino acid residue 258 (G258Y), a glycine-to-arginine substitution at amino acid residue 258 (G258R), a glycine-to-tryptophan substitution at amino acid residue 258 (G258W), an isoleucine-to-lysine substitution at amino acid residue 171 (I171K), an isoleucine-to-threonine substitution at amino acid residue 171 (I171T), an isoleucine-to-glutamic acid substitution at amino acid residue 171 (I171E), an isoleucine-to-valine sub-

5

stitution at amino acid residue 171 (I171V), a leucine-to-alanine substitution at amino acid residue 262 (L262A), a methionine-to-valine substitution at amino acid residue 269 (M269V), or a leucine-to-alanine substitution at amino acid residue 326 (L326A), or any combination thereof.

In one aspect of the invention, such amino acid substitution(s) confer to the polypeptide variant a preference for catalysis of medium- or long-chain fatty acids, when compared to the un-substituted "parent" lipase from which the mutated polypeptide was derived. Similarly, in other aspects, the one or more amino acid substitutions confer to the polypeptide variant an increased or an enhanced hydrolytic or ethanolytic activity towards *trans-fatty* acid substrates, as compared to the trans-fatty acid selectivity exhibited by the un-substituted, starting, or "parent," lipase from which the mutated polypeptide was derived.

In further embodiments, the at least a first amino acid substitution confers to the polypeptide an about 5-fold increase in hydrolytic or ethanolytic activity in the presence of an at least a first *trans-fatty* acid moiety, when compared to a non-selective or un-substituted lipase. In some embodiments, the at least a first amino acid substitution confers to the polypeptide an about 10-fold increase in hydrolytic or ethanolytic activity in the presence of an at least a first *trans*-fatty acid moiety, when compared to a non-selective or un-substituted lipase. In still other embodiments, the at least a first amino acid substitution confers to the polypeptide an about 15-fold increase in hydrolytic or ethanolytic activity in the presence of an at least a first *trans*-fatty acid moiety, when compared to the un-substituted, starting, or "parent," lipase from which the mutated polypeptide was derived.

In some embodiments, the at least a first amino acid substitution confers to the polypeptide an increased preference for catalyzing fatty acid moieties having a carbon chain length greater than or equal to about six ($\geq C_6$), greater than or equal to about eight ($\geq C_8$), greater than or equal to about ten ($\geq C_{10}$), or even greater than or equal to about twelve ($\geq C_{12}$), when compared to the wild-type, un-substituted, parent lipase from which the mutant lipase was derived.

In other embodiments, the at least a first amino acid substitution confers to the polypeptide an increased preference for catalyzing fatty acid moieties having a carbon chain length greater than or equal to six ($\geq C_6$), and about a 15-fold increase in hydrolytic or ethanolytic activity in the presence of an at least a first *trans*-fatty acid moiety, when compared to wild-type, un-substituted, starting lipase from which the mutant lipase was derived.

In some embodiments the isolated lipase variant includes: an amino acid sequence that is at least about 95% identical, at least about 96% identical, at least about 97% identical, at least about 98% identical, or at least about 99% identical, to the amino acid sequence of SEQ ID NO:11, or to the complement thereof; and that contains one or more amino acid substitutions at one or more of amino acid residues 170, 171, 239, 242, 243, 254, 258, 262, 269, 322, and/or 326, wherein each amino acid position is numbered by correspondence to a position in the amino acid sequence of the lipase polypeptide as set forth in SEQ ID NO:11; and further wherein the one or more amino acid substitutions confers to the polypeptide: a) an increased preference for catalyzing fatty acid moieties having a carbon chain length greater than or equal to six ($\geq C_6$); b) an increase in the hydrolytic and/or the ethanolytic activity of the lipase in the presence of an at least a first *trans*-fatty acid moiety, as compared to that of an un-substituted, wild-type, or "parent" lipase.

Some embodiments of the present invention include formulations of the lipase variants, and compositions comprising them. Such compositions may optionally include one or more additional enzymes, including, for example, one or more of the lipase variants described herein. In related embodiments, such compositions may optionally further include one or more enzyme substrates, including, without limitation, one or more of a lipid, a fatty acid, a sterol, a wax, an oil, a triglyceride, an ester, a carboxylic acid moiety, or any combination thereof. In other embodiments, the composition further includes a binding medium to which the lipase variant binds or cross-links. In some embodiments, the lipase variant is substantially bound or substantially chemically cross-linked to a matrix, a column, a fiber, a filter, a resin, a gel, a bead, or any combination thereof.

In some embodiments, the composition further includes one or more additional enzymes. In some embodiments, the one or more additional enzymes comprise a second, distinct, variant lipase. In further embodiments, the one or more additional enzymes include one or more lipases, one or more esterases, one or more lyases, one or more deproteinases, one or more phosphatases, one or more dehydrogenases, one or more transglutaminases, oxidases, or any one or more combinations thereof.

In further embodiments, the invention provides isolated polynucleotides that encode one or more of the claimed lipase variants, as well as expression constructs, recombinant vectors, and host cells comprising them that are capable of expressing the variant polypeptide under suitable conditions. In some embodiments, such expression vectors may include one or more isolated polynucleotides that are codon-optimized for expression in a particular bacterial or a particular non-basidomyceteous yeast cell. In exemplary embodiments, constructs are provided for expression of the disclosed lipase enzymes in bacterial cells such as *E. coli,* or in non-basidomyceteous yeast cells such as *P. pastoris.* In an illustrative example, the expression vector is defined as pGA15-pproCAL-Aop.

Further embodiments encompass a microbial host cell transformed with the isolated polynucleotide that encodes the lipase variant, or the expression vector that includes an isolated polynucleotide encoding one or more of the lipase variants. In some embodiments, the microbial host cell is defined as an *E. coli* or a *P. pastoris* host cell. In further embodiments, the expression vector encodes, in a non-*Pseudozyma* (formerly *Candida*) *antarctica* cell, the variant lipase.

Further embodiments provide for a method for producing a lipase variant, comprising (a) culturing a population of microbial host cells that encode the lipase variant, under conditions conducive to the expression of the lipase variant, and recovering the expressed lipase variant, from the population of cells, or, in instances where the variant is secreted from the cells, from the medium in which the cells were cultured.

While exemplary mutant lipases have been described herein that contain one, two, or three amino substitutions, the inventors contemplate that additional lipase mutants may be prepared that include four or five amino acid substitutions within the sequence of the parent polypeptide from which the variant is derived.

Further embodiments encompass methods for obtaining and preparing lipase variants in accordance with the invention. Such methods may include mutagenizing a polynucleotide that encodes a polypeptide that is at least about 95% identical to the amino acid sequence of SEQ ID NO:11 under conditions effective to generate at least a first lipase variant that comprises an amino acid substitution in at least a first amino acid residue therein that confers to the polypeptide an increased *trans*-selectivity, or an increased preference for catalyzing medium- or long-chain fatty acid moieties ($\geq C_6$) over that of short-chain fatty acid moieties ($< C_6$); transforming a suitable microbial host cell with the mutagenized polynucleotide; culturing the microbial host cell under conditions effective to express the first lipase variant; and recovering the first expressed lipase variant from the culture.

Another embodiment provides for a method of reducing or eliminating one or more *trans*-unsaturated fatty acid compounds or one or more medium- or long-chain ($\geq C_6$) fatty acid moieties from a substrate, including contacting the substrate with an effective amount of a composition comprising the lipase variant, for a time sufficient to hydrolyze or esterify at least a portion of the substrate thereby reducing or eliminating the one or more *trans*-unsaturated fatty acid compounds or the one or more medium- or long-chain ($\geq C_6$) fatty acid moieties from the substrate. In some embodiments, the method further includes removing the lipase variant from the composition after the one or more *trans*-unsaturated fatty acid compounds or the one or more medium- or long-chain ($\geq C_6$) fatty acid moieties has been substantially reduced or eliminated from the substrate. In further embodiments, the substrate includes an edible lipid, an edible fat, an edible fatty acid, an edible sterol, an edible wax, an edible oil, or an edible triglyceride, or any combination thereof. Further embodiments encompass a fat- or an oil-containing product (including, for example, foods, feedstocks and the like) having an altered (and preferably, reduced or eliminated *trans*-fatty acid content) produced by the methods of the invention. In some embodiments, the fat- or oil-containing product is suitable for animal, and particularly human, consumption. In illustrative embodiments, the fat- or oil-containing product is characterized as a cooking ingredient, a vegetable or animal fat or oil, or a commercial foodstuff, a food product, or cooking, and/or frying fats and/or oils.

In another embodiment, there is a substitution of any non-native amino acid at, near or facing the fatty acid binding tunnel or active site of the isolated polypeptide. In further embodiments, the substitution is selected from the group consisting of: (a) a substitution of a non-native amino acid at position 170 in SEQ ID NO:11; (b) a substitution of a non-native amino acid at position 171 in SEQIDNO:SEQIDNO:11; (c) a substitution of a non-native amino acid at position 239 in SEQ ID NO: 11; (d) a substitution of a non-native amino acid at position 242 in SEQ ID NO:11; (e) a substitution of a non-native amino acid at position 243 in SEQ ID NO:11; (g) a substitution of a non-native amino acid at position 254 in SEQ ID NO: 11; (h) a substitution of a non-native amino acid at position 258 in SEQ ID NO:11; (i) a substitution of a non-native amino acid at position 262 in SEQ ID NO:11; (j) a substitution of a non-native amino acid at position 269 in SEQ ID NO:11; (k) a substitution of a non-native amino acid at position 322 in SEQ ID NO:11; (1) a substitution by a non-native amino acid at position 326 in SEQ ID NO: 11; and (m) any combination of substitutions (a) - (1).

Exemplary amino acid substitutions in accordance with the present invention include, but are not limited to, substitution of the wild-type amino acid residue at one or more selected positions within the CAL-A lipase sequence shown in SEQ ID NO: 11. Such substitutions include, without limitation, (a) an F243S or an F243C substitution in the amino acid sequence of SEQ ID NO:11; (b) an F170D substitution, a F170G substitution, a F170M substitution, a F170T substitution, a F170N substitution, and a F170V substitution; (c) an I322H substitution, an I322R substitution, and an I322A substitution; (d) a T242I substitution, a T242I substitution, and a T242H substitution; (e) an I326F substitution, an I326N substitution, an I326Y substitution, an I326C substitution, and an I326Q substitution, (f) a F244R substitution; (g) a L246R substitution; (h) a A239N substitution; (i) a G258A substitution, a G258L substitution, a G258V substitution, a G258Y substitution, a G258R substitution, and a G258W substitution; (j) an I171K substitution, an I171 H substitution, an I171R substitution, an I171T substitution, an I171E substitution, and an I171V substitution; (k) a L262A substitution, an L262N substitution; (1) a M269V substitution; (m) a L326A substitution; and (n) any combination of one or more of the amino acid substitutions defined in in one of (a) to (g). Exemplary lipase sequences comprise, consist essentially of, or alternatively, consist of the amino acid sequence of SEQ ID NO: 11 substituted in at least one, at least two, at least three, at least four, or at least five or more amino acid residues of the sequence of SEQ ID NO:11.

Other embodiments provide for a composition comprising at least a first polypeptide according to any of those stated above and a binding medium to which the polypeptide binds or cross-links so as to act as a filter. In other embodiments, the composition further includes at least a second distinct polypeptide in accordance with any of those stated above. In further embodiments, the composition includes from about 1% to about 99% by weight of the polypeptide. In still further embodiments, the composition is formulated as a powder, dust, pellet, granule, spray, emulsion, colloid, or solution. In other embodiments, the composition is prepared by desiccation, lyophilization, homogenization, extraction, filtration, centrifugation, sedimentation, or a concentration of a culture of *E. coli* or *P. pastoris* cells.

Another embodiment provides for an isolated nucleic acid molecule that encodes the lipase variant of any of the polypeptides stated above. Also described herein is the nucleotide sequence selected from the group consisting of: (a) a nucleotide sequence, or the complement thereof, that comprises, consisting essentially of, or alternatively, consists of, an at least 300 continuous nucleotide segment from SEQ ID NO:12; (b) a nucleotide sequence, or the complement thereof, that encodes an about 396 continuous amino acid segment of the amino acid sequences of SEQ ID NO: 11, wherein the segment has lipolytic activity; or (c) a nucleotide sequence, or the complement thereof, encoding at least a 417 continuous amino acid segment of the amino acid sequences set forth in SEQ ID NO: 11, wherein the lipase variant polypeptide has a modified lipolytic activity *in vivo* and/or *in vitro* as compared to the starting lipase polypeptide from which the variant was prepared or obtained.

In further embodiments, a recombinant nucleic acid vector comprising at least a first sequence region that encodes the polypeptide of any one of those stated above or the isolated nucleic acid molecule as stated above. In some embodiments, the nucleic acid vector is further defined as a plasmid vector.

In still other embodiments, a transformed non-human host cell comprising a nucleic acid segment that encodes the polypeptide of any one of those stated above is disclosed. In some embodiments, the transformed non-human host cell is further defined as a prokaryotic or eukaryotic host cell. In other embodiments, the transformed non-human host cell is further defined as a bacterial cell or a yeast cell. In some embodiments the bacterial cell is an *E. coli* cell or the yeast cell is a *P. pastoris* cell.

Also disclosed herein are methods for producing a recombinant protein with lipolytic activity comprising the amino acid sequence of SEQ ID NO:11, or any variant thereof, and comprising introducing a nucleotide sequence encoding the polypeptide into a host cell, culturing the host cell under conditions in which the polypeptide is expressed, and recovering the polypeptide from the culture.

Also disclosed herein is a method for obtaining a modified *Candida antarctica* protein segment with lipolytic activity having increased *trans*-selectivity as compared to a wild type *Candida antarctica* including: providing a polynucleotide encoding the polypeptide sequence of SEQ ID NO:11; altering the nucleic acid sequence encoding for any non-native amino acid at, near or facing a proposed three-dimensional binding, active site or tunnel of the polypeptide or removing or rendering non-functional the C-terminal flap of the polypeptide; introducing the altered nucleotide sequence encoding the polypeptide into a host cell; culturing the host cell under conditions in which the polypeptide is expressed; and recovering the polypeptide from the culture. In some embodiments, the altering of the nucleic acid results in the amino acid substitutions any of those stated above.

Further embodiments encompass a method to reduce or eliminate the content of *trans*-unsaturated fatty acid compounds from a substrate including: providing a solution including a stable substrate containing *trans*-unsaturated fatty acid moieties; and contacting the lipase variant of any one of claims 1 to 8 with the solution including a stable substrate for a sufficient amount of time to hydrolyze or esterify at least a portion of the *trans*-unsaturated fatty acid moieties. In some embodiments, the method further includes exposing the recovered polypeptide to a substrate containing *trans*-unsaturated fatty acid compounds. In other embodiments, the stable substrate is an edible oil composition. In still further embodiments, the method further includes removing or inactivating at least a portion of the composition of those stated above after contacting the solution including a stable substrate. The invention further encompasses a fat-containing product having reduced *trans-fatty* acid moieties or no *trans-fatty* acid moieties as produced from the method.

Also disclosed herein is a method of producing a low *trans*-fatty acid composition or a composition that lacks *trans*-fatty acid(s) including: providing a polynucleotide encoding the polypeptide sequence of SEQ ID NO: 11; altering the nucleic acid sequence encoding for any non-native amino acid at, near or facing a proposed three-dimensional binding, active site or tunnel of the polypeptide or removing or rendering non-functional the C-terminal flap of the polypeptide; introducing the altered nucleotide sequence encoding the polypeptide into a host cell; culturing the host cell under conditions in which the polypeptide is expressed; recovering the polypeptide from the culture; providing a solution including a stable substrate containing *trans*-unsaturated fatty acid moieties; and contacting a composition containing the recovered polypeptide with the solution including a stable substrate for a sufficient amount of time to hydrolyze or esterify at least a portion of the *trans*-unsaturated fatty acid moieties. In some embodiments, the method further includes removing or inactivating the composition after contacting the solution including a stable substrate.

## BRIEF DESCRIPTION OF THE DRAWINGS

Reference will now be made to the embodiments, or examples, illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one of ordinary skill in the art to which the invention relates.

The following drawings form part of the present specification and are included to demonstrate certain aspects of the present invention. The invention may be better understood by reference to the following description taken in conjunction with the accompanying drawings, in which like reference numerals identify like elements, and in which:

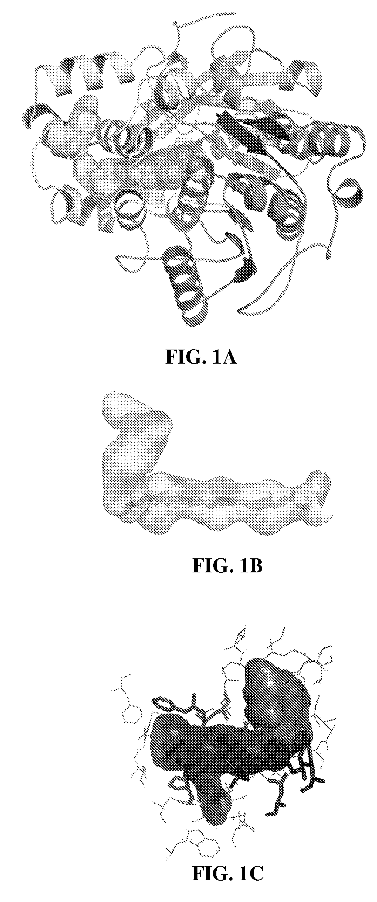

**FIG. 1A, FIG. 1B** and **FIG. 1C** show models of the substrate binding tunnel of wild-type CAL-A (hereinafter "CAL-A" or "wild-type CAL-A" or "CAL-A (WT)") from YASARA, PYMOL, and CASTp programs respectively;

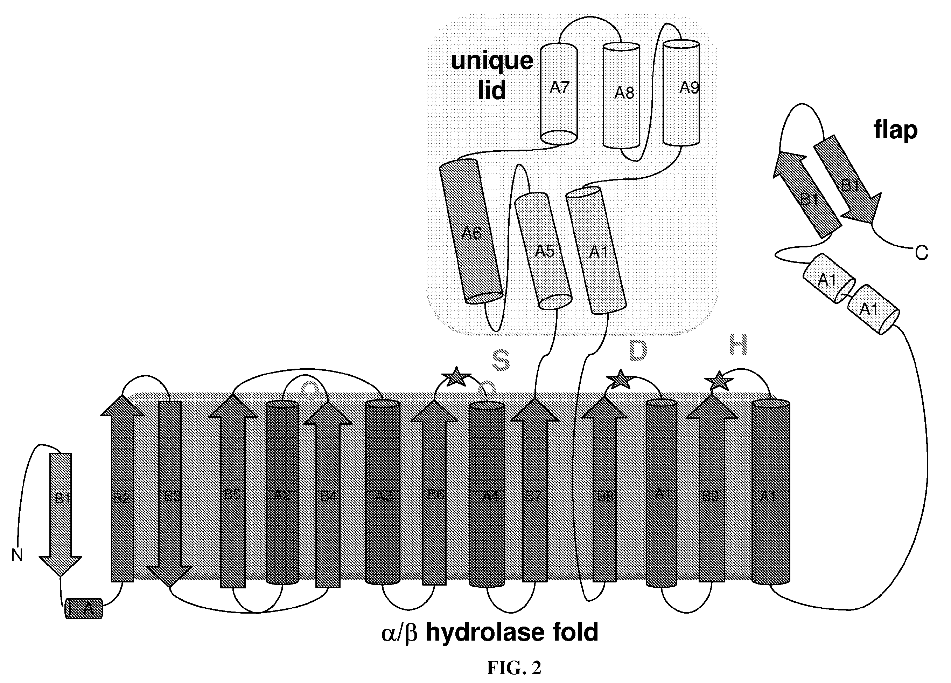

**FIG. 2** shows the secondary protein structure of CAL-A including the $\alpha/\beta$ hydrolase fold, unique lid and flap structures;

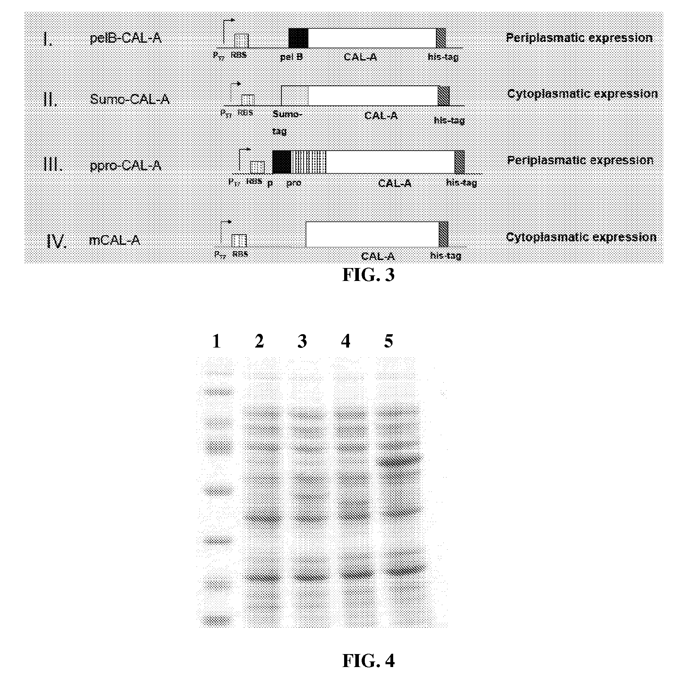

**FIG. 3** shows a schematic of four CAL-A constructs used in evaluating the location of the expressed protein, or variants thereof, in *E. coli* cells. Two of the constructs *(i.e.,* Constructs I and III) demonstrated periplasmic expression, due to the pelB signal and pre-pro sequence attached to the CAL-A gene *(i.e.,* the ppro-CAL-A construct), respectively. The other two constructs *(i.e.,* Constructs II and IV) demonstrated cytoplasmic expression of the CAL-A protein, due to the attachment of the SUMO-tag and absence of the pre-pro secretory sequence, respectively;

**FIG. 4** shows the expression of a shortened form of the lipase from *Ustilago maydis* (lipUMs) (lane 3), CAL-A *(i.e.,* the ppro-CAL-A construct or Construct III) (lane 4) and the full length form of the lipase from *Ustilago maydis* (lipUMf) (lane 5) in crude extracts of transformed *E. coli* (DE3) strain cells. The gel as shown compares these lanes to *E. coli* cells transformed with an empty pET-22b(+) expression vector as a negative control (lane 2) and a standard marker (lane 1);

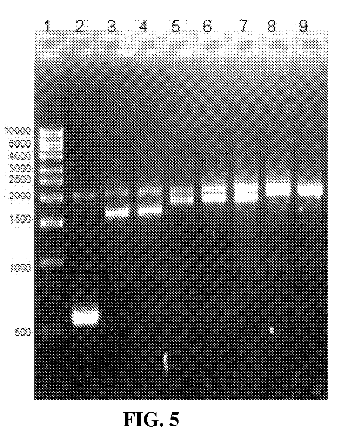

**FIG. 5** shows the integration of a shortened or truncated form of a lipase sequence from *Ustilago maydis* (lipUMs) (lanes 3 and 4), the full sequence of the lipase from *Ustilago maydis* (lip UMf) (lanes 8 and 9) and CAL-A (lanes 5-7) into the genome of *Pichia pastoris.* The gel as shown compares these lanes to a standard 1-Kb marker (lane 1) and the PCR product from the pPICZ$\alpha$(C) empty vector. The upper PCR product is the AOX1 gene, while the band below shows the specific integrated product;

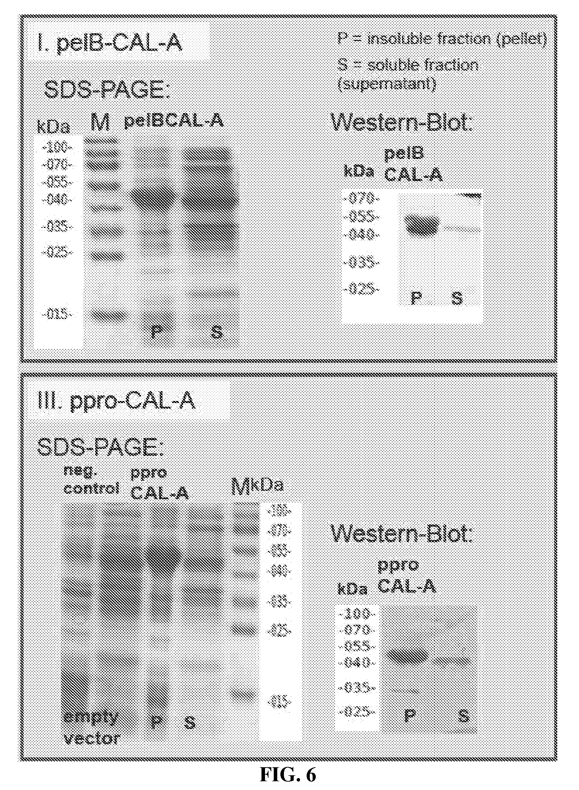

**FIG. 6** shows SDS-PAGE and Western Blot results for Constructs I and III, demonstrating that periplasmic expression of CAL-A results in higher enzyme activity in the supernatant than does cytoplasmic expression;

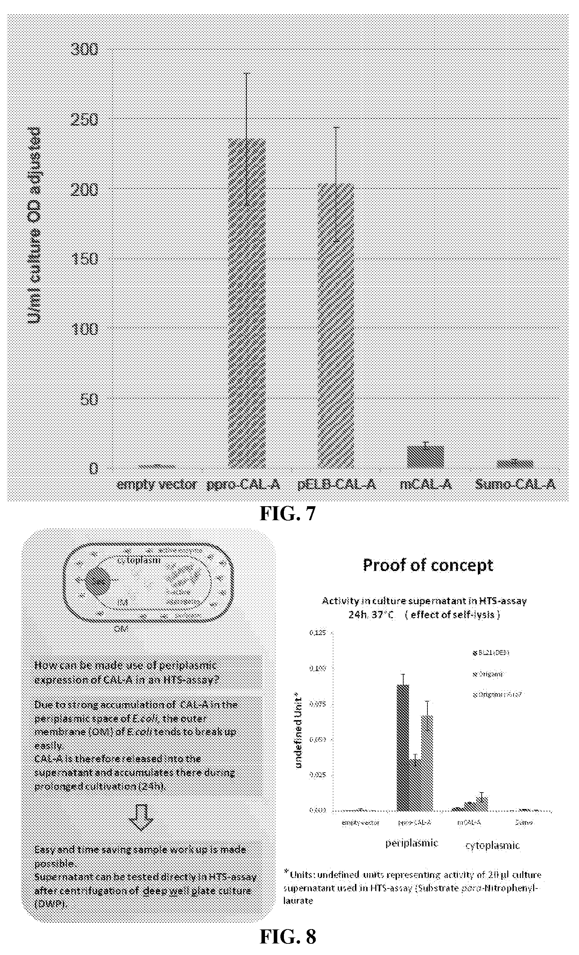

**FIG. 7** shows the activity (U/mL culture, OD adjusted), of recombinantly expressed CAL-A from Constructs I through IV *(i.e.,* the vectors pET22-pelB-CAL-Aophis for Construct I, pET22-SUMO-CAL-Aophis for Construct II, pET22-pproCAL-Aophis for Construct III, and pET22-mCAL-Aophis for Construct IV were used wherein "ophis" indicates codon optimization and the attachment of a his-tag) when para-nitrophenyl (p-NP) laurate ($C_{12}$) is the substrate. The empty vector, pET22b(+), without the CAL-A sequence, was used as a control;

**FIG.8** similarly shows the activity (U/mL culture, OD adjusted), of recombinantly expressed CAL-A from Constructs I through IV when pNP-laurate (C12) is the substrate. CAL-A constructs were transformed into *E. coli* C41 (DE3), Origami and Origami pGro7 cells. The empty vector, pET22b(+) without the CAL-A sequence, was used as a control;

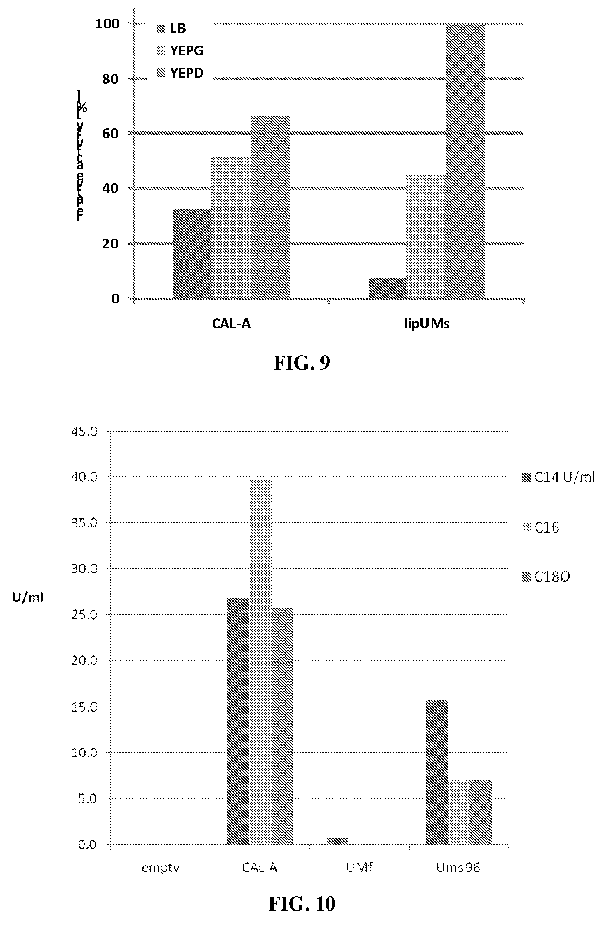

**FIG. 9** shows a comparison of the hydrolytic activity of *E. coli* C41 (DE3) crude extracts transformed with Construct III, or the pproCAL-A sequence, cultured on three different cultivation media, namely standard complex medium (LB), glycerol enriched complex medium (YEPG) and glucose enriched complex medium (YEPD), at 20°C and 180rpm. The photometric assay was performed with pNP-laurate ($C_{12}$) as the substrate;

**FIG. 10** shows the volumetric lipase activity of *E. coli* C41 (DE3) crude extracts of CAL-A, lipUMs, and lipUMf at 30°C for three different pNP-esters, *p*-NP-C14 ($C_{14}$), *p*-NP-C16 ($C_{16}$) and *p*-NP-C18:1$\Delta$9*cis* (C18O);

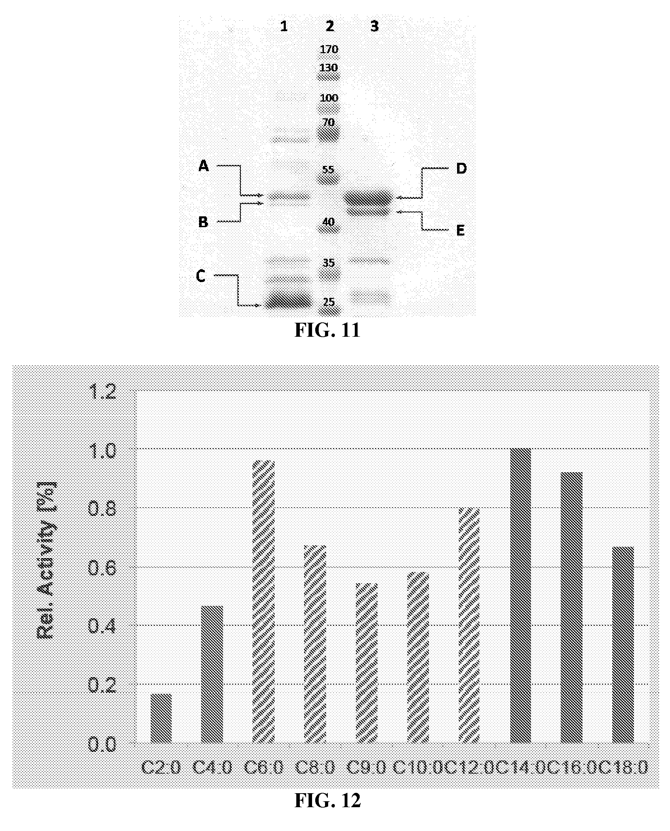

**FIG. 11** shows the detection of processed and degraded lipUMs form variants (lane 3), as well as CAL-A (lane 1) by a Coomassie™ Brilliant Blue G-250-stained (AkzoNobel, London, England, UK) 10% sodium dodecyl sulfate-polyacrylamide gel electrophoresis (SDS-PAGE) gel. The CAL-A and lipUMs fractions were purified by His-tag purification. A pre-stained protein ruler marker is shown for comparison (lane 2). The identity of the protein bands was analyzed by MALDI-ToF. Band A represent the unprocessed form of lipUMs (~49 kDa), band B represents the processed form of lipUMs without the pre-pro leader sequence (~47 kDa), band C represents the degraded and/or cleavage product of the lipUMs (~28 kDa), band D represents the unprocessed form of CAL-A (~49 kDa) and band E represents the processed form of CAL-A without the pre-pro leader sequence (~46 kDa);

**FIG. 12** depicts the chain-length preference of CAL-A. The hydrolytic activity towards *p*-NP esters with different chain lengths ($C_2$-$C_{18}$) was normalized to the myristic acid ester ($C_{14}$);

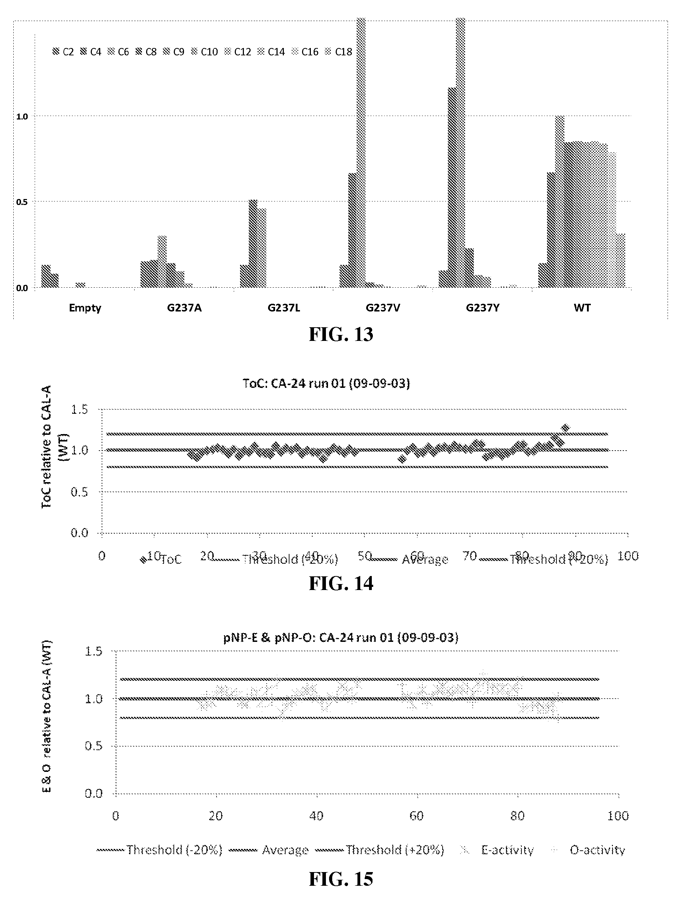

**FIG. 13** demonstrates the fatty acid chain length preference for four CAL-A variants, *i.e.,* G237A, G237L, G237V and G237Y as compared to wild-type CAL-A (WT) and an empty vector;

**FIG. 14** summarizes the "*trans* over *cis"* (ToC) values, as an indication of preference for *trans*-substrates, as determined using elaidic acid *p*-NP ester hydrolysis over oleic acid *p*-NP ester hydrolysis of CAL-A, normalized to random samples of CAL-A;

**FIG. 15** shows the residual activities of CAL-A normalized to random measurements of either hydrolysis activity of *p*-NP-elaidic acid (E-activity) or *p*-NP-oleic acid (O-activity) of CAL-A samples;

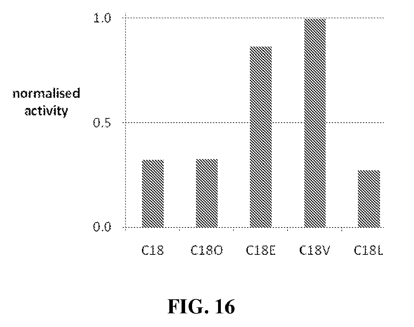

**FIG. 16** shows the preference of CAL-A for *p*-NP-esters with *trans*-fatty acids when transformed into *E. coli.* Five different $C_{18}$ fatty acid chains, *i.e.* stearic acid ($C_{18}$), vaccenic acid (C18V), elaidic acid (C18E), oleic acid (C18O), and linolenic acid (C18L), were used as substrates;

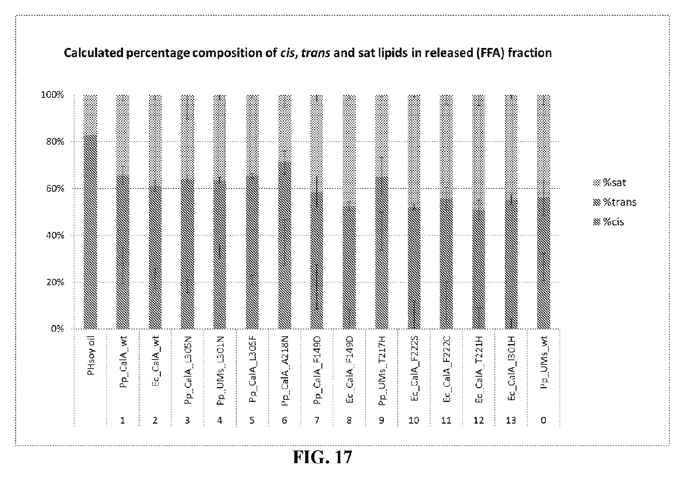

**FIG. 17** shows the percentage composition of *cis-, trans-* and saturated lipids in the released free fatty acid fraction for CAL-A, lipUMs and related variants;

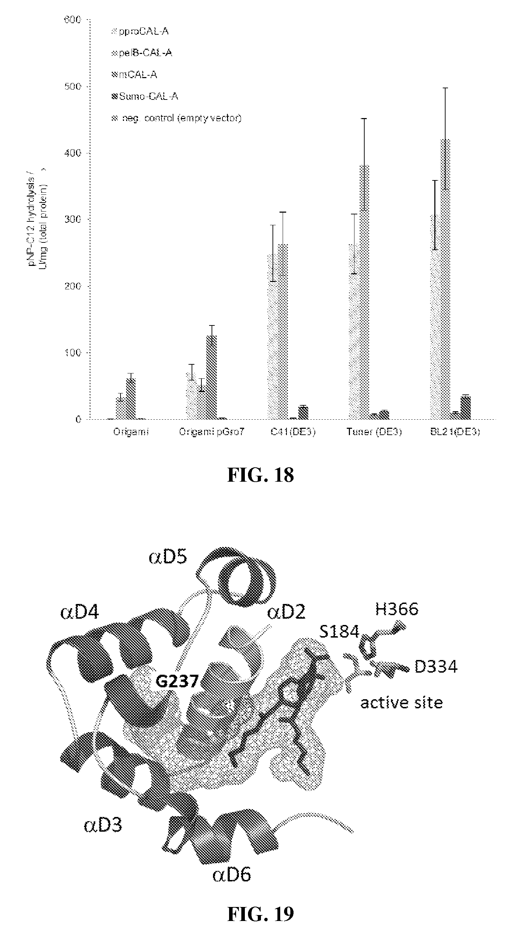

**FIG. 18** illustrates the activity (pNP-C12 hydrolysis / U/mg (total protein)) of various *E. coli* strains, *i.e.,* Origami, Origami pGro7 (Origami co-expressing GroES/EL complex), C41 (DE3), Tuner (DE3) and BL21(DE3), expressing different CAL-A constructs, *i.e.*, pproCAL-A, pelB-CAL-A, mCAL-A, Sumo-CAL-A, and a negative control (empty vector) when *para*-nitrophenyl (*p*-NP) laurate ($C_{12}$) is the substrate. The functional expression of two CAL-A constructs for periplasmic expression of CAL-A (pproCAL-A and pelB-CAL-A) was compared with the functional expression of two cytoplasmic-expressed CAL-A variants (mCAL-A and Sumo-CAL-A);

**FIG. 19** shows possible positions of substrates in the acyl-binding site of CAL-A. The fatty acid binding tunnel is mainly formed by the six consecutive helices *(i.e.,* αD2, αD3, αD4, αD5, αD6 as well as an unlabeled helix). Helix αD1 has been removed for a better view onto the binding tunnel. The position of G237 is shown as dots in helix αD2;

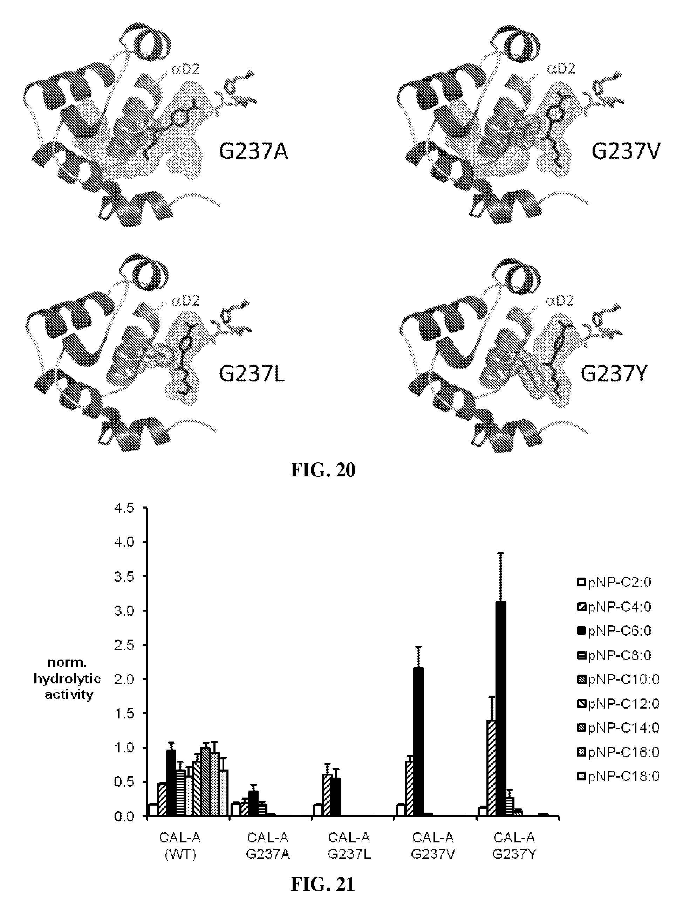

**FIG. 20** shows the acyl binding sites of the CAL-A G237A, G237L, G237V and G237Y mutants. Substitutions at position G237X in helix αD2 indicated as dots and sticks. Helix αD1 has been removed for a better view onto the binding tunnel. Structure visualisation was done with Pymol v1.5 and docking with Autodock 4. Individual binding pockets were identified by fpocket;

**FIG. 21** shows the chain length profile of CAL-A G237A, G237L, G237V and G237Y variants compared to CAL-A (WT) for hydrolysis of pNP-esters. For better comparison, the highest hydrolytic activity of CAL-A (WT), *i.e.* for pNP-C14:0, was set to 1.0. All other values have been standardized to this reference value. Photometric measurements of hydrolytic activity of *E. coli* crude extracts were done at least in triplicate. Enzymatic background measured with *E. coli* transformed with empty vector was insignificant; and

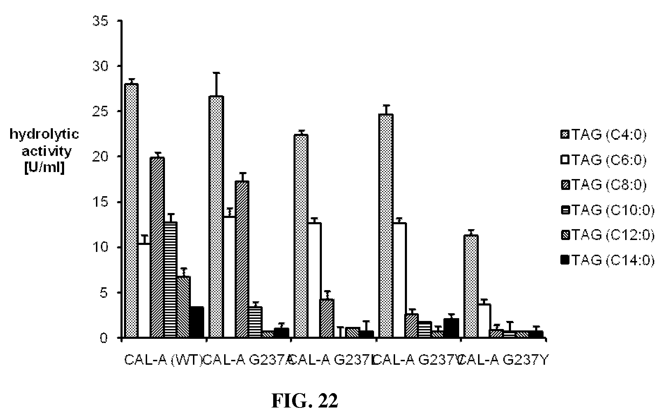

**FIG. 22** shows the chain length profile of CAL-A G237A, G237L, G237V and G237Y mutants compared to CAL-A(WT) for hydrolysis of different TAGs. Hydrolysis activity was determined in triplicate with *E. coli* crude extracts by pH-stat. Enzymatic background measured with *E. coli* transformed with empty vector was insignificant.

## DESCRIPTION OF ILLUSTRATIVE EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would be a routine undertaking for those of ordinary skill in the art having the benefit of this disclosure.

## LIPID ASSAYS

Numerous lipase assay methods have been used to determine lipase activity, including, but not limited to, using colored or fluorescent substrates, which allow spectroscopic and fluorimetric detection of lipase activity, chromatography techniques including high-performance liquid chromatography (HPLC), silver ion chromatography, gas chromatography and thin layer chromatography, titration of fatty acids released from the substrate, mass spectrometry and controlled surface pressure or oil drop tensiometry.

## CAL-A SUPERFAMILY

A variety of lipolytic enzymes may be used in the preparation of lipase variants in accordance with the present disclosure In one exemplary embodiment, the parent lipase is a microbial polypeptide (and preferably a bacterial or a fungal polypeptide), and more particularly, a polypeptide of yeast origin (including, without limitation) a polypeptide, sequence obtained from any one or more species of the genera *Acremonium*, *Aspergillus, Aureobasidium, Candida, Cryptococcus, Filobasidium, Fusarium, Humicole, Kluyveromyces*, *Kurtzmanomyces, Magnaporthe, Mucor, Myceliophthora, Neocallimastix, Neurospora, Paecilomyces, Penicillium*, *Pichia, Piromyces, Pseudozyma, Saccharomyces*, *Schizophyllum, Schizosaccharomyces, Sporisorium, Talaromyces*, *Thermoascus, Thielavia, Tolypoceladium, Trichoderma, Ustilago,* or *Yarrowia.*

In other embodiments, the lipase variants of the present disclosure may be prepared from a parent lipase polypeptide that is a member of the "CAL-A lipase superfamily" or "CAL-A superfamily." Members of the CAL-A lipase superfamily include, without limitation, Lipase A from *Candida antartica* (*i.e.,* CAL-A), LipK from *Kurtzmanomyces species 1-11,* LipUMf from *Ustilago maydis,* and two lipase precursors found in *Sporisorium relianum* (*i.e.,* SpoRei_E7 and SpoRei_E6).

Some members of this lipase superfamily, including CAL-A, lipK, SpoRei_E7 and SpoRei_E6, contain protein signal sequences. These sequences direct the translocation of the polypeptide to which it is linked through one or more secretory pathways of the cell in which it is synthesized. This secretory signal sequence may include both "pre" and "pro" regions. CAL-A has a "pre" region consisting of 21 codons, and a "pro" region consisting of 10 codons. The pro-sequence is only recognized and processed in yeast cells that possess the Kex2 protease. Kexin-like proteinases are a subfamily of the subtilisin-like serine proteinases with multiple regulatory functions in eukaryotes and catalyze or cleave proteins at the Kex2 cleavage site. Site-specific proteolysis is a feature in protein maturation and plays a crucial role in activation of many enzymes (and in the generation of peptide hormones). In the late secretory pathway of eukaryotic cells this mechanism is mainly mediated by kexin-like proteinases.

Table 1 shows a homology matrix for the CAL-A lipase superfamily:

**TABLE 1**

**IDENTITY BETWEEN THE DIFFERENT LIPASES OF THE CAL-A SUPERFAMILY**

| Identity (%) | UniProt_ Accession | CAL-A | lipK | lipUMf | SpoRei_E7 | SpoRei_E6 |
|---|---|---|---|---|---|---|
| **CAL-A** | D4PHA8 | 100% | 75% | 67% | 66% | 57% |
| **lipK** | Q8NIP2 | 75% | 100% | 66% | 65% | 55% |
| **lipUMf** | Q4P903 | 67% | 66% | 100% | 68% | 57% |
| **SpoRei_E7** | E7A3D1 | 66% | 65% | 68% | 100% | 60% |
| **SpoRei_E6** | E6ZNA3 | 57% | 55% | 57% | 60% | 100% |

The following amino acid sequence is unique to the CAL-A superfamily, *i.e.* sequences with more than 50% homology: (Q/E)ASY(T/A)V (SEQ ID NO:1), wherein the first position in this unique sequence may be occupied by glutamine or glutamic acid and the fifth position may be occupied by threonine or alanine. Lipases not belonging to the CAL-A superfamily (<50% homology) show a gap at this position, when aligned. Table 2 shows the unique amino acid sequence for members of the CAL-A superfamily when in their pro-forms:

**TABLE 2**

**AMINO ACID SEQUENCE FOR IDENTIFYING MEMBERS OF THE CAL-A SUPERFAMILY**

| Pro-Form/Amino Acid Position | Amino Acid Sequence |
|---|---|
| pro-CAL-A/312-320 | **QAEASYTVS** (SEQ ID NO:2) |
| pro-lip_K/308-316 | **QSEASYAVP** (SEQ ID NO:3) |
| pro-UMf/432-440 | **QAEASYTVP** (SEQ ID NO:4) |
| pro-SpoRei_E7/308-316 | **QTQASYTVP** (SEQ ID NO:5) |
| pro-SpoRei_E6/309-317 | **QAEASYTVP** (SEQ ID NO:6) |

In one embodiment, the parent lipase is a *Pseudozyma* (formerly *Candida*)-derived lipase, with *P. antartica* CAL-A-derived lipase variants being particularly useful in the practice of making lipase variants with improved enzymatic properties.

Alternatively, the parent lipase is an *Ustilago maydis* derived lipase, with *U. maydis* LipUM-derived variant polypeptides being particularly useful. In illustrative embodiments, the parent lipase comprises, consists essentially of, or alternatively, consists of, an amino acid sequence that is at least 98% identical to the amino acid sequence set forth in SEQ ID NO:7 (*U. maydis* LipUM; Uniprot ID No. Q4P903):

MWGRIRNVIQPTWAPPLFGTLNIIFSLFFRAGIARSHKWTWCCYRPTRM

ARSRTFSNSAPTRRRPERLRLQKGSSNTTIRPRPSAILPDEMNHGSLLTVVPHTVVA
STPSFRSSFPDSLIASVQMRFIAVRAIVTLAAAAAVSLAVPTERRAAFADPNDDLFY
TTPDNINTYANGQVIQSRKADTDIGNSNKVEAFQLQYRTTNTQKEAQANVATVWI
PNKPASPPKIFSYQVYQDSTQLNCAPSYSFLKGLDKPNKATTILEAPIIGWALQQGF
YVVSSDHEGPRSSFIAGYEEGMAILDGIRALKNYAKLPTDSAIGFYGYSGGAHATG
WAANLAGSYAPEHNIIGAAYGGLPASARDTFNFLNKGAFAGFAIAGVSGLALAYP
DVETYIQSRLNAKGEKVFKQVRSRGFCIGQVVLTYPFVDAYSLINDTNLLNEEPVA
STLKSETLVQAEASYTVPVPKFPRFIWHALLDEIVPFHSAATYVKEQCSKGADINW
NVYSFAEHISAELFGLLPGLDWLNKAYKGQAPKVPCGGGAQSVMGASGPPAQDV
LGADLASQLRSLQGKPSAFGNKPFGSISP (SEQ ID NO:7)

The lipase variants of the present invention may typically be prepared by one or more specific modification of the parent lipase from which the polypeptide sequence was derived. For example, a "CAL-A lipase variant," is a polypeptide that possesses lipolytic activity, but is altered in its primary amino acid sequence at one or more amino acid residues of the polypeptide sequence of native, wild-type, or the "parent" CAL-A lipase. Such variants can differ in amino acid sequence from its parent lipase by one or more amino acid substitutions, one or more amino acid deletions, one or more amino acid insertions, or may be a truncated polypeptide, a polypeptide fusion, or any combination of such alterations. The resulting lipases - termed "variants," are therefore modified lipase proteins, that retain at least a portion of the enzymatic activity associated with the wild-type sequence from which the variant was obtained. The variant polypeptides prepared according to the present invention may include one or more additional modifications, including, for example, one or more post-translational changes, as compared to the wild-type. The variant lipases prepared according to the invention are preferably fully functional, but may also lack function in one or more activities associated with the parent lipase, or, alternatively, may perform one or more additional activities, or any combination thereof. In particular aspects of the invention, the polypeptide variants display an increased preference for catalysis of medium- and/or long-chain fatty acid moieties, and/or an enhanced *trans*-fatty acid selectivity.

In the disclosure the parent lipase may be a lipase with an amino acid sequence having at least about 90% to about 99.9% homology to the sequence of one or more of the lipase or lipase variants described and listed herein. In another part of the disclosure there is at least about a 94% to about 99.5% homology to the amino acid sequence of one or more of the lipase or lipase variants described and listed herein. In yet another part of the disclosure, there is at least about an 96% to about a 99% homology to the amino acid sequence of one or more of the lipase or lipase variants described and listed herein.

As known to those of ordinary skill in the art, polypeptide and/or polynucleotide sequence comparisons, alignments and calculations of percent homology may be readily performed, to determine the percent similarity, percent homology, and/o percent identity between two or more sequences. Nucleic acid sequences, such as deoxyribonucleic acid (DNA) and ribonucleic acid (RNA), either synthetically or naturally occurring, that encodes the lipase, or variant thereof, of the present invention are also within the scope of the present invention.

Lipase polynucleotides can include nucleic acids including RNA, such as mRNA, DNA, including cDNA and genomic DNA, and can be either single or double stranded. The lipase polynucleotides include, but are not limited to, the sequence encoding the mature parent polypeptides alone, or variants thereof, including fragments, the sequence encoding the mature polypeptide, or variants thereof, including additional coding sequences, such as a leader or secretory sequence (*e.g.* a pre-pro or pro-protein sequence), the sequence encoding the mature parent polypeptide, or variants thereof, with or without additional coding sequences, with additional non-coding sequences (*e.g.*, introns, transcribed but not translated sequences, mRNA processing sequences, ribosome binding sequences and stability sequences for mRNA). Additionally, the nucleotide sequence may be fused to a marker sequence. In one embodiment, the lipase nucleic acids include only the coding regions. The nucleotide sequence may encompass fragments of the entire sequence of the respective variant lipases and the sequence may differ than those described due to degeneracy of the genetic code.

For example, a parent lipase polynucleotide includes the following DNA sequence, which encodes the amino acid sequence of SEQ ID NO:7:

ATGTGGGGGCGCATCCGCAACGTTATTCAGCCAACTTGGGCGCCGCC
GTTATTTGGCACCCTGAATATCATTTTTAGCCTTTTTTTCCGTGCCGGGATTGCA
AGGTCGCACAAATGGACATGGTGCTGCTACAGACCGACTCGAATGGCCAGAA
GCCGCACATTCTCGAATTCGGCTCCAACCAGACGGCGGCCCGAACGATTACGG
TTGCAGAAGGGTTCGTCTAATACTACCATTCGCCCGCGCCTTCGGCTATTTTG
CCTGACGAGATGAACCATGGCTCGCTGCTACGGTTGTCCCGCACACTGTAGTC
GCCTCCACCCCTCCTTTCGTTCTTCCTTTCCAGATTCGTTGATCGCCTCGGTTC
AGATGAGGTTCATTGCTGTTCGGGCTATCGTGACGCTAGCGGCTGCAGCCGCC
GTGTCGCTTGCAGTGCCCACAGAGCGAAGGGCAGCGTTCGCCGATCCAAACGA
CGATCTCTTCTACACCACGCCGGACAACATCAACACATATGCCAATGGTCAGG
TCATCCAGTCACGCAAGGCTGATACCGATATTGGGAACAGCAACAAGGTTGAA
GCTTTCCAGCTTCAATATCGCACTACCAATACGCAAAAGGAGGCGCAGGCCAA
CGTTGCTACCGTATGGATCCCCAACAAGCCCGCTTCACCTCCCAAGATCTTCTC
TTATCAGGTCTATCAGGACTCGACACAGCTCAACTGTGCTCCGAGCTATAGCTT
TTTGAAGGGCCTTGACAAGCCTAACAAAGCTACCACGATCCTCGAAGCACCCA
TCATCATCGGCTGGGCGCTCCAACAAGGTTTCTACGTCGTCTCGTCTGATCACG
AAGGCCCGCGCTCATCGTTCATTGCGGGCTACGAGGAAGGTATGGCTATTCTC
GACGGCATACGTGCGCTCAAGAACTACGCCAAACTGCCCACGGACAGCGCGAT
CGGCTTTTACGGATACAGCGGCGGTGCCCATGCAACCGGCTGGGCAGCTAATC
TGGCAGGGAGCTACGCTCCTGAGCACAACATCATCGGTGCTGCCTACGGAGGA
CTGCCTGCTAGCGCCAGAGACACATTCAACTTCCTCAACAAAGGCGCGTTTGC
CGGCTTCGCCATTGCGGGTGTCTCGGGTCTTGCGCTGGCCTACCCGGACGTGGA
GACCTACATCCAGTCGCGCCTCAACGCCAAGGGAGAAAAGGTGTTTAAACAGG
TCCGAAGTCGCGGCTTCTGCATTGGCCAAGTGGTCCTAACCTACCCATTCGTCG
ACGCCTATTCACTCATCAACGACACAAACCTTCTCAACGAGGAACCGGTCGCC
AGCACGTTGAAATCCGAGACGTTGGTTCAGGCCGAGGCTAGCTACACGGTTCC

TGTTCCCAAATTCCCGCGTTTCATCTGGCATGCGCTCTTGGACGAGATTGTTCC
CTTCCACTCGGCTGCGACCTATGTCAAGGAGCAGTGTTCAAAGGGCGCCGACA
TCAACTGGAATGTCTACTCATTTGCCGAGCACATCTCTGCCGAGCTTTTCGGCT
TGCTGCCTGGTCTCGACTGGTTAAACAAGGCTTACAAGGGTCAAGCACCCAAA
GTGCCTTGTGGCGGAGGGGCTCAAAGCGTGATGGGTGCCTCAGGCCCGCCTGC
GCAGGACGTTCTGGGAGCTGACCTGGCAAGCCAACTCCGATCTCTCCAGGGTA
AGCCTTCTGCGTTTGGCAACAAACCTTTTGGCTCCATCTCCCCCTGA
(SEQ ID NO:8).

In another embodiment, the parent lipase is a lipase precursor found in *Sporisorium relianum*, *i.e.,* SpoRei_E7. In another embodiment, the parent lipase is a sequence essentially as set forth in SEQ ID NO:9 which contains the following sequence (hereinafter referred to as the amino acid sequence or sequence of "SpoRei_E7") (Uniprot E7A3D1):

MRFFAQTLVALAAAATVSLAAPLERRAQFPDPNDDPFYSAPANIGSYVNGQVIQS
RSATTDIGTSNNAASFQLLYRTTNTSNLPEATVATVWIPAKPASPPKIFSYQVYEDA
TQLNCAPSYSYLSGLDEPGKGTVILDTPIVISWALQQGYYVVSADHEGPKAAFIAG
CQEGRAILDGVRALRNFQNLASNSAVGFYGYSGGGHATGWAVSLAGSYAPDVNII
GAAYGGVPTSTRDIFNFLNGGAFAGFAVAGVSGLGLAYPELEAYIEPRLNAKGQD
ALKRFRSRGYCIGQVVTSENFVDIYTLVNDSNILNEPIPSQVLAKETLLQTQASYTV
PVPKSPRFIWHALEDEIVPFKPAQQYVTEQCAKGANINWNVFPIAEHISAELFGLVP
GLDWLSKAYRGQAPKVICGSSIPAITGVNSPSAQQVLGADLAQQLSNLNGKQSAF

GKPYGPITPPTA (SEQ ID NO:9),

or a lipase variant thereof defined herein.

In part of the disclosure the parent lipase may be a lipase with an amino acid sequence having at least about 80% to about 99.9% homology to the sequence of the lipase shown in SEQ ID NO:9. In another part of the disclosure, there is at least about a 85% to about 99.5% homology to the amino acid sequence of the lipase as shown in SEQ ID NO:9. In a yet another part of the disclosure, there is at least about a 90% to about an 99% homology to SEQ ID NO:9.

In another embodiment, the parent lipase is another lipase precursor found in *Sporisorium relianum, i.e.,* SpoRei_E6. In another embodiment, the parent lipase is a sequence essentially as set forth in SEQ ID NO: 10 which contains the following sequence (hereinafter referred to as the amino acid sequence or sequence of "SpoRei_E6") (Uniprot E6ZNA3):

MRFSPQNWIALAIAATIVVAAPNPQKNKRAPFPDPNDDDFYKNPANITSYSNGEIIR
SRKAATDLGNLNNVDSFQLSYRTTNTQNEAQANVATVWLPAKPASPPKIFFVQLW

EDSTQLNCAPSYNYLTGLDQPNKVPATIDTLVIVSWALQQGYYVVSSDHAGPRSA
FIGGWENGMAVLDGVRALKHYKSLPSDSAVGFYGYSGGGHATARAVNLQESYAP
ELNVVAAAYGGVPTSALLLFLNKGFVAGFVVAGLSGISLAYPEVEAYLKPRLTAR
GQQALAQVQSRDFCVANVVTHNNFVDVFSLVNQNDFLDQDPVRQVIARETLLQA
EASYTVPVPRFPRFMWHGNIDEIVPFIPDADYVKEQCRKGANINWNVLPTGHAG
AEIVGLVPALDWMGKVFRGQTPKVSCGNGVPAISGINSPSAKQVLGSDLAKRLEE

LKSPQSSLVKLFSGVAPS (SEQ ID NO:10),

or a lipase variant thereof defined herein.

In a part of the disclosure, the parent lipase may be a lipase with an amino acid sequence having at least about 80% to about 99.9% homology to the sequence of the lipase shown in SEQ ID NO: 10. In another part of the disclosure there is at least about a 90% to about 99% homology to the amino acid sequence of the lipase as shown in SEQ ID NO:10. In a yet another part of the disclosure there is at least about a 93% to about an 97% homology to SEQ ID NO: 10.

In one embodiment, the parent lipase is a putative wild-type lipase A from *Pseudozyma antartica* (*i.e.,* CAL-A) (Protein Data Bank (pdb) entry 2veo). In another embodiment, the parent lipase is a sequence essentially as set forth in SEQ ID NO: 11 which contains the following amino acid sequence:

MRVSLRSITSLLAAATAAVLAAPAAETLDRRAALPNPYDDPFYTTPSNIGTFAKGQ
VIQSRKVPTDIGNANNAASFQLQYRTTNTQNEAVADVATVWIPAKPASPPKIFSYQ
VYEDATALDCAPSYSYLTGLDQPNKVTAVLDTPIIIGWALQQGYYVVSSDHEGFK
AAFIAGYEEGMAILDGIRALKNYQNLPSDSKVALEGYSGGAHATVWATSLAESYA
PELNIVGASHGGTPVSAKDTFTFLNGGPFAGFALAGVSGLSLAHPDMESFIEARLN
AKGQRTLKQIRGRGFCLPQVVLTYPFLNVFSLVNDTNLLNEAPIASILKQETVVQA
EASYTVSVPKFPRFIWHAIPDEIVPYQPAATYVKEQCAKGANINFSPYPIAEHLTAEI
FGLVPSLWFIKQAFDGTTPKVICGTPIPAIAGITTPSADQVLGSDLANQLRSLDGKQ
SAFGKPFGPITPP (SEQ ID NO:11).

Variants of these sequences are also within the scope of the invention. The expression, sequence and cloning methods as per this sequence have been described by Hoegh *et al.* (1995). In a part of the disclosure, the parent lipase may be a lipase with an amino acid sequence having at least about 60% to about 99.9% homology to the sequence of the lipase shown in SEQ ID NO: 11. In another part of the disclosure, there is at least about a 70% to about 99.5% homology to the amino acid sequence of the lipase as shown in SEQ ID NO: 11. In an yet another part of the disclosure, there is at least about a 80% to about an 99% homology to SEQ ID NO: 11.

In another embodiment, a parent lipase polynucleotide includes the following DNA sequence, which encodes for the amino acid sequence of SEQ ID NO: 11:

ATGGCGGCGCTGCCCAACCCCTACGACGATCCCTTCTACACGACGCCATCCAA
CATCGGCACGTTTGCCAAGGGCCAGGTGATCCAATCTCGCAAGGTGCCCACGG
ACATCGGCAACGCCAACAACGCTGCGTCGTTCCAGCTGCAGTACCGCACCACC
AATACGCAGAACGAGGCGGTGGCCGACGTGGCCACCGTGTGGATCCCGGCCA
AGCCCGCTTCGCCGCCCAAGATCTTTTCGTACCAGGTCTACGAGGATGCCACG
GCGCTCGACTGTGCTCCGAGCTACAGCTACCTCACTGGATTGGACCAGCCGAA
CAAGGTGACGGCGGTGCTCGACACGCCCATCATCATCGGCTGGGCGCTGCAGC
AGGGCTACTACGTCGTCTCGTCCGACCACGAAGGCTTCAAAGCCGCCTTCATC
GCTGGCTACGAAGAGGGCATGGCTATCCTCGACGGCATCCGCGCGCTCAAGAA
CTACCAGAACCTGCCATCCGACAGCAAGGTCGCTCTTGAGGGCTACAGTGGCG
GAGCTCACGCCACCGTGTGGGCGACTTCGCTTGCTGAATCGTACGCGCCCGAG
CTCAACATTGTCGGTGCTTCGCACGGCGGCACGCCCGTGAGCGCCAAGGACAC
CTTTACATTCCTCAACGGCGGACCCTTCGCCGGCTTTGCCCTGGCGGGTGTTTC
GGGTCTCTCGCTCGCTCATCCTGATATGGAGAGCTTCATTGAGGCCCGATTGAA
CGCCAAGGGTCAGCGGACGCTCAAGCAGATCCGCGGCCGTGGCTTCTGCCTGC
CGCAGGTGGTGTTGACCTACCCCTTCCTCAACGTCTTCTCGCTGGTCAACGACA
CGAACCTGCTGAATGAGGCGCCGATCGCTAGCATCCTCAAGCAGGAGACTGTG
GTCCAGGCCGAAGCGAGCTACACGGTATCGGTGCCCAAGTTCCCGCGCTTCAT
CTGGCATGCGATCCCCGACGAGATCGTGCCGTACCAGCCTGCGGCTACCTACG
TCAAGGAGCAATGTGCCAAGGGCGCCAACATCAATTTTTCGCCCTACCCGATC
GCCGAGCACCTCACCGCCGAGATCTTTGGTCTGGTGCCTAGCCTGTGGTTTATC
AAGCAAGCCTTCGACGGCACCACACCCAAGGTGATCTGCGGCACTCCCATCCC
TGCTATCGCTGGCATCACCACGCCCTCGGCGGACCAAGTGCTGGGTTCGGACC
TGGCCAACCAGCTGCGCAGCCTCGACGGCAAGCAGAGTGCGTTCGGCAAGCCC
TTTGGCCCCATCACACCACCTTAG (SEQ ID NO:12).

In SEQ ID NO: 12, both the pre-sequence (signal sequence) and the pro-sequence have been removed to form the mature form of CAL-A (mCAL-A), which is potentially the secreted form of CAL-A by *Pseudozyma antartica.* A methionine has been added at the beginning of the sequence.

In one embodiment, the lipase variants increase the *trans*-selectivity of the parent lipase. For example, the lipase variants act on or show a preference for *trans*-fatty acid esters or carboxylic acid moieties. In another embodiment, the lipase variants demonstrate a preference for medium chain fatty acid esters or carboxylic acid moieties with a chain length less than $C_{12}$. As used herein, the term "medium chain fatty acid" refers to a saturated or unsaturated fatty acid containing about 6 to about 12 carbon atoms, unless otherwise specified. Examples of medium chain fatty acid include caproic acid, heptanoic acid, caprylic acid, nonanoic acid, capric acid, undecanoic acid, lauric acid, and the like. Medium chain length fatty acids are major components of such seed oils such as coconut oil, palm kernel oil and *Cuphea* species. Reduced chain length means that medium chain length fatty acids are more rapidly absorbed by the body and more quickly metabolized than long chain length fatty acids. In yet another embodiment, the lipase variants have both, act on or show a preference for *trans*-fatty acid esters or carboxylic acid moieties and act on or show a preference for medium chain fatty acid esters or carboxylic acid moieties. In other embodiments, the lipase variants demonstrate a preference for long-chain fatty acid esters or carboxylic acid moieties with a chain length greater than or equal to $C_{12}$. As used herein, the term "long- chain fatty acid" refers to a saturated or unsaturated fatty acid containing about 12 carbon atoms or more, unless otherwise specified.

In describing some lipase variants, the following standard protein mutational nomenclature is used for ease of reference: $N_1$###$N_2$, where $N_1$ = single-letter abbreviation of original amino acid, ### = amino acid position, and $N_2$ = single-letter abbreviation of the substituted amino acid. For example, according to this nomenclature, the substitution of phenylalanine for aspartic acid in position 145 is shown as F145D.

In one embodiment, substitution of at least a portion of the amino acid sequence of CAL-A may occur so as to increase the *trans*-selectivity of the lipase encoded by the CAL-A sequence and/or act on or show a preference for long- or medium- chain fatty acid esters or carboxylic acid moieties. For example, the amino acid sequence of CAL-A may be altered or mutated to increase access of a substrate to the active site or binding tunnel of the lipase. Preferably, in the case of hydrolysis of unsaturated triacylglycerides, the location of the *cis*- or *trans*- double bond is not placed into close contact with the active site.

In describing some lipase variants, the following nomenclature is used for ease of reference: multiple mutations are separated by plus signs, *i.e.,* F145D+T217H, representing a double mutation that includes a phenylalanine-to-aspartate substitution at amino acid residue 145, and a threonine-to-histidine substitution at amino acid residue 214.

In one embodiment, substitution of at least a portion of the amino acid sequence of CAL-A may occur so as to increase *trans*-selectivity of the lipase encoded by the CAL-A sequence and/or act on or show a preference for long- or medium- chain fatty acid esters or carboxylic acid moieties.

Some particular substitutions within the CAL-A sequence were chosen based on the residues that were facing the model substrate in the acyl or fatty acid binding tunnel of CAL-A in homology and structural analysis. These can be separated into those residues that are close to the active site, such as phenylalanine at amino acid residue 149 (F149) and I150 and those that form part of the unique lid structure, for example, A218, T221, F222, L225, F233, G237, L241, M248, I301 and L305. Residues A218, T221 F222 and L225 are found within the A5 α-helix as depicted in **FIG. 2.** Residues F223, G237, and L241 are found within the A6 α-helix as depicted in **FIG. 2.** Residue M248 is found within the A7 α-helix and residues I301 and L305 are found within the A10 α-helix. It will be understood by those of ordinary skill in the art that any amino acid may be substituted into these positions within the CAL-A sequence. Some particular substitutions within the CAL-A sequence are, for example, F222S, F222C, F149D, F149G, F149M, F149T, F149N, F149V, I301H, I301R, I301A, T221I, T221H, L305F, L305N, L305Y, L305C, L305Q, F223R, L225R, A218N, G237A, G237L, G237V, G237Y, G237R, G237W, I150K, I150H, I150R, I150T, I150E, I150V, L241A, L241N, M248V and L305A, or a combination thereof where the substitutions do not overlap with one another. Particular substitutions can be selected from the group of: F222S, F222C, F222H, F149D, I301H, T221I, T221H, L305F, L305N, A218N, G237A, G237L, G237V, and G237Y. The lipase variant may optionally include substitutions of one or more amino acids. Such substitutions may be made according to principles known to one of ordinary skill in the art.

In one embodiment, substitution of at least a portion of the amino acid sequence of the lipUMf may occur so as to increase the *trans*-selectivity of the lipase encoded by the lipUMf sequence and/or act on or show a preference for long chain fatty acid esters or carboxylic acid moieties. In one embodiment, portions of the lipUMf sequence may be substituted for portions of the CAL-A sequence. For example, the sequences encoding the lid structures may be exchanged. In another embodiment, portions of the CAL-A sequence may be substituted for portions of the lipUMf sequences. For example, the amino acid sequence of lipUMf may be altered or mutated to increase access of a substrate to the active site or binding tunnel of the lipase. Preferably, these alterations or mutations occur in either the predicted lid structure and/or C-terminal flap of CAL-A or lipUMf. More specifically, amino acid residues at about position 441 through about position 449 of lipUMf may be substituted with the amino acid residues at about position 300 through about position 308 in SEQ ID NO:11, *i.e.* substitution of the amino acid sequence PVASTLKSE (SEQ ID NO:109) in SEQ ID NO:7 for the amino acid sequence LTYPFLNVF (SEQ ID NO:110), or *vice versa.* Alternatively, or in addition to this substitution, the C-terminal flap portion of CAL-A or lipUMf sequence may be cleaved, mutated or altered. More specifically, any amino acid residue at about position 545 to about position 586 in SEQ ID NO: 11 can be substituted with a STOP codon. In addition, any amino acid residue at about position 541 to about position 582 in SEQ ID NO:7 can be substituted with a STOP codon (denoted by '*'). For example, possible mutations within SEQ ID NO:7 may include a truncation of the sequence at amino acid 543 with a stop codon (S541*), V542*, M543*, G544*, A545*, S546*, G547*, P548*, P549*, A550*, Q551*, D552*, V553*, L554*, G555*, A556*, D557*, L558*, A559*, S560*, Q561*, L562*, R563*, S564*, L565*, Q566*, G567*, K568*, P569*, S570*, A571*, F572*, G573*, N574*, K575*, P576*, F577*, G578*, S579*, I580*, S581* and/or P582*.

In describing some lipase variants, the following nomenclature is used for ease of reference: symbol of first amino acid deleted; numerical position of first amino acid deleted _ symbol of last amino acid deleted; numerical position of last amino acid deleted; del. For example, according to this nomenclature and referring to the sequence of SEQ ID NO:7, the deletion of methionine at position 1 through glutamine at position 124 would result in the following notation:

M1_Q124del. If only one amino acid is deleted, this would result in only one amino acid and its position identified in the notation. For example, a deletion of methionine at position 125 in SEQ ID NO:7 would result in the following notation: M125del. In all cases, the accepted IUPAC single letter or triple letter amino acid abbreviation is employed. All amino acid substitutions are numbered beginning with the N-terminus of the processed form of the sequence. For example, the amino acid sequence numbering starts at the N-terminus of the protein without the actual and/or proposed signal sequence in the case of secreted proteins.

In one part of the disclosure the lipase variant includes one or more deletions of the N-terminus of the *Ustilago maydis* lipase. In a part of the disclosure the N-terminus deletion of the lipase sequence maintains and/or increases the lipolytic activity of the lipase when compared to lipUMf. In another part of the disclosure, the N-terminus deletion of the lipase sequence increases the ability of the resultant recombinant protein to be secreted from a host system. For example, in some parts of the disclosure, removal of the N-terminus increases the movement of the lipase variant into the periplasmic space of an organism such as bacteria or yeast. In other parts of the disclosure removal of the N-terminus increases the movement of the lipase variant outside of the host organism itself. The N-terminus accounts for about 0.01% to about 30% of the full length amino acid sequence, about 5% to about 25% of the full length amino acid sequence, or about 10% to about 20% of the full length amino acid sequence.

Alternatively, the N-terminus can account for up to about the first 175 amino acid residues of SEQ ID NO:7, up to about the first 146 residues of SEQ ID NO:7, or up to about the first 117 amino acid residues of SEQ ID NO:1. Deletions can be of any length and can take place at any point along the N-terminus of the amino acid sequence of SEQ ID NO:7, and new translation starting or initiation sites can be added to the resulting or remaining sequence as necessary. In one part of the disclosure the amino acids at about position M1 to about position A34 are removed from the parent lipase of SEQ ID NO:7 (*i.e.* a M1_A34del mutant). In part of the disclosure the amino acids at about position M1 to about position Q124 are removed from the parent lipase of SEQ ID NO:7 (*i.e.* a M1_Q124del mutant). In another embodiment, the lipase variant's sequence essentially as set forth in SEQ ID NO: 13 (hereinafter referred to as the amino acid sequence or sequence of "LipUMs") :

MRFIAVRAIVTLAAAAAVSLAVPTERRAAFADPNDDLFYTTPDNINTYANGQVIQS
RKADTDIGNSNKVEAFQLQYRTTNTQKEAQANVATVWIPNKPASPPKIFSYQVYQ
DSTQLNCAPSYSFLKGLDKPNKATTILEAPIIIGWALQQGFYVVSSDHEGPRSSFIAG
YEEGMAILDGIRALKNYAKLPTDSAIGFYGYSGGAHATGWAANLAGSYAPEHNII
GAAYGGLPASARDTFNFLNKGAFAGFAIAGVSGLALAYPDVETYIQSRLNAKGEK
VFKQVRSRGFCIGQVVLTYPFVDAYSLINDTNLLNEEPVASTLKSETLVQAEASYT
VPVPKFPRFIWHALLDEIVPFHSAATYVKEQCSKGADINWNVYSFAEHISAELFGL
LPGLDWLNKAYKGQAPKVPCGGGAQSVMGASGPPAQDVLGADLASQLRSLQGK

PSAFGNKPFGSISP (SEQ ID NO:13),

or a lipase variant thereof defined herein.

In one embodiment, a lipUMs DNA polynucleotide sequence encompasses the following sequence:

ATGAGGTTCATTGCTGTTCGGGCTATCGTGACGCTAGCGGCTGCAGCCGCCGT
GTCGCTTGCAGTGCCCACAGAGCGAAGGGCAGCGTTCGCCGATCCAAACGACG
ATCTCTTCTACACCACGCCGGACAACATCAACACATATGCCAATGGTCAGGTC
ATCCAGTCACGCAAGGCTGATACCGATATTGGGAACAGCAACAAGGTTGAAGC
TTTCCAGCTTCAATATCGCACTACCAATACGCAAAAGGAGGCGCAGGCCAACG
TTGCTACCGTATGGATCCCCAACAAGCCCGCTTCACCTCCCAAGATCTTCTCTT
ATCAGGTCTATCAGGACTCGACACAGCTCAACTGTGCTCCGAGCTATAGCTTTT
TGAAGGGCCTTGACAAGCCTAACAAAGCTACCACGATCCTCGAAGCACCCATC
ATCATCGGCTGGGCGCTCCAACAAGGTTTCTACGTCGTCTCGTCTGATCACGAA
GGCCCGCGCTCATCGTTCATTGCGGGCTACGAGGAAGGTATGGCTATTCTCGA

CGGCATACGTGCGCTCAAGAACTACGCCAAACTGCCCACGGACAGCGCGATCG
GCTTTTACGGATACAGCGGCGGTGCCCATGCAACCGGCTGGGCAGCTAATCTG
GCAGGGAGCTACGCTCCTGAGCACAACATCATCGGTGCTGCCTACGGAGGACT
GCCTGCTAGCGCCAGAGACACATTCAACTTCCTCAACAAAGGCGCGTTTGCCG
GCTTCGCCATTGCGGGTGTCTCGGGTCTTGCGCTGGCCTACCCGGACGTGGAG
ACCTACATCCAGTCGCGCCTCAACGCCAAGGGAGAAAAGGTGTTTAAACAGGT
CCGAAGTCGCGGCTTCTGCATTGGCCAAGTGGTCCTAACCTACCCATTCGTCGA
CGCCTATTCACTCATCAACGACACAAACCTTCTCAACGAGGAACCGGTCGCCA
GCACGTTGAAATCCGAGACGTTGGTTCAGGCCGAGGCTAGCTACACGGTTCCT
GTTCCCAAATTCCCGCGTTTCATCTGGCATGCGCTCTTGGACGAGATTGTTCCC
TTCCACTCGGCTGCGACCTATGTCAAGGAGCAGTGTTCAAAGGGCGCCGACAT
CAACTGGAATGTCTACTCATTTGCCGAGCACATCTCTGCCGAGCTTTTCGGCTT
GCTGCCTGGTCTCGACTGGTTAAACAAGGCTTACAAGGGTCAAGCACCCAAAG
TGCCTTGTGGCGGAGGGGCTCAAAGCGTGATGGGTGCCTCAGGCCCGCCTGCG
CAGGACGTTCTGGGAGCTGACCTGGCAAGCCAACTCCGATCTCTCCAGGGTAA
GCCTTCTGCGTTTGGCAACAAACCTTTTGGCTCCATCTCCCCCTGA
(SEQ ID NO:14).

In another part of the disclosure the deletion of the N-terminus may start anywhere from about position M1 to about A34 and may end around amino acid Q124. In yet another part of the disclosure the deletion of the N-terminus of SEQ ID NO:7 begins at about position M1 and ends around position A145 (*i.e.* a M1_A145del mutant). In an inllustrative embodiment, the lipase variant's modified amino acid sequence is set forth in SEQ ID NO: 15.

MVPTERRAAFADPNDDLFYTTPDNINTYANGQVIQSRKADTDIGNSNKVEAFQLQ
YRTTNTQKEAQANVATVWIPNKPASPPKIFSYQVYQDSTQLNCAPSYSFLKGLDKP
NKATTILEAPIIIGWALQQGFYVVSSDHEGPRSSFIAGYEEGMAILDGIRALKNYAK
LPTDSAIGFYGYSGGAHATGWAANLAGSYAPEHNIIGAAYGGLPASARDTFNFLN
KGAFAGFAIAGVSGLALAYPDVETYIQSRLNAKGEKVFKQVRSRGFCIGQVVLTY
PFVDAYSLINDTNLLNEEPVASTLKSETLVQAEASYTVPVPKFPRFIWHALLDEIVP
FHSAATYVKEQCSKGADINWNVYSFAEHISAELFGLLPGLDWLNKAYKGQAPKV
PCGGGAQSVMGASGPPAQDVLGADLASQLRLQGKPSAFGNKPFGSISP

(SEQ ID NO:15),

or a lipase variant thereof defined herein. In this instance, a starting sequence, such as methionine, has been added to the lipase variant encompassed by or essentially is set forth in SEQ NO:15.

In another part of the disclosure the lipase variants include at least one or more deletions of the N-terminus of the *Ustilago maydis* lipase and removal, mutation or cleavage of the C-terminus sequence, which substantially comprises the flap structure of the lipase. This includes substitutions or frame insertions of a stop codon anywhere between about position 395 to about position 408 in the lipUMs sequence and/or between about position 251 to about position 264 in the sequence encompassed by SEQ ID NO:15. The disclosure also encompasses these two sequence removals, plus any other mutation mentioned.

In a part of the disclosure the length of a lipase variant sequence aligned for comparison to the parent lipase is at least 50%, at least 60%, at least 70%, at least 80%, or at least 90% the length of the parent lipase sequence. In other parts of the disclosure the alignment is to about 95%, 98% or 99% of the length of the parent lipase sequence, *i.e.* SEQ ID NO:7, SEQ ID NO:9, SEQ ID NO:10, or SEQ ID NO:11. Substantial homology or similarity can also be to the entire nucleic acid or amino acid sequence of the parent lipase or to fragments of these sequences. Some parts of the disclosure thus also encompass polypeptide fragments of the variant lipases. Some parts of the disclosure encompass polypeptides having a lower degree of homology but having sufficient similarity so as to perform one or more of the same functions performed by the lipase parent, or variant thereof.

Fragments can retain one or more of the biological activities of the protein and can comprise a domain or motif such as a catalytic site, active site, transmembrane domain, etc. Fragments can also be fused to create chimeric or fusion proteins. These can include a lipase sequence operably linked to another sequence not substantially homologous to the lipase. Such fusion proteins, for example, can facilitate the purification of a recombinant lipase protein or can enable secretion from certain host cells. Additionally, chimeric lipase proteins can be produced where one or more functional sites is derived from a different lipase member. In another part of the disclosure and without being bound by any theory, the entire proposed sequence of the CAL-A that correlates to a proposed A10 helix structure, corresponding to essentially amino acid position 300 to about position 308, is substituted for the helix of another lipase, for example the A10 helix of lipUM. In another part of the disclosure and without being bound by any theory, the entire proposed sequence of the lipUMs that correlates to a proposed A10 helix structure, corresponding to essentially amino acid position 296 to about position 304, is substituted for with the helix of another lipase, for example the A10 helix of CAL-A. The same helix exchange can occur in the sequence encompassed by SEQ ID NO:13 or SEQIDNO:15, in that amino acids at essentially positions 152 through 160 can be substituted for the A10 helix of CAL-A. The lipase variant may include additional substitutions as listed above, that are not present in the A10 helix of CAL-A or SEQ ID NO:15 sequence as listed.

Such chimeric or fusion proteins are known to those of ordinary skill in the art, and methods for preparing and obtaining such proteins can also be accomplished using one or more published methods that are well known to those of ordinary skill.

Certain embodiments also provide for nucleic acid sequences encoding the variant lipases of other embodiments, expression vectors harboring the nucleic acid sequences and transformed host cells containing the nucleic acid sequences and/or the expression vectors. The sequences of the parent lipase or lipase variants can be aligned with heterologous sequences, such as promoters, enhancers, transcriptional control elements, etc. in order to amplify or diminish their expression, as well as the expression of any co-factors.

Nucleic acid sequences, such as DNA, RNA, or cDNA, encoding the parent or variant lipases as defined herein may be isolated from any cell or organism producing them, by methods well known to those of ordinary skill in the art. Suitable nucleic acid sequences can be obtained by back-translation of the polypeptide sequence according to the genetic code. The codons used are preferably those frequently used in accordance with the usage within the specific organism, for example, *E. coli* or *P. pastoris.* Nucleic acid constructs containing the nucleic acid(s) of the present invention are also within the scope of the invention. Often, these nucleic acid constructs will also contain regulatory sequences for expression, replication and/or recombination, in addition to the nucleic acid sequences encoding the parent or variant lipases.

Lipase variants can be naturally occurring or can be made by recombinant means or chemical synthesis to provide the lipase polypeptide with useful characteristics. Useful variations can include, but are not limited to, increased hydrolysis or alcoholysis, *i.e.,* ethanolysis, when in contact with a substrate, increased binding to a substrate, altered affinity for other molecules such as co-factors, recognition of a fatty acid moiety that is normally not recognized by the enzyme, increased preference for *trans*-fatty acids, increased preference for fatty acid substrates having fatty acid chain lengths greater than or equal to $C_6$, greater than or equal to $C_8$, greater than or equal to $C_{10}$, greater than or equal to $C_{12}$, greater than or equal to $C_{14}$, greater than or equal to $C_{16}$, greater than or equal to $C_{18}$, greater than or equal to $C_{20}$, greater than or equal to $C_{22}$, *etc.*

Host organisms that can be transformed by using the nucleic acid(s) of the present invention are also within the scope of the invention. Host organisms include, but are not limited to, unicellular or multicellular organisms, and preferably microorganisms. Cloning of the DNA sequence encoding the parent or variant lipase can involve inserting genomic DNA into an expression vector, such as a plasmid, in order to transform lipase-negative host cells. Expression vectors can express a portion of, or all of, the lipase polypeptides described herein. In one embodiment, host cells are eukaryotic cells. In another embodiment, host cells are prokaryotic. In an embodiment, host cells are bacteria or yeast cells. In another embodiment, the bacterial host cells are *E. coli* cells and the yeast host cells are *P. pastoris* cells. The expression vector may be introduced into any host cell so that the protein, or portion thereof, can be expressed by the host cell. The protein can then be isolated from the cells by appropriate purification methods that are known to those of ordinary skill in the art.

Many amino acids can be modified by processing, other post-translational modifications or other chemical modification methods well known by those of ordinary skill in the art before collection of the polypeptide. Modifications can include, but are not limited to, acetylation, acylation, ADP-ribosylation, amidation, covalent attachment of flavin, covalent attachment of a heme group, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cysteine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-mRNA mediated addition of amino acids to proteins such as arginylation, and ubiquitination. Modification can occur anywhere in the protein, including the backbone, C- and N-termini, or the side chains and the various modifications can occur in more than one location.

Saturation mutagenesis and/or site-specific mutagenesis may be used to create the lipase variants; such methods are routine in the biological arts, and are exemplified in Zheng *et al.,* (2004).

Yet another part of the disclosure provides for a method of producing lipase variants by culturing the transformed host cells and recovering the lipase from the resulting culture or broth. In some instances, the entire transformed organism may be used, in others the lipase may be removed from the culture and purified before use. Alternatively, the lipase may be synthesized using known protein synthesis methods.

Still other parts of the disclosure provide for a method to increase the *trans*-selectivity of a lipase. In this method, the nucleic acid sequence encoding for the parent lipase is altered so that the encoded amino acid located at, near or facing a three-dimensional binding, active site or tunnel of the polypeptide is changed. This may result in increased relative accessibility of the substrate to the active site or increased difference in the activation energy needed for enzymatic turnover. The altered nucleic acid sequence is then introduced into a host cell, as described above. The host cell is then cultured under conditions to express the polypeptide variant lipase and the variant lipase is recovered from the culture. The activated catalyst composition containing at least one of the lipase variants described above is then contacted with a trans-fatty acid moiety containing substrate, such as, but not limited to, such as, but not limited to, glycerides including butterfat, cocoa butter, cocoa butter substitutes, illipe fat, kokum butter, milk fat, mowrah fat, phulwara butter, sal fat, shea fat, borneo tallow, lard, lanolin, beef tallow, mutton tallow, tallow or other animal fat, canola oil, castor oil, coconut oil, coriander oil, corn oil, cottonseed oil, hazlenut oil, hempseed oil, linseed oil, mango kernel oil, meadowfoam oil, neat's foot oil, olive oil, palm oil, palm kernel oil, peanut oil, rapeseed oil, rice bran oil, safflower oil, sasanqua oil, soybean oil, sunflower seed oil, tall oil, tsubaki oil, vegetable oils, marine oils which can be converted into plastic or solid fats such as menhaden, candlefish oil, cod-liver oil, orange roughy oil, pile herd, sardine oil, whale and herring oils, 1,3-dipalmitoyl-2-monooleine (POP), 1(3)-palmitoyl-3(1)-stearoyl-2-monooleine (POSt), 1,3-distearoyl-2-monooleine (StOSt), glycerol, triglyceride, diglyceride, monoglyceride, behenic acid triglyceride, trioleine, tripalmitine, tristearine, palm olein, palm stearin, palm kernel olein, palm kernel stearin and triglycerides of medium chain fatty acids; or, processed partial or fully hydrogenated or fractionated oils thereof; esters including wax esters, alkyl esters, methyl esters, ethyl esters, isopropyl esters, octadecyl esters, aryl esters, propylene glycol esters, ethylene glycol esters, 1,2-propanediol esters and 1,3-propanediol esters; and fatty acids including saturated, unsaturated or polyunsaturated fatty acids. Preferably, the one or more fatty acids comprise carbon chains from 4 to 22 carbons long. Also preferably, the fatty acids are selected from the group consisting of palmitic acid, stearic acid, oleic acid, linoleic acid, linolenic acid, arachidonic acid, erucic acid, caproic acid, caprylic acid, capric acid, lauric acid, myristic acid, eicosapentaenoic acid (EPA), docosahexaenoic acid (DHA), 5-eicosenoic acid, butyric acid, y-linolenic acid and conjugated linoleic acid.. In preferred embodiments, substrates include edible oil compositions including, but not limited to vegetable, cooking or frying oil, such as corn oil, grape seed oil, nut oil such as hazelnut oil, linseed oil, safflower oil, sesame oil, olive oil, palm oil, soybean oil, canola oil, pumpkin seed oil, flax seed oil, sunflower oil, argan oil, rice bran oil, and the like, so as to hydrolyze the *trans*-fatty acid and/or long chain fatty acid moieties.

The activity can be measured by conventional procedures well known to one of ordinary skill in the art, such as hydrolysis and alcoholysis (*i.e.,* ethanolysis), assays, turbidimetric assays, alkalimetric methods, colorimetric methods, and the like. In a particular embodiment, the inventors employed the modified method of hydrolysis and ethanolysis as set forth in Examples 5 and 6.

Certain embodiments relate to the selective elimination or reduction of *trans*-unsaturated fatty acid compounds from a substrate containing such compounds by interaction of the above-mentioned variant lipases with the substrate so as to catalyze the breakdown of the *trans*-fatty acid moieties. Particular embodiments relate to the elimination of *trans*-unsaturated fatty acid moieties in triglycerides from edible liquid oils and are therefore related to the food technology industry. For example, these embodiments can eliminate or reduce the amount of *trans*-unsaturated fatty acid moieties in triglycerides from frying oils and frying oil systems. The lipase variants of the invention may be bound to one or more surfaces, substrates, matrices, and the like, either by covalent bonding, or by chemical cross-linking, so as to create an enzyme-bound surface over which a lipid-containing substrate may be passed. Optionally, the lipase enzyme variants of the present invention may be immobilized or contained with populations of microbial cells such as is found in bioreactors, microbial fermentation devices, and such like.

Another part of the disclosure provides a method using the above-mentioned variant lipases for hydrolysis. One method of hydrolysis includes, but is not limited to, preparing the glyceride substrate as an emulsion in water or buffer (phosphate, 50 mM, pH 7.5) using gum arabic and shear mixed at 24000 rpm for 2 min (Ultra-Turrax® T-25 basic, IKA Labortechnik, Staufen, Germany). The substrate emulsion is then exposed to, by mixing or otherwise coming into contact with, at least one of the above-mentioned variant lipases, during which time the glyceride component is hydrolyzed to the free fatty acid component, until the desired percentage of hydrolysis has been achieved. The *trans*-rich free fatty acid component is then separated from the remaining glyceride component using conventional methods of separation such as washing or aqueous extraction, soap precipitation, crystallization, distillation, vacuum distillation or chromatography. The invention further relates to a method of reducing, or eliminating, *trans*-fatty acids from liquid oil stable enough for frying systems.

Another part of the disclosure provides a method of using the above-mentioned variant lipases for alcoholysis, and preferably, for ethanolysis. Methods of ethanolysis include, but are not limited to, preparing the glyceride substrate as an emulsion in a mixture of ethanol and water (*e.g.*, in a 1:1 ratio) using any conventional magnetic stirring equipment for a period of about 10 minutes. The substrate emulsion is then exposed to at least one of the above-mentioned variant lipases, during which time the glyceride component is ethanolysed to the ethyl ester component, until the desired percentage ethanolysis has been achieved. The *trans*-rich ethyl ester component is then separated from the remaining glyceride component using one or more conventional separation methods, including, without limitation, crystallization, distillation, vacuum distillation, chromatography, or any combination thereof.

## EXEMPLARY DEFINITIONS

As used herein, the term "lipase(s)", "lipolytic enzyme(s)" or "lipase enzyme(s)" means a hydrolytic enzyme that acts to break down one or more lipid substrates. Exemplary lipases may be of microbial origin, including, for example, bacterial and /or fungal hydrolases and/or triacylglycerol hydrolases.

As used herein, the term "carboxylic acid moiety" means a fatty acid having a carboxyl group with an aliphatic tail or chain length of 2 or more carbons, which may be abbreviated herein as "Cn" or "$C_n$", wherein the number of carbon atoms is represented by n. For example, oleic acid is a carboxylic acid moiety having a carbon-chain length of 18 carbon atoms, which may be abbreviated as a chain length of C18:1$\Delta$9, in which the 1$\Delta$9 represents the presence of one double bond at the ninth carbon ($C_9$).

As used herein, the term "homology" is an indicator of the degree of identical elements between biological sequences, for example, the percent of identical amino acids between two aligned protein sequences. Gaps can be introduced into one or both of a first and a second amino acid or nucleic acid sequence for optimal alignment and non-homologous sequences may be disregarded for comparison purposes. As used herein, the term "similarity" is an indicator of the degree of similarity between biological sequences, for example, the percent of similar amino acids with similar functions and/or structures at similar positions between two aligned protein sequences.

As used herein, the term "variant" means a lipase derived from a *Pseudozyma* (*Candida*) *antarctica* lipase, or a naturally occurring variant. Typically, the variant differs from the native *Pseudozyma* (*Candida*) *antarctica* lipase by one or more amino acid residues, which may have been added, deleted or inserted at one or more sites within the amino acid sequence of the *Pseudozyma* (*Candida*) *antarctica* lipase, or substituted at one or more amino acid residues by another non-native amino acid residue.

As used herein, the terms "active," "biologically active," and "activity" mean biological activity associated with a particular protein or amino acid sequence and are used interchangeably herein. For example, the enzymatic activity associated with a lipase is hydrolysis and/or alcoholysis, *i.e.,* ethanolysis, so as to hydrolyze or esterify at least a portion of a *trans*-fatty acid or carboxylic acid moiety. Lipase activity is measured as the ability to hydrolyze and/or esterify at least a portion of a *trans*-fatty acid or carboxylic acid moiety.

Certain embodiments also encompass DNA sequences that are complementary, or essentially complementary, with one or more of the specific sequences set forth herein. Nucleic acid sequences that are "complementary" are those that are capable of base pairing according to the standard Watson-Crick complementarity rules. As used herein, the term "complementary sequences" means nucleic acid sequences that are substantially complementary, as may be assessed by the same nucleotide comparison set forth above, or are defined as being capable of hybridizing to one or more of the specific nucleic acid segments disclosed herein under relatively stringent conditions such as those described immediately above.

The term "a sequence essentially as set forth in SEQ ID NO:X" means that the sequence substantially corresponds to at least a first portion of SEQ ID NO:X and has relatively few nucleotides that are not identical to, or a biologically functional equivalent of, the nucleotides of SEQ ID NO:X. Accordingly, sequences that have about 85% to about 90%; about 91% to about 95%; or about 96% to about 99% of nucleotides that are identical or functionally equivalent to one or more of the nucleotide sequences provided herein are particularly contemplated to be useful in the practice of the invention. In the context of peptides, polypeptides, and proteins, the term "a sequence essentially as set forth in SEQ ID NO:X" means that the sequence substantially corresponds to at least a first portion of SEQ ID NO:X and has relatively few amino acid residues that are not identical to, or a biologically functional equivalent of, the amino acid residues of SEQ ID NO:X. The term "biologically functional equivalent" is well understood by one of ordinary skill in the art, and is further defined in detail herein. Accordingly, peptide, polypeptide, or protein sequences that have about 85% to about 90%; about 91% to about 95%; or about 96% to about 99% of the amino acids that are identical or functionally equivalent to one or more of the amino acid sequences provided herein are particularly contemplated to be useful in the practice of the invention.

As used herein, the term "primer" refers to a single-stranded oligonucleotide that acts as a point of initiation of template-directed DNA synthesis using well-known methods (*e.g.* PCR) including, but not limited to those described herein. The appropriate length of a primer depends on its particular use, but typically ranges from about 15 to about 30 nucleotides.

As used herein, the term "expression" means the biological production of a product encoded by a coding sequence. In most cases, a polynucleotide (*i.e.,* DNA) sequence, including the coding sequence, is transcribed to form a messenger-RNA (mRNA). The messenger-RNA is then translated to form a polypeptide product that has a relevant biological activity. The process of expression may involve further processing steps to the RNA product of transcription, such as splicing to remove introns, and/or post-translational processing of a polypeptide product.

As used herein, the term "heterologous" is used in relation to a predetermined referenced gene sequence. For example, with respect to a structural gene sequence, a heterologous promoter is defined as a promoter that does not naturally occur adjacent to the referenced structural gene, but which is positioned by laboratory manipulation. Likewise, a heterologous gene or nucleic acid segment or sequence is defined as a gene or segment that does not naturally occur adjacent to the referenced promoter and/or enhancer elements. Similarly, the term "heterologous" is also used in relation to a predetermined amino acid sequence. For example, with respect to an amino acid sequence, a heterologous protein tag, such as a poly-histidine tag, is defined as a peptide sequence that does not naturally occur adjacent to the referenced amino acid sequence. Likewise, a heterologous amino acid segment or sequence is defined as a segment or sequence that does not naturally occur adjacent to the referenced tag. Additionally, a heterologous protein refers to a protein that is not natively produced by or found within a particular organism. This can occur, for example, by the cloning and expression of a non-native lipase gene in a host organism such as *E. coli.*

As used herein, the term "operably linked" means a linkage of two or more polynucleotides or two or more nucleic acid sequences in a functional relationship. A nucleic acid is "operably linked" when it is placed into a functional relationship with another nucleic acid sequence. For instance, a promoter or enhancer is operably linked to a coding sequence if it affects the transcription of the coding sequence. "Operably linked" means that the nucleic acid sequences being linked are typically contiguous, or substantially contiguous, and, where necessary to join two protein coding regions, contiguous and within a reading frame. Since enhancers generally function when separated from the promoter by several kilobases (Kb) and intronic sequences may be of variable lengths; however, some polynucleotide elements may be operably linked but not contiguous.

The phrases "isolated" or "biologically pure" means material that is substantially, or essentially, free from components that normally accompany the material as it is found in its native state. Thus, an embodiment provides that the isolated peptides do not contain materials normally associated with the peptides in their *in situ* environment.

"Link" or "join" means any method well known by those of ordinary skill in the art for functionally connecting two or more molecules, including, without limitation, recombinant fusion, covalent bonding, disulfide bonding, ionic bonding, hydrogen bonding, electrostatic bonding, and such like.

As used herein, the term "polypeptide" means a singular "polypeptide" as well as plural "polypeptides," and includes any chain or chains of two or more amino acids. Thus, as used herein, terms including, but not limited to "peptide," "dipeptide," "tripeptide," "protein," "enzyme," "amino acid chain," and "contiguous amino acid sequence" are all encompassed within the definition of a "polypeptide," and the term "polypeptide" can be used instead of, or interchangeably with, any of these terms. The term further includes polypeptides that have undergone one or more post-translational modification(s), including, without limitation, glycosylation, acetylation, phosphorylation, amidation, derivatization, proteolytic cleavage, post-translation processing, or modification by inclusion of one or more non-naturally occurring amino acids. Throughout the disclosure, common one-letter and three-letter amino acid abbreviations have been employed following the conventional nomenclature in the art: Alanine (A; Ala), Arginine (R; Arg), Asparagine (N; Asn), Aspartic Acid (D; Asp), Cysteine (C; Cys), Glutamine (Q; Gln), Glutamic Acid (E; Glu), Glycine (G; Gly), Histidine (H; His), Isoleucine (I; Ile), Leucine (L; Leu), Methionine (M; Met), Phenylalanine (F; Phe), Proline (P; Pro), Serine (S; Ser), Threonine (T; Thr), Tryptophan (W; Trp), Tyrosine (Y; Tyr), Valine (V; Val), and Lysine (K; Lys). Amino acid residues described herein are preferred to be in the "L" isomeric form. However, residues in the "D" isomeric form may be substituted for any L-amino acid residue provided the desired properties of the polypeptide are retained. All amino-acid residue sequences represented herein conform to the conventional left-to-right amino-terminus to carboxy-terminus orientation.

The term "protein" is used herein interchangeably with "peptide" and "polypeptide," and includes both peptides and polypeptides produced synthetically, recombinantly, or *in vitro* and peptides and polypeptides expressed *in vivo* after nucleic acid sequences are administered into a host animal or human subject. The term "polypeptide" is intended to refer to any amino acid chain length, including those of short peptides from about 2 to about 20 amino acid residues in length, oligopeptides from about 10 to about 100 amino acid residues in length, and longer polypeptides including from about 100 amino acid residues or more in length. Furthermore, the term is also intended to include enzymes, *i.e.,* functional biomolecules, including at least one amino acid polymer. Polypeptides and proteins of the present invention also include polypeptides and proteins that are or have been post-translationally modified, and include any sugar or other derivative(s) or conjugate(s) added to the backbone amino acid chain.

As used herein, the term "substantially free" or "essentially free" in connection with the amount of a component means a composition that contains less than about 10 weight percent, less than about 5 weight percent, and less than about 1 weight percent of a compound. In an embodiment, these terms refer to less than about 0.5 weight percent, less than about 0.1 weight percent, or less than about 0.01 weight percent.

As used herein, the term "substantially homologous" encompasses sequences that are similar to the identified sequences, such that antibodies raised against peptides having the identified sequences will specifically bind to (*e.g.*, cross-react with) peptides having the substantially homologous sequences. In some variations, the amount of detectable antibodies induced by the homologous sequence is identical to the amount of detectable antibodies induced by the identified sequence. In other variations, the amounts of detectable antibodies induced are substantially similar, thereby providing immunogenic properties. For example, homologous can refer to at least about 75%, at least about 80% identical and "substantially homologous" can refer to at least about 85% or at least about 90% identity, at least about 95%, at least about 97% identical, at least about 98% identical, at least about 99% identical, and at least substantially or entirely 100% identical (*i.e.,* "invariant"). When one or more amino acid residues in the lipase or lipase variant are mutated by substitution of a conservative amino acid, two or more peptides may have substantially similar activity and therefore be considered homologous or substantially homologous.

As used herein, the term "substrate" means a chemical compound that can be catalyzed by lipases. For example, alcohols, amines, amino esters, amides, carboxylic acid esters, thioesters, thiols, cyanohydrins, cyanohydrin esters, and meso-diols and their sterioisomers may be substrates.

The term "for example" or "*e.g.,*" as used herein, means by way of example, without limitation intended, and should not be construed as referring only those items explicitly enumerated in the specification.

In accordance with long standing patent law convention, the words "a" and "an" when used in this application, including the claims, denote "one or more."

**EXAMPLES**

The following examples are included to demonstrate illustrative embodiments of the invention. It should be appreciated by those of ordinary skill in the art that the techniques disclosed in the examples that follow represent techniques discovered by the inventors to function well in the practice of the invention, and thus can be considered to constitute modes for its practice. However, those of ordinary skill in the art should, in light of the present disclosure, appreciate that many changes can be made in the specific embodiments that are disclosed and still obtain a like or similar result without departing from the scope of the invention.

## EXAMPLE 1- MODELING OF THE SUBSTRATE BINDING TUNNEL OF CAL-A

*Molecular modeling-* Molecular modeling was performed using YASARA version 7.11.28 (YASARA Bioscience, Graz, Austria). Graphical representations were performed using the PYMOL visualization software (version 0.99rc6; DeLano Scientific LLC, CA, USA) and the Computed Atlas of Surface Topography of Proteins (CASTp; Dundas, 2006). These programs were used to determine the location of the substrate binding tunnel. CAL-A was visualized with modeled *cis-* and *trans-* fatty acid isomers and was based on energy minimizations with YASARA applying the standard force field for energy-minimization calculations and Molecular Dynamics (MD) simulations (YASARA Biosciences GmbH, Vienna, Austria). Energy minimizations were performed by using the Powell method (Powell, 1977). All crystal structure coordinates were obtained from Research Collaboratory for Structural Bioinformatics (RCSB) Protein Data Bank available on their website (Piscataway, NJ; La Jolla, CA; Madison, WI; USA). **FIG. 1A** shows the model of the substrate binding tunnel with YASARA, **FIG. 1B** shows the PYMOL model of the substrate binding tunnel and how modeled $C_{18}$ fatty acids are located in the 30 Å long, narrow binding tunnel of CAL-A; and **FIG. 1C** shows the model of the substrate binding tunnel with CASTp. Analysis of the CAL-A X-ray structure (PDB code: 2veo, 2008 and 3guu, 2010) with the CASTp pocket identification algorithms along with simulated docking calculations as would be known to one of ordinary skill in the art. Modeling of different $C_{18}$ fatty acid esters into the crystal structure of CAL-A were performed to highlight areas of interest for mutagenesis studies. Darker areas on **FIG. 1C** indicate the amino acid residues that were selected for saturation mutagenesis, while lighter areas depict residues that form the substrate binding tunnel. In total, 12 residues within the fatty acid binding tunnel were selected as they were facing the modeled $C_{18}$ fatty acid ester substrates within about a 5-Å distance. Sites located at the far end of the modeled $C_{18}$ fatty acid ester were also included in the mutagenesis to probe the CAL-A substrate binding tunnel. The active site pocket and the small appendix near the active site are not depicted.

*Homology to CAL-A-* In order to identify enzymes with a high homology to CAL-A, sequence and structure-based alignments were performed. **FIG 2** shows the structure of CAL-A, including the α/β hydrolase fold, unique lid and flap structures. Based on the CAL-A structure, the amino acid sequences with the highest homologies were found to be Q8NIP2 (UniProt), which encodes for the already described lipK (Kakugawa et al., 2022), the amino acid sequence Q4P903 (UnitProt), lipUMf, the amino acid sequence E7A3D1 (UniProt) which encodes for SpoRei_E7 and the amino acid sequence E6ZNA3 (UniProt), which encodes for SpoRei_E6. Notably, *Pseudozyma antarctica*, *Kurtzmanomyces* spec., *Sporisorium reilianum* and *Ustilago maydis* all belong to the same group of yeast-producing basidiomycetes. All other amino acid sequences identified during the structure-based search displayed a homology of less than 35%. The crystal structure with the highest homology to CAL-A according to the Distance matrix ALIgnment Server (DALI) of the European Bioinformatics Institute (EBI) (Holm and Sander, 1993), was found to be a rice cell-wall-degrading esterase from *Xanthomonas oryzae* (pdb-file: 3h2g) (Aparna *et al.,* 2009). Sequence based alignments gave similar results as published already (Pfeffer *et al.,* 2006). Thus, it was confirmed that lipK, lipUM and SpoRei_E6 and SpoRei_E7 were all members of the CAL-A "superfamily."

Table 3 shows a comparison of the size, length, and homology of the sequences of CAL-A, lipK, the full sequence of lipUM (hereinafter "lipUMf") and the shortened sequence of lipUM (hereinafter "lipUMs") with the N-terminus of lipUMf removed.

### TABLE 3
#### COMPARISON OF SIZE, LENGTH AND HOMOLOGY OF CAL-A, LIPK, LIPUMF AND LIPUMS

| Lipase | Number of amino acids | Sequence homology to CAL-A [%] | Probability of presence of a N-terminal signal sequence (Target P1.1) | Probability for certain signal sequence cleavage site (SignalP 3.0) | Prediction of possible transmembrane segments (TMS) within the sequence (TMHMM 2.0) |
|---|---|---|---|---|---|
| **CAL-A** | 462 | | 0.8 | VLA-AP (100%) | 0 |
| **lipK** | 458 | 76 | 0.9 | ALA-AP (100%) | 0 |
| **lipUMf** | 582 | 69 | 0.6 | GIA-RS (88%) | 2 TMS possible |
| **lipUMs** | 458 | 69 | 0.9 | SLA-VP (100%) | 0 |

VLA-AP refers to the amino acid sequence of the CAL-A sequence, where potential cleavage by the signal peptidase may occur during protein processing. ALA-AP refers to the amino acid sequence of the lipUMf sequence, where potential cleavage by the signal peptidase may occur during protein processing. GIA-RS refers to amino acid sequence numbers 32 through 36 of the lipUMf sequence, where potential cleavage by the signal peptidase may occur during protein processing. SLA-VP refers to amino acid sequence numbers 19 through 23 of the lipUMs sequence, where potential cleavage by the signal peptidase may occur during protein processing. The hyphen in each case indicates the position where the cleavage would likely occur, according to the software.

**EXAMPLE 2 - THE FUNCTIONAL EXPRESSION OF CAL-A, LIPUMF AND LIPUMS, AND**

**VARIANTS THEREOF, IN *E. COLI* AND *P. PASTORIS***

CAL-A, lipUMf and lipUMs, and variants thereof were cloned and functionally expressed in *E. coli* cells. Synthetic DNA sequences of each of the enzymes were codon-optimized for expression and subcloned into pET-22b(+) expression vectors (Novagen).

CAL-A, lipUMs, and variants thereof were also cloned and functionally expressed in *P. pastoris* cells.

*Gene synthesis and subcloning of CAL-A and variants thereof*- Codon usage, periplasmic versus cytoplasmic expression, and influence of various *E. coli* strains on recombinant CAL-A expression were analyzed and optimized.

FIG. 3 shows a schematic of the four CAL-A constructs that were created to evaluate whether periplasmic expression or cytoplasmic expression of CAL-A was more amenable to high throughput applications. Construct I contained the wild-type CAL-A sequence, without its pre-pro sequence and with a His-tag, and a pelB leader sequence that directs the protein into bacterial periplasm, where the sequence is then removed by a signal peptidase. Construct II contained the wild-type CAL-A sequence, without its pre-pro sequence and with a His-tag, and a Small Ubiquitin-like Modifier (SUMO) tag, for cytosolic transport and/or solubility. Construct III contained the wild-type CAL-A sequence, with its pre-pro sequence, which may have a role in protein folding or in supporting translocation of the peptide, and a His-tag which was shown to have periplasmic expression. Construct IV contained the wild-type CAL-A sequence ("mCAL-A" or the information contained within the mature part of the protein) without its pre-pro sequence and with a His-tag for cytoplasmic expression.

For gene synthesis and subcloning of CAL-A into *E. coli,* the nucleotide sequence of CAL-A was derived from the published amino acid sequence (Hoegh *et al..* 1995). For expression of CAL-A, its sequence was codon optimized by GENEART with the software "GeneOptimizer" (Geneart, Regensburg, Germany). For Construct III, a composite plasmid, named pGA15-pproCAL-Aop, carried the codon-optimized, full-length (*i.e.* the signal pre-pro amino acid sequence) CAL-A sequence. This plasmid was used as the template in a polymerase chain reaction (PCR) for subcloning the CAL-A gene into the pET-22b(+) expression vector (Novagen). The codon-optimized CAL-A contained a histidine (His) tag for detection and purification purposes. The codon-optimized DNA sequence for CAL-A used for cloning into *E. coli* without the addition of the His-tag included the following polynucleotide sequence:

ATGCGTGTGAGCCTGCGTAGCATTACCAGCCTGCTGGCTGCGGCAACCGCAGC

AGTTCTGGCTGCGCCGGCAGCGGAAACCCTGGATCGTCGTGCGGCGCTGCCGA

ATCCGTATGATGATCCGTTTTATACCACCCCGAGCAACATTGGCACCTTTGCGA

AAGGCCAGGTGATTCAGAGCCGTAAAGTGCCGACCGATATTGGCAACGCGAA

CAACGCGGCGAGCTTTCAGCTGCAATATCGTACCACCAACACCCAGAACGAAG

CGGTGGCGGATGTGGCGACCGTGTGGATTCCGGCGAAACCGGCGAGCCCGCCG

AAAATTTTTAGCTACCAGGTGTATGAAGATGCGACCGCGCTGGATTGCGCGCC

GAGCTATAGCTATCTGACCGGCCTGGATCAGCCGAACAAAGTGACCGCGGTGC

TGGATACCCCGATTATTATTGGCTGGGCGCTGCAACAGGGCTATTATGTGGTG
AGCAGCGATCATGAAGGCTTTAAAGCGGCGTTTATTGCGGGCTATGAAGAAGG
CATGGCGATTCTGGATGGCATTCGTGCGCTGAAAAACTATCAGAACCTGCCGA
GCGATAGCAAAGTGGCGCTGGAAGGCTATAGCGGCGGTGCGCACGCGACCGT
TTGGGCGACCAGCCTGGCCGAAAGCTATGCGCCGGAACTGAACATTGTGGGCG
CGAGTCATGGTGGCACCCCGGTGAGCGCGAAAGATACCTTTACCTTTCTGAAC
GGCGGTCCGTTTGCGGGTTTTGCGCTGGCCGGTGTGAGCGGTCTGAGCCTGGC
CCATCCGGATATGGAAAGCTTTATTGAAGCGCGTCTGAACGCGAAAGGTCAGC
GTACCCTGAAACAAATTCGTGGCCGTGGCTTTTGCCTGCCGCAGGTGGTGCTG
ACCTATCCGTTTCTGAACGTGTTTAGCCTGGTGAACGATACCAACCTGCTGAAC
GAAGCGCCGATTGCGAGCATTCTGAAACAGGAAACCGTTGTTCAGGCGGAAGC
GAGCTATACCGTGAGCGTGCCGAAATTTCCGCGTTTTATTTGGCATGCGATTCC
GGATGAAATTGTGCCGTATCAGCCGGCAGCGACCTATGTGAAAGAACAGTGCG
CGAAAGGCGCGAACATTAACTTTAGCCCGTATCCGATTGCGGAACATCTGACC
GCGGAAATTTTTGGCCTGGTGCCGAGCCTGTGGTTTATTAAACAGGCGTTTGAT
GGCACCACCCCGAAAGTGATTTGCGGCACCCCGATTCCGGCGATTGCGGGCAT
TACCACCCCGTCTGCGGATCAGGTGCTGGGCAGCGATCTGGCCAACCAGCTGC
GTAGCCTGGATGGCAAACAGAGCGCGTTTGGCAAACCGTTTGGCCCGATTACC
CCGCCG (SEQ ID NO:16).

For PCR, the following oligonucleotides were used to amplify the CAL-A sequence: Forward primer: 5'-TAAGGTACCATATGCGTGTGAGCCT-3' (SEQ ID NO: 17) and Reverse primer: 5'-TAAGAATGCGGCCGCCGGCGGGGTAATCGGGCC-3' (SEQ ID NO:18).

The PCR-product was digested with restriction enzymes, *NdeI* and *NotI* (Fermentas St. Leon-Rot, Germany), and ligated into the correspondingly digested pET22b(+) vector using standard cloning protocols. Constructs I, II, and IV were similarly created.

*Genre syntheses and subcloning of lipUMf, lipUMs and variants thereof-*Subcloning of lipUMs and lipUMf were performed by GENEART. The genes of lipUMs and lipUMf were codon optimized for *E. coli,* newly synthesized and subcloned into the pET22b(+) vector via *NdeI* and *Not1* restriction sites, respectively. This plasmid was used as the template in a polymerase chain reaction (PCR) for subcloning both the lipUMs and lipUMf gene into the pET-22b(+) expression vector (Novagen) which contained a histidine (His) tag for detection and purification purposes.

The codon-optimized DNA sequence for lipUMf used for cloning into *E. coli* contained the following sequence:

ATGTGGGGTCGTATTCGTAATGTTATTCAGCCGACCTGGGCACCTCCGCTGTTT
GGCACCCTGAATATTATTTTTAGCCTGTTTTTTCGTGCAGGTATTGCACGTAGC
CATAAATGGACCTGGTGTTGTTATCGTCCGACCCGTATGGCACGTAGCCGTACC
TTTAGCAATAGCGCACCGACCCGTCGTCGTCCGGAACGTCTGCGTCTGCAGAA
AGGTAGCAGCAATACCACCATTCGTCCGCGTCCGAGCGCAATTCTGCCGGATG
AAATGAATCATGGTAGCCTGCTGACCGTTGTTCCGCATACCGTTGTTGCAAGCA
CCCCGAGCTTTCGTAGCAGCTTTCCGGATAGCCTGATTGCAAGCGTTCAGATGC
GTTTTATTGCAGTTCGTGCCATTGTTACCCTGGCAGCAGCAGCAGCCGTTAGCC
TGGCAGTTCCGACCGAACGTCGTGCAGCATTTGCAGATCCGAATGATGACCTG
TTCTATACCACACCGGATAACATCAACACCTATGCCAATGGCCAGGTTATTCA
GAGCCGTAAAGCCGATACCGATATTGGCAATAGCAATAAAGTGGAAGCATTTC
AGCTGCAGTATCGTACCACCAATACCCAGAAAGAAGCACAGGCCAACGTTGCA
ACCGTTTGGATTCCGAATAAACCGGCATCTCCTCCGAAAATTTTTAGCTATCAG
GTGTATCAGGATAGCACCCAGCTGAATTGTGCACCGAGCTATAGCTTTCTGAA
AGGTCTGGATAAACCGAATAAAGCAACCACCATTCTGGAAGCACCGATTATTA
TTGGTTGGGCACTGCAGCAGGGTTTTTATGTTGTTAGCAGCGATCATGAAGGTC
CGCGTAGCTCTTTTATTGCCGGTTATGAAGAAGGTATGGCCATTCTGGATGGTA
TTCGTGCCCTGAAAAATTATGCAAAACTGCCGACCGATAGCGCAATTGGTTTTT
ATGGTTATAGCGGTGGTGCACATGCAACCGGTTGGGCAGCAAATCTGGCAGGT
AGCTATGCACCGGAACATAATATTATTGGTGCAGCCTATGGTGGTCTGCCTGC
AAGCGCACGTGATACCTTTAATTTTCTGAATAAAGGTGCCTTTGCAGGTTTTGC
AATTGCCGGTGTTAGCGGTCTGGCACTGGCATATCCGGATGTGGAAACCTATA
TTCAGTCTCGCCTGAATGCAAAAGGCGAAAAAGTGTTTAAACAGGTTCGTAGC
CGTGGTTTTTGTATTGGTCAGGTGGTTCTGACCTATCCGTTTGTTGATGCCTATA
GCCTGATTAATGATACCAATCTGCTGAATGAAGAACCGGTTGCCAGCACCCTG
AAAAGCGAAACCCTGGTTCAGGCAGAAGCAAGCTATACCGTTCCGGTTCCGAA
ATTTCCGCGTTTTATTTGGCATGCACTGCTGGATGAAATTGTTCCGTTTCATAG
CGCAGCAACCTATGTTAAAGAACAGTGTAGCAAAGGTGCCGATATTAATTGGA
ATGTGTATAGCTTTGCCGAACATATTAGCGCAGAACTGTTTGGTCTGCTGCCTG
GTCTGGATTGGCTGAATAAAGCCTATAAAGGTCAGGCACCGAAAGTTCCGTGT
GGTGGTGGTGCACAGAGCGTTATGGGTGCAAGCGGTCCTCCGGCACAGGATGT
TCTGGGTGCAGATCTGGCAAGCCAGCTGCGTAGCCTGCAGGGTAAACCGAGCG

CATTTGGCAATAAACCGTTTGGTAGCATTTCTCCTGCGGCCGCACTCGAGCACC
ACCACCACCACCACTGA (SEQ ID NO:19)

The codon-optimized DNA sequence for lipUMs used for cloning into *E. coli* contained the following sequence:

ATGCGTTTTATTGCCGTTCGTGCAATTGTTACCCTGGCTGCAGCAGCAGCAGTT
AGCCTGGCCGTTCCGACCGAACGTCGTGCAGCATTTGCAGATCCGAATGACGA
CCTGTTTTATACCACACCGGATAACATCAATACCTATGCGAATGGTCAGGTTAT
TCAGAGCCGTAAAGCCGATACCGATATTGGCAATAGCAATAAAGTGGAAGCAT
TTCAGCTGCAGTATCGTACCACCAATACCCAGAAAGAAGCACAGGCAAACGTC
GCAACAGTTTGGATTCCGAATAAACCGGCAAGCCCTCCGAAAATTTTTAGCTA
TCAGGTGTATCAGGATAGCACCCAGCTGAATTGTGCACCGAGCTATAGCTTTCT
GAAAGGTCTGGATAAACCGAATAAAGCAACCACCATTCTGGAAGCACCGATTA
TTATTGGTTGGGCACTGCAGCAGGGTTTTTATGTTGTTAGCAGCGATCATGAAG
GTCCGCGTAGCAGCTTTATTGCAGGTTATGAAGAAGGTATGGCCATTCTGGAT
GGTATTCGTGCCCTGAAAAATTATGCAAAACTGCCGACCGATAGCGCAATTGG
TTTTTATGGTTATAGCGGTGGTGCACATGCAACCGGTTGGGCAGCAAATCTGG
CTGGTAGCTATGCACCGGAACATAATATTATTGGTGCAGCCTATGGTGGTCTGC
CTGCCAGCGCACGTGATACCTTTAATTTTCTGAATAAAGGTGCCTTTGCAGGTT
TTGCAATTGCAGGTGTTAGCGGTCTGGCCCTGGCCTATCCGGATGTTGAAACCT
ATATTCAGTCTCGCCTGAATGCAAAAGGCGAAAAAGTGTTTAAACAGGTTCGT
AGCCGTGGTTTTTGTATTGGTCAGGTGGTTCTGACCTATCCTTTTGTTGATGCCT
ATAGCCTGATTAATGATACCAATCTGCTGAATGAAGAACCGGTTGCAAGCACC
CTGAAAAGCGAAACCCTGGTTCAGGCAGAAGCAAGCTATACCGTTCCGGTTCC
GAAATTTCCGCGTTTTATTTGGCATGCACTGCTGGATGAAATTGTTCCGTTTCA
TAGCGCAGCAACCTATGTTAAAGAACAGTGCTCTAAAGGTGCCGATATTAATT
GGAATGTGTATAGCTTTGCCGAACATATTAGCGCAGAGCTGTTTGGTCTGCTGC
CTGGTCTGGATTGGCTGAATAAAGCCTATAAAGGTCAGGCACCGAAAGTTCCG
TGTGGTGGTGGTGCACAGAGCGTTATGGGTGCAAGCGGTCCTCCGGCACAGGA
TGTTCTGGGTGCAGATCTGGCCAGCCAGCTGCGTAGCCTGCAGGGTAAACCGA
GCGCATTTGGCAATAAACCGTTTGGTAGCATTTCTCCTGCGGCCGCACTCGAGC
ACCACCACCACCACCACTGA (SEQ ID NO:20).

The genes of lipUMs and lipUMf were also codon optimized by GENEART for subcloning into *P. pichia.* The codon optimized lipUMs and lipUMf sequences were inserted into the pPICZ B vector (Invitrogen) and contained a His-tag for detection and purification purposes when performing PCR. The codon-optimized DNA sequence for lipUMf used for cloning into *P. pastoris* contained the following sequence:

ATGTGGGGCCGTATCCGTAATGTCATCCAGCCTACCTGGGCACCGCCTTTGTTT
GGCACCCTGAATATCATCTTTAGCCTGTTCTTTCGTGCGGGTATTGCTAGATCT
CATAAGTGGACTTGGTGTTGCTATAGACCAACACGCATGGCGCGCTCCCGTAC
GTTTAGCAACAGTGCTCCAACCCGTAGACGCCCGGAAAGACTGCGCTTGCAGA
AAGGATCTTCCAACACCACTATCCGTCCGAGACCTTCTGCCATTCTGCCTGATG
AAATGAATCATGGTTCCCTGTTGACCGTTGTGCCACACTGTCGTTGCGTCAA
CACCTAGCTTTAGATCAAGCTTCCCAGATAGTTTGATTGCTTCTGTTCAAATGC
GCTTTATCGCCGTTCGTGCAATTGTTACCCTGGCTGCCGCAGCGGCTGTCTCCT
TGGCGGTTCCAACCGAACGTAGAGCCGCATTTGCTGATCCGAACGATGACCTG
TTCTATACAACGCCTGACAACATCAATACGTACGCCAATGGCCAGGTCATTCA
ATCCCGTAAGGCAGATACCGACATCGGAAACTCAAACAAAGTGGAAGCTTTCC
AGCTGCAATACAGAACCACTAATACTCAGAAGGAGGCCCAAGCAAACGTGGC
CACAGTCTGGATTCCTAATAAGCCAGCATCTCCACCGAAAATCTTTTCCTATCA
GGTTTACCAAGATTCTACTCAGCTGAACTGTGCCCCGTCTTATTCCTTCCTGAA
AGGTTTGGACAAACCAAATAAGGCGACAACGATTCTGGAAGCTCCGATTATCA
TTGGTTGGGCATTGCAGCAAGGCTTTTACGTGGTCAGTTCTGATCATGAGGGTC
CGAGATCCTCATTCATTGCCGGCTATGAAGAGGGAATGGCAATCTTGGATGGC
ATTCGCGCGCTGAAGAACTACGCTAAATTGCCTACTGACAGTGCCATTGGCTTT
TATGGATACTCTGGTGGCGCGCATGCTACAGGATGGGCGGCTAACCTGGCCGG
TTCATATGCACCGGAACACAATATCATTGGTGCTGCATATGGAGGTCTGCCTGC
AAGCGCACGCGATACTTTTAACTTCTTGAACAAAGGAGCGTTTGCTGGCTTCGC
CATTGCTGGTGTGTCAGGCCTGGCGTTGGCTTATCCTGACGTCGAAACCTACAT
CCAAAGCCGTCTGAATGCTAAAGGAGAGAAGGTGTTTAAACAGGTCCGCAGTC
GTGGATTCTGCATTGGTCAAGTTGTGCTGACTTATCCTTTTGTTGATGCCTACTC
TTTGATCAACGACACAAATCTGTTGAACGAAGAGCCAGTTGCATCCACGTTGA
AGTCAGAAACCCTGGTGCAGGCCGAGGCATCTTATACTGTCCCAGTTCCGAAA
TTTCCACGTTTCATCTGGCATGCGCTGTTGGATGAAATTGTTCCGTTCCACAGC
GCGGCTACCTATGTGAAGGAGCAATGTAGTAAAGGTGCTGACATTAACTGGAA
TGTTTACTCATTTGCCGAACACATCAGCGCAGAGCTGTTCGGCCTGTTGCCGGG
ACTGGATTGGTTGAATAAGGCGTACAAAGGCCAGGCTCCGAAAGTCCCTTGCG
GCGGAGGTGCTCAAAGCGTTATGGGAGCCAGTGGTCCTCCAGCACAGGATGTG
CTGGGTGCGGACTTGGCTTCTCAACTGCGTAGCCTGCAAGGTAAACCATCAGC
ATTCGGTAACAAACCATTCGGAAGTATCTCCCCGGCGGCCGCCAGCTTTCTAG

AACAAAAACTCATCTCAGAAGAGGATCTGAATAGCGCCGTCGACCATCATCAT CATCATCATTGA (SEQ ID NO:21).

The codon-optimized DNA sequence for lipUMs used for cloning into *P. pastoris* contained the following sequence:

ATGCGCTTCATCGCTGTGCGTGCTATTGTCACTTTGGCGGCGGCGGCGGCGGTG TCATTGGCAGTCCCTACGGAACGTCGTGCGGCTTTTGCTGATCCGAACGATGAC CTGTTCTATACCACTCCTGACAACATCAATACCTACGCAAATGGCCAGGTTATT CAATCCAGAAAGGCGGATACTGACATCGGAAACTCAAACAAAGTGGAAGCCT TTCAGTTGCAATATCGCACAACGAATACCCAGAAGGAGGCACAAGCGAACGTT GCTACTGTGTGGATTCCAAATAAGCCGGCCTCTCCACCGAAAATCTTTTCCTAT CAGGTTTACCAAGATTCTACCCAGCTGAACTGTGCACCAAGTTATTCTTTCCTG AAAGGTTTGGACAAACCAAATAAGGCTACCACTATTCTGGAAGCCCCGATTAT CATTGGTTGGGCCTTGCAGCAAGGCTTTTACGTTGTGTCTTCCGATCATGAAGG CCCTCGCTCAAGCTTCATTGCAGGCTATGAAGAGGGAATGGCGATCTTGGATG GTATTCGTGCTCTGAAGAACTACGCCAAATTGCCAACAGACAGTGCTATTGGC TTTTATGGATACTCTGGTGGCGCTCATGCAACCGGATGGGCTGCAAACCTGGC AGGTAGCTATGCGCCTGAACACAATATCATTGGTGCAGCGTACGGAGGTCTGC CAGCAAGTGCGCGTGATACCTTTAACTTCTTGAACAAAGGTGCTTTTGCCGGCT TCGCAATTGCGGGTGTCTCTGGCCTGGCTTTGGCCTATCCGGATGTTGAAACTT ACATCCAATCCAGACTGAATGCCAAAGGAGAGAAGGTCTTTAAACAGGTTCGT TCAAGAGGATTCTGCATTGGTCAAGTCGTTCTGACATATCCATTTGTTGATGCT TACTCCTTGATCAACGACACGAATCTGTTGAACGAAGAGCCGGTGGCCTCCAC ATTGAAGTCAGAAACGCTGGTCCAGGCAGAGGCGTCATATACTGTGCCGGTCC CTAAATTTCCGCGTTTCATCTGGCATGCACTGTTGGATGAAATTGTGCCTTTCC ACAGCGCTGCCACATATGTCAAGGAGCAATGTAGTAAGGGTGCGGACATTAAC TGGAATGTCTACTCATTTGCAGAACACATCAGCGCGGAGCTGTTCGGCCTGTTG CCTGGACTGGATTGGTTGAACAAGGCTTACAAAGGCCAGGCCCCTAAAGTTCC ATGCGGCGGAGGTGCTCAAAGCGTGATGGGAGCAAGTGGTCCTCCAGCGCAG GATGTGCTGGGTGCTGACTTGGCCTCTCAACTGCGTAGCCTGCAAGGTAAACC ATCCGCATTCGGTAACAAGCCATTCGGTAGCATCTCACCAGCGGCCGCCAGCT TTCTAGAACAAAAACTCATCTCAGAAGAGGATCTGAATAGCGCCGTCGACCAT CATCATCATCATCATTGA (SEQ ID NO:22).

Oligonucleotides were obtained from Biomers.net (Ulm, Germany). As described above, the probes and primers of the present invention may be of any length, and are about 60% to about 99% similar to the primers in SEQ ID NO: 17 and SEQ ID NO:18, about 75% to about 95% similar, or about 80% to about 90% similar. By assigning numeric values to a sequence, for example, the first residue is 1, the second residue is 2, *etc.,* an algorithm defining all primers can be proposed:

$$(\textbf{Eq. 1}) \qquad n \text{ to } n + y$$

where n is an integer from 1 to the last number of the sequence and y is the length of the primer minus one, where n + y does not exceed the last number of the sequence. Thus, for a 25-mer, the probes correspond to bases 1 to 25, 2 to 26, 3 to 27 ... and so on. For a 45-mer, the probes correspond to bases 1 to 45, 2 to 46, 3 to 47 ... and so on. For a 60-mer, the probes correspond to bases 1 to 60, 2 to 61, 3 to 62 ... and so on.

In the practice of the invention, it may be advantageous in certain aspects to employ nucleic acid sequences of the present invention in combination with an appropriate detectable marker (*i.e.,* a "label") for determining hybridization. A wide variety of appropriate indicator compounds and compositions are known in the art, including, without limitation, fluorescent labels, radioactive labels, enzymatic detection, and detectable ligands (such as, without limitation, avidin/biotin, *etc*.).. In particular embodiments, one may employ a fluorescent label, or an enzyme tag (such as urease, alkaline phosphatase, or peroxidase), instead of radioactive or other environmentally less-desirable reagents. In the case of enzyme tags, a variety of colorimetric, chromogenic, and/or fluorogenic indicator substrates are known,which can be employed in methods for detecting the sample that is visible to the human eye, or by analytical methods such as scintigraphy, fluorometry, spectrophotometry, and the like, to identify specific hybridization with samples containing one or more complementary or substantially complementary nucleic acid sequences. Constructs and lipase variants were then sequenced.

*Expression of CAL-A, lipUMf, lipUMs and variants thereof* - *E. coli* BL21(DE3) (Studier and Moffatt, 1986), C41(DE3) or C43(DE3) (Miroux and Walker, 1996) cells were transformed with pET-22b(+) or derivatives thereof, *i.e.* pET-22b vectors containing the codon optimized lipUMf, lipUMs or CAL-A sequences, or variants thereof, using standard procedures known to those of ordinary skill in the art (Hanahan, 1983). Complex media used during cultivation of the cells included LB-medium (1% NaCl, 0.5% yeast extract and 1% tryptone), YEPD-medium (1% yeast extract, 2% peptone, 2% glucose), or YEPG-medium (1% yeast extract, 2% peptone, 1% glycerol). Depending on the experimental set-up, *E. coli* cells were grown in 20, 200 or 400 mL complex media (LB, YEPD or YEPG medium) containing ampicillin (final concentration 100 $\mu$g/mL) up to an optical density, OD$_{600}$, of 1.0 at 180 rpm and 37°C (measurements performed on Fuostar Optima, BMG Labteach GmbH, Offenburg, Germany). For protein expression, isopropylthiogalactoside (IPTG), with a final concentration 0.5 mM, was added. *E. coli* cells were grown for additional 22 hours at 180 rpm and 20°C or 30°C depending on the experiment. FIG. 4 illustrates the expression of CAL-A, lipUMs and lipUMf in *E. coli* (DE3) strains.

Cultivation temperature was set to about 20°C during induction to avoid misfolding of the recombinant proteins and to reduce formation of insoluble protein aggregates. The hydrolytic activity of crude extracts from two *E. coli* strains, C41(DE3) and C43(DE3), expressing lipUMs or CAL-A was tested. The activity was measured photometrically with *p*-NP laurate (C$_{12}$) as a substrate. There is about a one-fold increase when lipUMs is expressed in C41 cells, rather than C43 cells, however the expression of CAL-A in each strain is about equivalent.

*Pichia pastoris* X33 cells (Invitrogen, Carlsbad, USA) were transformed with the following vectors: pPICZ(B), pPICZ(B) that contained the lipUMf sequence and pPICZ(B) that contained the CAL-A, lipUMf, or lipUMs sequence, or variants thereof, using standard procedures known to those of ordinary skill in the art (*see* for an example, EasySelect™ *Pichia* Expression Kit, Invitrogen, Carlsbad, USA). Successful integration of the three main lines, *i.e.,* CAL-A, lipUMf and lipUMs, and the variants thereof, was confirmed by PCR as described in the EasySelect™ manual, batch cultivations were performed as described for the secreted Mut$^+$ type of *Pichia pastoris* integrants as described in the EasySelect™ manual. The three lines of transformed cells were then grown in both buffered glycerol-complex medium (BMGY) and buffered methanol complex medium (BMMY) medium supplemented with Zeocin™ (copper-chelated *Streptomyces CL990* glycopeptide antibiotic; InvivoGen, C orp., San Diego, CA, USA). BMGY was used for preculturing of cells and BMMY was used in the main culture. 0.5% MeOH was added every 24 hours. After 4-5 days of grows the *Pichia pastoris* cells were harvested. FIG. 5 shows the PCR analysis of the integration of CAL-A, lipUMs and lipUMf into the genome of *P. pastoris* X33. Both CAL-A and lipUMs integrate into the genome of *P. pastoris* while lipUMf does not, suggesting that the N-terminal domain of lipUMf interacts with a target sequence and may therefore be part of a regulatory subunit.

*Cell harvesting and disruption*- For harvesting, transformed *E. coli* cells were centrifuged at 4500 $\times$ *g* for 20 min at 4°C. Cells were washed once with 50 mM sodium phosphate buffer that was kept on ice at pH 7.5. For disruption of cells cultured in a volume $\geq$ 200 mL, a French pressure cell (Thermo Scientific®, Waltham, MA, USA) was used. Cell pellets were resuspended in 30 mL of 50 mM sodium phosphate buffer that was kept on ice at a pH 7.5 containing 60

μL DNAseI (10 mg/mL). The cell slurry was passed through the French pressure cell twice at 10.324 MPa. Insoluble cell debris was removed by centrifugation at 11000 × g for 30 min at 4°C. The supernatant contained the soluble protein fraction. For cell disruption of smaller culture volumes (≤100 mL) a sonicator (BransonUltrasonics Corp, Danbury, CT, USA) was used. For this, the cell pellet was resuspended in 50 mM sodium phosphate buffer that was kept on ice in a volume 1/10 of the original cell culture. The suspension was sonicated at 0°C and interval pulsed at alternating cycles (Branson sonicator: 30% power and 50% pulse). Sonication was continued up to 20 mins depending on the culture volume. Insoluble cell debris was removed by centrifugation at 11000 × g for 30 min at 4°C. The supernatant contained the soluble protein fraction.

Harvesting of transformed *P. pichia* cells was performed using procedures as known to those of ordinary skill in the art using protocols provided in Invitrogen's EasySelect™ *Pichia* Expression Kit. Cells were harvested by centrifugation at 1.5000-3.000 × g for 30 mins at 4°C. The supernatant was then either stored at 4°C or concentrated further by centrifuge concentrators for small volumes (Amicon® Ultra 30 from Millipore (Billerica, MA, USA) and then stored at 4°C. For SDS-PAGE analysis the supernatant was concentrated by trichloroacetic acid (TCA) precipitation. The proteins of the supernatant were precipitated by adding 10% TCA for 12 hrs at 4°C. The precipitated proteins were washed successively in 100% and 80% acetone and dried at room temperature.

Table 4 shows the results of a fractionation study wherein LipUMs, LipUMf and CAL-A were expressed in *P. pastoris* X33 in the presence of different substrates, *i.e., p*-NP-C16, *p*-NP-C14, *p*-NP-C18:1Δ9*cis*, *p*-NP-C18-1Δ9*trans* and *p*-NP-C18:1Δ11*cis*. pPICZB and pPICZalphaB indicates *P. pastoris* cells transformed with empty vectors, and serve as controls. As can be seen from the table, lipUMf was not expressed and/or secreted in *P. pastoris* or did not act on the substrates present, however both CAL-A and lipUMs were present and active to varying degrees in the supernatant and whole cell-extract.

**TABLE 4**

| FRACTIONATION RESULTS FOR LIPUMS, LIPUMF AND CAL-A | | | | | | | | | | | | | | |
|---|---|---|---|---|---|---|---|---|---|---|---|---|---|---|
| | pPICZB | | | lipUMs | | | lipUMf | | | pPICZalphaB | | | CAL-A | | |
| | S | CE | UF | S | CE | UF | S | CE | UF | S | CE | UF | S | CE | UF |
| *p*NP-C16 | - | - | - | ++ | + | - | - | - | - | - | - | - | +++ | + | - |
| *p*NP-C14 | - | - | - | ++ | + | - | - | - | - | - | - | - | +++ | + | - |
| *p*NP-C18:1A9 *cis* | - | - | - | ++ | + | - | - | - | - | - | - | - | +++ | + | - |
| *p*NP-C18:1 Δ9 *trans* | - | - | - | ++ | + | - | - | - | - | - | - | - | +++ | + | - |
| *p*NP-C18:1Δ1 1 *cis* | - | - | - | ++ | + | - | - | - | - | - | - | - | +++ | + | - |
| ( - ) = no activity, (+) = activity, (++) = high activity, (+++) = very high activity, S: supernatant, CE: whole cell-extract, UF: unsoluble fraction | | | | | | | | | | | | | | |

*Protein determination by BCA-Assay-* For quantifying the protein content of the cell crude extracts, soluble and insoluble fractions or the purified enzyme, the BCA-assay system was used. The BCA-Assay kit from Uptima (Montlucon, France), was used according to the manufacturer's instructions.

*SDS-PAGE-* For the separation of proteins, discontinuous polyacrylamide gel electrophoresis (PAGE) was used (Laemmli, 1970) under denaturing conditions in the presence of SDS. For this 4% stacking gels combined with 10% or 12% stacking gels were used. For electrophoresis, the "Mini Protean® 3" gel apparatus system from Bio-Rad (München, Germany) was applied at 160V. Before electrophoresis, the protein samples were mixed with SDS, and sample buffer and then denatured for 5 min at 98°C. After electrophoresis, the separated proteins were either blotted on a polyvinylidene fluoride (PVDF) membrane (Carl Roth GmbH + Co. KG, Karlsruhe, Germany) or stained with Coomassie™ reagent. The gel was stained for at least 1 hour in staining solution and then destained with destaining solution until clear blue protein bands became visible. For visualization of separated protein bands, the SDS gel was stained for at least 3 hours in a solution containing Coomassie™ Brilliant Blue G-250, and then de-stained until clear, and blue protein bands became visible in the gel. To determine hydrolysis activity of the SDS-gel-separated lipases, the gel was blotted on a PVDF membrane and immersed in 100 mL renaturation solution for 1 hour. Then the gel was incubated for 2 to 20 min in a mixture of equal amounts of solution A [0.04% (wt./vol.) 1-naphthyl acetate, 10% (vol./vol.) acetone in sodium phosphate buffer (50 mM, pH 7.0)] and solution B [0.1% (wt./vol.) SigmaFast® (Fast Red; 4-chloro-2-methylbenzenediazonium/3-hydroxy-2-naphthoic acid 2,4-dimethylanilide phosphate; Sigma, Steinheim, Germany) in sodium phosphate buffer (50 mM, pH 7.0)]. Development of an insoluble red color indicated hydrolase activity of the enzyme (Higerd and Spizizen, 1973).

*Western blot*- For specific determination of protein bands in an SDS gel by antibodies or antibody conjugates, the Western Blot method is used. For blotting the proteins onto a PVDF membrane, the "semi-dry" method was applied using the HEP-3 Panther™ system (Owl SemiDry Electroblotting System; Thermo Scientific, Portsmouth, NH, USA) according to the manufacturer's instructions. The blotting was done at 15V with 0.8 mA/cm$^2$ PVDF membrane for 1.5 hrs. For blocking of unspecific binding sites in proteins, the membrane was incubated in skim milk solution or 5% bovine serum albumin (BSA) solution for 1 hour at room temperature. After washing the membrane 3 times for 10 min each with TBS-Tween buffer, the membrane was incubated for 1 hr in 1/1000 diluted Ni-NTA-conjugate solution. To remove unbound conjugate, the membrane was washed again three times in TBS-Tween® (polysorbate-20) buffer. For detection of specific protein bands, the PVDF membrane was incubated with 33 μL of 5-bromo-4-chloro-3-indolyl phosphate (BCIP) solution and 66 μL of nitro-blue tetrazolium chloride (NBT) solution in 10 mL detection buffer for 2 to 5 min. The color reaction was stopped by addition of 3% TCA.

FIG. 6 shows the SDS-PAGE and Western Blot results for Constructs I and III. Periplasmic expression of CAL-A led to higher activities in the supernatant than cytoplasmic expression, independent of *E. coli* strain chosen. Due to the accumulation of CAL-A in the periplasmic space, the outer membrane of the *E. coli* tends to break up, releasing CAL-A into the supernatant, which makes collection of the protein faster and easier.

*Purification of CAL-A, LipUMf, LipUMs Using Immobilized Metal Affinity Chromatography* - The presence of the C-terminal His-tag enabled a one-step purification procedure of CAL-A, lipUMf, lipUMs, or variants thereof by immobilized metal affinity chromatography (IMAC) with the Äktapurifier® (GE Healthcare, USA). The soluble protein fraction was applied to a chelating Sepharose column (Ni Sepharose 6 Fast Flow) preloaded with Ni$^{2+}$ (HisTrap™ FF crude, 5 mL, GE Healthcare, Uppsala, Sweden), equilibrated with 50 mM sodium phosphate buffer containing 300 mM NaCl and 30 mM imidazole, at pH 7.0. The column was washed with equilibrating buffer at a flow rate of 3 to 5 mL/min in order to remove the unbound material. The bound protein was eluted with equilibrating buffer supplemented with 300 mM imidazole. Fractions were collected, concentrated and buffer was exchanged for 50 mM sodium phosphate buffer (pH 7.0) with a polydextran desalting column (Sephadex G25, exclusion size 10 kDa) to remove the imidazole. The homogeneity of the purified protein was determined by SDS-PAGE as described above. For identification of certain proteins in specific bands, matrix-assisted laser desorption ionization-time of flight (MALDI-TOF) mass spectrometry with an Ultraflex TOF/TOF instrument (Bruker Daltonik GmbH, Bremen, Germany), and electrospray ionization mass spectrometry was performed with a quadrupole TOF (Q-TOF) instrument (Micromass Ltd., Manchester, UK).

*Protein Analysis and Enzyme Activity Determination* - The functional expression of CAL-A, lipUMf, lipUMs and variants thereof was followed by measuring hydrolytic activity according to a slightly modified protocol from Winkler and Stuckmann (1979), by measuring the initial hydrolysis rate of p-nitrophenyl esters (*p*-NP esters) in a 96-well micro titer plate (MTP) format with a Varioskan multiplate reader spectrophotometer (Thermo Scientific, USA) at λ = 410 nm (ε = 11.9 × 10$^3$ M$^{-1}$cm$^{-1}$). The assay solution contained the *p*-NP ester concentration (1 mM) in sodium phosphate buffer (pH 7.0, 50 mM). The reaction was initiated by addition of 30 μL enzyme solution to 270 μL of assay solution; absorbance was followed for 5 min. One unit of lipase activity is defined as the amount of enzyme releasing 1 μmol *p*-nitrophenol per mine. All measurements were performed in triplicate. Protein concentration was determined by the bicinchoninic acid (BCA) assay (Uptima, France) with bovine serum albumin as a standard. Expression of the recombinant lipases was also verified by Western blot analysis using a Ni-NTA-conjugate (Qiagen, Hilden, Germany). Samples were separated by sodium dodecyl sulfate-polyacrylamide gel electrophoresis (SDS-PAGE) according to the method of Laemmli (1970). Protein samples were loaded on a 10% SDS gel, together with a pre-stained molecular weight marker (Fermentas, Litvania). A mini protean II system (Biorad, Munich, Germany) was used for electrophoresis. After SDS-PAGE, the proteins were blotted onto a polyvinylidene fluoride (PVDF) membrane (Roth, Germany) by the semi-dry method. After blocking and washing the membrane, the Ni-NTA-conjugate was used to detect the C-terminal 6X His-tag. The complex was visualized by enzymatic color formation upon the addition of the 5-bromo-4-chloro-3-indolyl phosphate/nitro blue tetrazolium (BCIP/NBT) substrate (Sigma-Aldrich, Steinheim, Germany).

FIG. 7 is a graph that shows the expression analysis and activity test of recombinant expression of the various CAL-A constructs in *E. coli* when pNP-laurate (C12) is the substrate. Both Constructs I and III (pELB-CAL-A and ppro-CAL-A, respectively) demonstrate high enzyme activity rates in comparison with Constructs II and IV, which were expressed in the cytoplasm.

FIG. 8 is a graph that shows the expression analysis and activity test of the various CAL-A constructs transformed into DE3, Origami and Origami pGro7 cells in deep well plates when pNP-laurate (C$_{12}$) is the substrate. Periplasmic expression of ppro-CAL-A clearly demonstrated higher activity levels. Unidentified units represented the activity of 20 μL of supernatant. FIG. 18 similarly is a graph that shows an expression analysis and activity of various CAL-A constructs transformed into different *E. coli* strains when pNP-laurate (C$_{12}$) is the substrate. Periplasmic expression of CAL-A, as determined by the functional expression of pproCAL-A (full-length CAL-A including its pre- and pro-sequences) and pelB-CAL-A (the *E. coli* pelB signal sequence fused in-frame to the mature part of CAL-A), in C41, Tuner and BL21 cells demonstrates higher activity levels than cytoplasmic expression of CAL-A, as determined by the functional expression of mCAL-A (mature CAL-A) and Sumo-CAL-A (the mature part of CAL-A fused at its C-terminal to the Sumo solubility tag).

FIG. 9 is a graph that compares the hydrolytic activities of transformed *E. coli* C41 cells on three different cultivation media, standard Luria broth medium (LB), glycerol enriched cultivation medium (YEPG) and glucose enriched complex medium (YEPD), at 20°C and 180 rpm. The photometric assay was performed with pNP laureate (C12) as a substrate. The hydrolytic activity of crude extracts of CAL-A was increased by about twofold by cultivation on glucose-enriched medium, the activity of lipUMs increased up to fourteen times when used in glucose enriched medium.

FIG. 10 shows that when the soluble fraction was analyzed, only with the pNP-C14 substrate could significant activity be detected for lipUMs (Ums96). CAL-A demonstrated higher activity for the pNP-C16 substrate. This graph also indicated that a lipUMf was also functionally expressed in *E. coli* (DE3) strains; however, its activity was only 5% when compared to that of lipUMs expressed under the same conditions. In *P. pastoris* only lipUMs could be detected as a secreted protein, not lipUMf.

Table 5 shows a summary of the purification results of the lipase lipUMs and CAL-A. The lipases were expressed in the *E. coli* C41 (DE3) strain at 20°C and 180 rpm. Purification was performed by His-tag chromatography followed by gel filtration. pNP myristate ($C_{14}$) was used as a substrate.

**TABLE 5**

**PURIFICATION RESULTS OF THE LIPASES CAL-A AND LIPUMS**

| Lipase | Purification step | Total protein [$mg_{protein}$/ml ] | Spec. activity [$U/mg_{protein}$] | Yield [%] | Purification factor |
|---|---|---|---|---|---|
| CAL-A | Crude extract | 145 | 0.27 | 100 | 1 |
| | $His_6$-tag | 4.9 | 29 | 52 | 109 |
| | $His_6$-tag | 9 | 0.29 | 1.6 | 10 |
| lipUMs | Crude extract | 28 | 1.1 | 100 | 1 |
| | $His_6$-tag | 0.26 | 40 | 4.5 | 36 |

High total protein and yield was observed for CAL-A, leading to a higher purification factor. Therefore, this purification procedure was optimal for obtaining high quantities of CAL-A, or variants thereof. High specific activities of 40 U/mg were found for lipUMs, which exceeded the value of 29 U/mg for CAL-A, demonstrating the preference of lipUMs for long chain ($C_{14}$ or greater) length fatty acids using this purification procedure.

FIG. 11 shows a Coomassie stained 10% SDS-PAGE gel, which identifies different forms of CAL-A and lipUMs. These fractions were purified by His-tag purification. MALDI-TOF analysis further confirmed that the purified protein product in this experiment was that of lipUMs.

## EXAMPLE 3 - MUTAGENESIS

*Sequencing* - DNA and protein sequencing was performed by published methods. DNA sequencing was performed by GATC Biotech AG (Konstanz, Germany).

*Saturation mutagenesis of CAL-A* - Table 6 shows the 12 sites chosen for saturation mutagenesis in CAL-A variants. For all the sites, the mutants could be created either by site-directed single mutagenesis or site-directed saturation mutagenesis. For the selected amino acid positions, a numerical mutagenesis score is also given. A higher mutagenesis score indicates that multiple amino acids, including dissimilar ones, can be swapped into this position with a low probability of compromising the function of the protein (Pavelka *et al.,* 2009). This score and the amino acid frequency, or number of occurrences of the individual amino acids at the position of the multiple sequence alignment corresponding to the residue, was calculated using the HotSpot Wizard tool.

**TABLE 6**

| AMINO ACID POSITIONS FOR MUTAGENESIS, MUTAGENESIS SCORES AND AMINO ACID FREQUENCY | | | | | | | | | | | |
|---|---|---|---|---|---|---|---|---|---|---|---|
| **Residue** | F 149 | 1150 | A 218 | T 221 | F 222 | L225 | F233 | G 237 | L 241 | M 248 | I 301 | L 305 |
| **Mutability** | low | Average | low | low | Average | Average | low | low | low | Average | Average | Average |
| **Amino Acid Frequency Multiple Sequence Alignment** | 42xF, 5×Y, 2xW, 1×L | 5×T, 11×G , 8xL, 5×A, 4xS, 4xV, 2×I, 1×M | 27×I, 12xL, 6xV, 2xA, 1×M, 1×P, 1×T | 21xT, 15xV , 5×L, 4×I, 2xM, 1×F, 1×N, 1×W | 9xA, 8×I, 7xL, 6xF, 3xM, 3xV, 2xY, 1×P, 1×T | 19×V , 12xL, 9×I, 6xT, 1×A, 1×C, 1×N, 1×S | 33xL, 11×F, 3×I, 2xV, 1×Y | 26xA 21×G , 1×L, 1×S, 1×T | 35xL, 6xV, 5×I, 2xY, 1×M, 1×Q | 18xL, 10×V, 9xF, 5×I, 3xA, 2xR, 1×M, 1×Q, 1×T | 17xV 13×I, 8xA, 4xF, 3xL, 3xP, 1×G, 1×M | 25xL, 7×I, 6xV, 3xF, 3xM, 3xT, 1×A, 1×E, 1×W |

*Site-directed mutagenesis* - Site-directed mutagenesis was used to generate substitutions at targeted codons of the gene of interest. A modified site-directed mutagenesis protocol was applied, which was based on the commercial Quickchange® site-directed mutagenesis kit protocol (Stratagene, Inc., La Jolla, CA, USA). To introduce specific amino acid substitutions in the amino acid sequence of the gene of interest, mutagenic primers (between 22 and 30 bases in length) were designed to contain the desired DNA codon that would result in the targeted amino acid substitution. For optimal primer design the PrimerX program was used as available online. The most suitable primer pair suggestion was identified and modified manually (Zheng *et al.,* 2004). In particular aspects, the pair of amplification primers includes a first and second oligonucleotide primers of less than about 50, preferably less than about 40, and more preferably still, less than about 30 nucleotides each in length that comprises, consists essentially of, or alternatively, consists of the nucleic acid sequence as identified by the above-noted process. In order to reduce the number of mutants to be screened without compromising on the quality of the library, the adapted saturation codon NDT (12 possible codons) was used (Reetz, 2008). With the NDT codon only 12 amino acids are encoded representing all types (neutral, basic, acidic, aromatic, and aliphatic) of amino-acid functional groups. The use of the NDT codon in saturation mutagenesis drastically reduces the screening effort. Next, all 24 mutagenic primers were designed and focused libraries were created at the 12 preselected sites: F149, I150, A218, T221, F222, L225, L225, F233, G237, L241, M248, I301 and L305 using standard protocols (adjusted QuikChange) for the introduction of the mutations into the CAL-A encoding gene. The commercially-available *E. coli* strain NEB 5-α F'Iq (New England Biolabs, Frankfurt, Germany) was used to increase the transformation efficiency of *E. coli* in the first round. For expression of the CAL-A variants, *E. coli* strain C41 (DE3) was used. In this strain, the transcription and translation of recombinant proteins is more balanced than in other *E. coli* expression strains, resulting in increased yields of properly folded, active enzyme. By combining all these steps, high mutagenesis and transformation efficiencies could be achieved. Roughly ~5000 transformants in the 96-well format needed to been screened in total.

Table 7 shows the primers used to create the point mutations in CAL-A and lipUMs. Forward primers (FW) are listed first, followed by the corresponding reverse primer (RV). Target sequences are indicated in parenthesis in the primer name.

### TABLE 7
### PRIMERS USED TO CREATE CAL-A AND LIPUMS MUTANTS

| SEQ ID NO. | Primer Name (Point Mutation - Forward/Reverse) | Sequence |
|---|---|---|
| **Oligos used in the NDT-saturation mutagenesis experiments of pET22-pproCAL-Aophis (pproCAL-A):** | | |
| SEQ ID NO:23 | F149(NDT)-FW | GCGGCGNDTATTGCGGGCTATGAAG |
| SEQ ID NO:24 | F149(AHN)-RV | CCGCAATAHNCGCCGCTTTAAAGCC |
| SEQ ID NO:25 | I150(NDT)-FW | GCGTTTNDTGCGGGCTATGAAGAAG |
| SEQ ID NO:26 | I150(AHN)-RV | GCCCGCAHNAAACGCCGCTTTAAAG |
| SEQ ID NO:27 | A218(NDT)-FW | CCCGGTGAGCNDTAAAGATACCTTTACCTT |
| SEQ ID NO:28 | A218(AHN)-RV | GGTATCTTTAHNGCTCACCGGGGTGCCACC |
| SEQ ID NO:29 | T221(NDT)-FW | GCGAAAGATNDTTTTACCTTTCTGAACGG |
| SEQ ID NO:30 | T221(AHN)-RV | GAAAGGTAAAAHNATCTTTCGCGCTCACCG |
| SEQ ID NO:31 | F222(NDT)-FW | GATACCNDTACCTTTCTGAACGGCG |
| SEQ ID NO:32 | F222(AHN)-RV | GAAAGGTAHNGGTATCTTTCGCGCTC |
| SEQ ID NO:33 | L225(NDT)-FW | CTTTACCTTTNDTAACGGCGGTCCGTTTGCGG |
| SEQ ID NO:34 | L225(AHN)-RV | GACCGCCGTTAHNAAAGGTAAAGGTATCTTTC |
| SEQ ID NO:35 | F233(NDT)-FW | GCGGGTNDTGCGCTGGCCGGTGT |
| SEQ ID NO:36 | F233(AHN)-RV | CAGCGCAHNACCCGCAAACGGAC |
| SEQ ID NO:37 | G237(NDT)-FW | CTGGCCNDTGTGAGCGGTCTGAGC |
| SEQ ID NO:38 | G237(AHN)-RV | CGCTCACAHNGGCCAGCGCAAAAC |
| SEQ ID NO:39 | L241(NDT)-FW | GCGGTNDTAGCCTGGCCCATC |
| SEQ ID NO:40 | L241(AHN)-RV | CAGGCTAHNACCGCTCACACCG |
| SEQ ID NO:41 | M248(NDT)-FW | CCGGATNDTGAAAGCTTTATTGAAGCGC |
| SEQ ID NO:42 | M248(AHN)-RV | GCTTTCAHNATCCGGATGGGCCAGGCT |
| SEQ ID NO:43 | I301(NDT)-FW | GAAGCGCCGNDTGCGAGCATTCTGAAACAGGAA |
| SEQ ID NO:44 | I301(AHN)-RV | GAATGCTCGCAHNCGGCGCTTCGTTCAGCAGGTT |
| SEQ ID NO:45 | L305(NDT)-FW | GCGAGCATTNDTAAACAGGAAACCGTTGTT |

## PRIMERS USED TO CREATE CAL-A AND LIPUMS MUTANTS

| SEQ ID NO. | Primer Name (Point Mutation - Forward/Reverse) | Sequence |
|---|---|---|
| **Oligos used in the NDT-saturation mutagenesis experiments of pET22-pproCAL-Aophis (pproCAL-A):** | | |
| SEQ ID NO:46 | L305(AHN)-RV | GTTTCCTGTTTAHNAATGCTCGCAATCGGCGCT |
| **Rational CAL-A variants expressed in *P. pastoris*:** | | |
| SEQ ID NO:47 | L305H(CAC)-FW | GCATCCACAAGCAGGAGACG |
| SEQ ID NO:48 | L305H(GTG)-RV | CTGCTTGTGGATGCCAGCGAT |
| SEQ ID NO:49 | L305C(TGC)-FW | CTGGCATCTGCAAGCAGGAGACGGT |
| SEQ ID NO:50 | L305C(GCA)-RV | CTGCTTGCAGATGCCAGCGATC |
| SEQ ID NO:51 | L305N(TTC)-FW | CTGGCATCTTCAAGCAGGAGACGGT |
| SEQ ID NO:52 | L305N(GAA)-RV | CCTGCTTGAAGATGCCAGCGATCG |
| **Rational CAL-A variants expressed in *P. pastoris* for flap removal:** | | |
| SEQ ID NO:53 | G387*-FW | GTTTGATTGAACCACCCCGAAAGTGAT |
| SEQ ID NO:54 | G387*-RV | GGGTGGTTCAATCAAACGCCTGTTTAA |
| SEQ ID NO:55 | D425*-FW | GTAGCCTGTAAGGCAAACAGAGCGCGT |
| SEQ ID NO:56 | D425*-RV | GTTTGCCTTACAGGCTACGCAGCTGG |
| **Rational LipUMs variants expressed in *P. pastoris*:** | | |
| SEQ ID NO:57 | G233A_proUMs | ATTGCGGCTGTCTCTGGCCTGG |
| SEQ ID NO:58 | G233A_proUMs | AGAGACAGCCGCAATTGCGAAG |
| SEQ ID NO:59 | G233V_proUMs | ATTGCGGTTGTCTCTGGCCTG |
| SEQ ID NO:60 | G233V_proUMs | GAGACAACCGCAATTGCGAA |
| SEQ ID NO:61 | G233Y_proUMs | CAATTGCGTATGTCTCTGGCCTGGCTT |
| SEQ ID NO:62 | G233Y_proUMs | CAGAGACATACGCAATTGCGAAGCCGG |
| SEQ ID NO:63 | G233L_proUMs | ATTGCGCTTGTCTCTGGCCTGG |
| SEQ ID NO:64 | G233L_proUMs | AGAGACAAGCGCAATTGCGAAGC |
| **Rational CAL-A variants as expressed in *P. pastoris*:** | | |
| SEQ ID NO:65 | F149D_proCAL-A_FW | GCCGCCGACATCGCTGGCTACG |
| SEQ ID NO:66 | F149D_proCAL-A_RV | GCGATGTCGGCGGCTTTGAAGC |
| SEQ ID NO:67 | A218N_proCAL-A_FW | CGTGAGCAACAAGGACACCTT |
| SEQ ID NO:68 | A218N_proCAL-A_RV | GTCCTTGTTGCTCACGGGCGT |
| SEQ ID NO:69 | T221H_proCAL-A_FW | CAAGGACCATTTTACATTCCTCA |
| SEQ ID NO:70 | F221H_proCAL-A_RV | GTAAAATGGTCCTTGGCGCTC |
| SEQ ID NO:71 | F222S_proCAL-A_FW | GACACCTCGACATTCCTCAACGG |
| SEQ ID NO:72 | F222S_proCAL-A_RV | GAATGTCGAGGTGTCCTTGGCG |
| SEQ ID NO:73 | I301H_proCAL-A_FW | GCGCCGCACGCTGGCATCCTC |
| SEQ ID NO:74 | I301H_proCAL-A_RV | GCCAGCGTGCGGCGCCTCGTTG |
| **Rational lipUMs variants expressed in *P.pastoris*:** | | |
| SEQ ID NO:75 | F145D _proUMs_FW | GCTCAAGCGACATTGCAGGCTAT |
| SEQ ID NO:76 | F145D _proUMs_RV | CTGCAATGTCGCTTGAGCGAGGG |
| SEQ ID NO:77 | A214N_proUMs_FW | CAGCAAGTAACCGTGATACCTTTAA |
| SEQ ID NO:78 | A214N_proUMs_RV | GTATCACGGTTACTTGCTGGCAGAC |
| SEQ ID NO:79 | T217H_proUMs_FW | GCGTGATCATTTTAACTTCTTGAA |
| SEQ ID NO:80 | T217H_proUMs_RV | GAAGTTAAAATGATCACGCGCACTTG |
| SEQ ID NO:81 | F218S _proUMs_FW | GATACCTCGAACTTCTTGAACAA |
| SEQ ID NO:82 | F218S _proUMs_RV | GAAGTTCGAGGTATCACGCGCA |
| SEQ ID NO:83 | V297H_proUMs_FW | GAGCCGCACGCCTCCACATTGA |
| SEQ ID NO:84 | V297H _proUMs_RV | GTGGAGGCGTGCGGCTCTTCGTTC |
| SEQ ID NO:85 | L301N_proUMs_FW | CTCCACAAACAAGTCAGAAACGCT |
| SEQ ID NO:86 | L301N_proUMs_RV | CTGACTTGTTTGTGGAGGCCACCG |

(continued)

**Oligos used to make CAL-A double mutants for expression in *E.coli* C41 (DE3) to combine the best hits of the PH soy assay:**

| SEQ ID NO:87 | I301H_proCAL_FW | GCCGCATGCGAGCATTC |
| SEQ ID NO:88 | I301H_proCAL_RV | CTCGCATGCGGCGCTT |
| SEQ ID NO:89 | T221H_proCAL_FW | GCGAAAGATCACTTTACCTTTCTG |
| SEQ ID NO:90 | T221H_proCAL_RV | GGTAAAGTGATCTTTCGCGCTC |

**Primers used to make rational CAL-A mutants with removed flap mutants for expression in E. coli C41(DE3):**

| SEQ ID N0:91 | I401*_proCAL_FW | CGGCGTAGGCGGGCATTACCA |
| SEQ ID NO:92 | I401*_proCAL_RV | CCGCCTACGCCGGAATCGG |
| SEQ ID NO:93 | D425*_proCAL_FW | GTAGCCTGTAGGGCAAACAGAGCGC |
| SEQ ID NO:94 | D425*_proCAL_RV | GTTTGCCCTACAGGCTACGCAGCT |

**Oligos used to make lipUMs medium chain length (MCL) mutants for expression in *E. coli* C41(DE3) at positon G233:**

| SEQ ID NO:95 | G233A_proUMS_FW | ACCGTTTCTTAGCATTAGCCCGT |
| SEQ ID NO:96 | G233A_proUMS_RV | CTCGCATGCGGCGCTTC |
| SEQ ID NO:97 | G233L_proUMS_FW | ACCGTTTCTTAGCATTAGCCCGT |
| SEQ ID NO:98 | G233L_proUMS_RV | CTAATGCTAAGAAACGGTTTATTGC |
| SEQ ID NO:99 | G233V_proUMS_FW | CCGTTTGTTAGCATTAGCCCG |
| SEQ ID NO: 100 | G233V_proUMS_RV | GGCTAATGCTAACAAACGGTTTATT |
| SEQ ID NO:101 | G233Y_proUMS_FW | CAATTGCATACGTTAGCGGTCTGG |
| SEQ ID NO: 102 | G233Y_proUMS_RV | CCGCTAACGTATGCAATTGCAAAAC |

**Oligos used to make lipUMs double mutants for expression in *E. coli* C41(DE3) to combine the best hits of the PH soy assay:**

| SEQ ID NO:103 | T217H_proUMS_FW | CACGTGATCACTTTAATTTTCTGA |
| SEQ ID NO:104 | T217H_proUMS_RV | GAAAATTAAAGTGATCACGTGCGCTG |
| SEQ ID NO:105 | V297H_proUMS_FW | GAACCGCATGCAAGCACCCTG |
| SEQ ID NO:106 | V297H_proUMS_RV | GCTTGCATGCGGTTCTTCATTC |
| SEQ ID NO:107 | V297I_proUMS_FW | GAACCGATAGCAAGCACCCTGAA |
| SEQ ID NO:108 | V297I_proUMS_RV | GCTTGCTATCGGTTCTTCATTCA |

The 50-$\mu$L reaction mixture contained each 2.5 ng/$\mu$L of upstream and downstream primers, 1 ng/$\mu$L of template plasmid, 200 $\mu$M of dNTP-Mix (Roth, Karlsruhe, Germany), and 0.05 U Pfu Turbo DNA polymerase from Roboklon (Berlin, Germany). The recommended PCR buffer for the Pfu Turbo DNA polymerase was applied. PCR was performed using the Touchgene Gradient TPersonal PCR-machine (Techne, Cambridge, UK). The following PCR program was used: 1 × 1 min @ 95°C, 18 × 1 min @ 95°C, 1 min at the $T_M$ of the primer with the lowest melting temperature, 12 min at 72°C). The DNA was digested with the restriction enzyme *Dpn*I (Fermentas, St. Leon-Rot, Germany) to degrade non-mutagenized parental DNA. The PCR-products were transformed into *E. coli* cells NEB 5-$\alpha$ F'Iq chemo-competent cells available from NEB New England Biolabs (Frankfurt, Germany). The *Dpn*I digestion was performed according to protocols provided by Fermentas, Inc. The transformation was done by the heat shock method (T = 42°C @ 45 sec) as described by Hanahan (1983). Mutants were confirmed by sequencing. This procedure was used for the expression of both lipUMs, CAL-A and variants thereof, in both *E. coli* and *P. pastoris* cells.

## EXAMPLE 4 - ANALYSIS OF CAL-A VARIANT SUBLIBRARIES

As an example for the analysis of NDT sublibraries, the sublibrary G237 (negative example) and L305 (positive example) were chosen. Due to the standard deep-well plate setup, 70 members of a library were analysed at the same time.

FIG. 13 shows that no library member with a ToC value higher than the wild type CAL-A could be detected within the G237 library. The ToC values were either lowered or the mutations were neutral. Similar results were obtained for the sublibraries M248X, F233X, L225X, A218X and I150X. False positives suggested a high or very high ToC because very low activity or even inactivity was observed, similar to the empty vector control included in the assay system. The observation was made that lower residual activity lead to a higher error in the TOC-values. Because of this, the residual activity of the mutants was evaluated as a second parameter. In the example for the G237X sublibrary, the residual activity of many mutants was not abolished but significantly lowered to about 50% of the wild-type activity.

Library L305, as the positive example, shows a different trend. Here, the activities were also strongly decreased similar to the transformants in the G237 library. However, this library showed many transformants with ToC-values *higher* than wild-type CAL-A.

In order to evaluate the quality of the sublibraries, outliers with increased selectivity were sequenced and re-tested with p-nitrophenylesters.

Table 8 shows the evaluation of the top sublibraries for *trans*-selectivity by demonstrating a comparison of *trans*-preference of CAL-A and lipUMs for pNP C18:1Δ9*trans* and pNP C19:1Δ9*cis* as substrate determined as quotient "Trans over Cis" (ToC). ToC aids in discriminating more *trans*-selective lipases from neutral or more *cis*-selective ones during the screening process. The initial velocities of these reactions were measured spectrophotometrically in a microtiter plate format (MTP).

**TABLE 8**

| *TRANS* OVER *CIS* PREFERENCE OF CAL-A POINT MUTANTS | | | | | |
|---|---|---|---|---|---|
| CAL-A Point Mutants | ToC | Factor of Improvement/data normalized to WT | % of WT activity Elaidinate | % of WT activity Oleate | Position of Mutation in Topology |
| F222S | 15 | 6 | 21 | 3 | Alpha helix A5: first helix in lid |
| F149D | 12 | 5 | 17 | 3 | Unstructured loop near active site |
| F222C | 8 | 3 | 8 | 2 | Alpha helix A5 |
| I130H | 6 | 2 | 8 | 3 | Alpha helix A10: last helix in lid |
| T221H | 5 | 2 | 5 | 2 | Alpha helix A5 |
| I305N | 4 | 2 | 19 | 8 | Alpha helix A10 |
| A218N | 4 | 2 | 25 | 15 | Alpha helix A5 |
| WT | 2,5 | 1 | 100 | 100 | n/a |

**EXAMPLE 5 - CHARACTERIZATION OF CAL-A AND LIPUMs**

Fatty acid or carboxylic acid moiety chain length profiles of lipUMs and CAL-A were determined. *p*NP esters of different chain lengths (C2 to C18) were used.

*Synthesis of p-NP oleate and p-NP elaidate* - To a 250-mL round-bottom flask, with magnetic stirring, under an inert atmosphere ($N_2$), the following was added: anhydrous zinc chloride ($ZnCl_2$) (1.5 g, 11.0 mmol), anhydrous dichloromethane (75 mL) and *p*-nitrophenol (2.8 g, 20.1 mmol). Oleic acid chloride (2.8 g, 8.83 mmol) was added dropwise by a syringe and the reaction mixture was refluxed until complete conversion was observed by thin layer chromatography (~1 hr). The reaction mixture was then cooled to room temperature, 75 mL water was added and the organic layer separated. The aqueous phase was extracted with diethyl ether ($Et_2O$) ($2 \times 75$ mL). The combined organic layers were further washed with sodium bicarbonate ($NaHCO_3$) ($2 \times 75$ mL). The organic layer was then dried with anhydrous sodium sulfate ($Na_2SO_4$), filtered and reduced *in vacuo* to give a dark brown oil. Purification was performed by flash chromatography (10:1 hexane:$Et_2O$) to afford the pure *p*-nitrophenol oleate (2.228 g, 5.51 mmol, 62 %) as a clear, colorless oil. $^1$H-NMR (300MHz; dilute solution in $CDCl_3$) δ = 8.27 (2H, d, *J*=9.25Hz, 2xAr-H), 7.27 (2H, d, *J*=9.25Hz, 2xAr-H), 5.43-5.30 (2H, m, -HC=CH-), 2.60 (2H, t, *J*=7.55Hz, α-$CH_2$), 2.11-1.91 (4H, m, $H_2$CHC=CHCH$_2$), 1.83-1.69 (2H, m, β-$CH_2$), 1.49-1.19 (20H, m), 0.88 (t, *J*=6.61Hz, $CH_3$). The analogous procedure for elaidic acid chloride led to the *p*-nitrophenol elaidate ester (64%) as a pale brown solid. $^1$H-NMR (300MHz; dilute solution in $CDCl_3$) δ = 8.27 (2H, d, *J*=9.30Hz, 2xAr-H), 7.27 (2H, d, *J*=9.30Hz, 2xAr-H), 5.40-5.37 (2H, m, -HC=CH-), 2.60 (2H, t, *J*=7.50Hz, α-$CH_2$), 2.01-1.91 (4H, m, $H_2$CHC=CHCH$_2$), 1.81-1.70 (2H, m, β-$CH_2$), 1.45-1.21 (20H, m), 0.90 (t, *J*=6.60Hz, $CH_3$). All other pNP esters used were obtained commercially from NU-Check Prep Inc. (Elysian, MN, USA).

*Spectrophotometric determination of lipase activity by para-nitrophenol esters* - Hydrolytic activity of culture supernatants or crude extracts were measured according to the method described by Winkler and Stuckmann (1979). The *p*-NP esters of different fatty acids were dissolved in 10 mL isopropanol and mixed with 90 mL of sodium phosphate buffer (pH 7.5, 50 mM), supplemented with gum arabic (100 mg). The final concentration of the substrates was 1 mM.

The reaction was initiated by addition of 20 $\mu$L enzyme solution to 180 $\mu$L assay solution (solution C) and adsorbance was followed for 5 min. In the assay the initial hydrolysis rate of *p*-nitrophenyl esters (*p*-NP esters) was determined in a 96-well MTP format with a Varioskan multiplate reader spectrophotometer (Thermo Scientific) at $\lambda$ = 410 nm ($\varepsilon$ = 11.9 $\times$ $10^3$ $M^{-1}cm^{-1}$). One unit of lipase activity is defined as the amount of enzyme releasing 1 $\mu$mol *p*-nitrophenol per min. All measurements were performed in triplicate.

*Determination of the cis- or trans- fatty acid selectivity of lipases in pH soy oil* - All reaction and work-up procedures were performed using glassware to prevent contamination by fatty acid like plasticizers such as *bis*(2-ethylhexyl)phthalate (DEHP). All reactions were performed in glass-vials with magnetic stirring. Mole calculations were performed assuming average molecular weight of the mixture was equal to triolein (885 g/mol).

The reaction was run in triplicate by using three separate reaction vessels. The reaction mixture was prepared by weighing partially hydrogenated soybean (soy) oil (PHSO) (1000 mg; 1.13 mmol of triacylglycerol (TAG) or 3.39 mmol of cleavable esters) into a tube and flushing with nitrogen. The PHSO was then fully melted by immersion in hot water (40°C). The PHSO was cooled and gum arabic (400 mg) and phosphate buffer (50 mM; pH 7.5; 20 mL) added. The mixture was emulsified by shear-mixing (24,000 rpm) for 2 min. Aliquots of this mixture (4 mL; 200 mg /0.678 mmol of FA equivalents) were added to each of the three reaction vessels equipped with magnetic stirring. The reaction mixture was heated to 40°C. Enzyme was either added to or prepared in phosphate buffer (50 mM; pH 7.5) and added to the reaction mixture. The reaction mixtures were sampled at appropriate times (either a time course of samples or at 20% conversion). Sampling was performed by removing an aliquot (0.5 mL; 25 mg/85 $\mu$mol of FA equivalents) and extracting between hydrochloric acid (HCl) (0.5 M; 1.5 mL) and hexane:chloroform ($CHCl_3$):methanol (MeOH) (49:49:2; 2 $\times$ 2 mL) in a glass vial. The combined organics were dried by filtering through $Na_2SO_4$ in a glass pipette and the solvent removed under nitrogen. When the solvent was fully removed, the remaining oil was quickly resuspended in hexane (1 mL) and a few drops removed for analysis by TLC-FID (Iatroscan). Derivatisation for gas chromatography analysis was performed by adding sodium methoxide (NaOMe) (2 N in MeOH; 50 $\mu$L; 100 $\mu$mol) and vortex mixing the sample for 1 min. The resulting mixture was filtered through $Na_2SO_4$ in a glass pipette to remove MeOH, residual NaOMe and solid precipitate, and the resulting filtrate analyzed by gas chromatography. For samples taken at high conversion, concentration of the resulting solution (low TAG and therefore low FAME concentrations) under nitrogen was required.

*Thin Layer Chromatography-Flame Ionization Detector (TLC FID)* - 1 $\mu$L of the extract was applied to each of three chromarods and developed in 67:3:0.5 toluene:hexane:acetic acid (AcOH) to the top of the chromarod. The chromarods were dried under a stream of nitrogen. Scanning was performed at 160 ml/min hydrogen, 2 1/min air and a 40 second scan rate, and recorded on a chart printer. Integration of the peaks was performed by scanning the image into a computer and manually integrating using ImageJ (NIH software). Appropriate correction factors were applied, and the results of the three scans averaged and standard deviation error calculated.

*Gas-chromatography (GC)* - The official Ce1h-05 AOCS method was used, with the exception that the column temperature was reduced from 180°C to 170°C, as the mobile phase. The FAME CP-select column (Varian, CA, USA) was used as the stationary phase. Peaks were integrated manually, and the appropriate correction factors (Ce-1h 05) were applied. The triplicate data (including the further triplicate determination of TLC-FID data) of conversion percentage and *trans*-content of the bound fraction was averaged and standard deviation error calculated. The *trans*-content of the FAEE fraction was converted to *trans*-content of the bound fraction as described.

FIG. 12 shows the chain length preferences of CAL-A. CAL-A showed a preference for medium chain length fatty acids and pNP myristate ($C_{14}$) and caproic acid ($C_6$) in particular. CAL-A also demonstrates a preference for long chain fatty acids.

The fatty acid profiles were determined as described above with *p*-NP esters of acetate ($C_2$), butyrate ($C_4$), caproate ($C_6$), caprylate ($C_8$), ($C_9$), caprate ($C_{10}$), laurate ($C_{12}$), myristate ($C_{14}$), palmitate ($C_{16}$), stearate ($C_{18}$), obtained from Sigma-Aldrich.

To make CAL-A specific for medium chain length fatty acid esters, a position was searched to block the primary acyl binding tunnel near the junction to the alternative binding pocket. As seen in **FIG. 20,** position G237 in $\alpha$-helix $\alpha$D2 of CAL-A was found to be a good candidate for this undertaking, since substitutions at this position with amino acids a bit larger would block the primary tunnel completely, bypassing the substrate to the alternative binding tunnel. Four different CAL-A mutants were generated at position G237 and their chain length profile was determined with pNP-esters and triacylglycerides as substrates. The substitutions were in detail: G237A, predicted to narrow the primary tunnel, G237V, hypothesized to constrict the main tunnel almost completely and the two substitutions G237L and G237Y, supposed to block the main tunnel, thereby shunting medium chain length substrates to the alternative binding tunnel. As can be seen in **FIG. 21,** the chain length profile of CAL-A (WT), determined photometrically with pNP-esters, shows, that CAL-A (WT) possess no pronounced chain length specificity, *i.e.* CAL-A (WT) does not differentiate between long chain and medium chain fatty acid esters. It accepts medium chain length fatty acid esters of C6 and C8 as good as long chain fatty acid esters of C14 and C16 chain length. Further, the CAL-A variant G237A, expected to have an intact but narrowed primary acyl-binding tunnel, showed an overall strongly repressed hydrolytic activity for pNP-esters as substrates. When triaclyglycerides of different chain length were used as substrates in pH-stat experiments, as seen in **FIG. 22,** however, the hydrolytic activity of CAL-A(WT) decreased with increasing chain length of the fatty acid moiety with the exception of the TAG (C6:0). This might be due to the lowered solubility of long-chain triacylglycerides despite the use of gum arabic. When using triacylglycerides as substrates **(FIG. 22),** CAL-A variant G237A showed a hydrolytic activity in similar range to CAL-A (WT) up to a chain length of C8., The hydrolytic activity of variant G237A was strongly reduced when exposed to triacylglycerides of C10 chain length and above. In particular, the CAL-A variants G237V and G237Y showed a strongly, up to three times, increased activity for the hydrolysis of pNP-butyrate and pNP-hexanoate as compared to CAL-A (WT). More importantly, all three variants, G237L, G237V and G237Y, do discriminate between ester substrates of a chain length of C6 and those which are longer. For fatty acids longer than C8, hydrolysis of pNP-esters is completely abolished in all three variants. This effect can also be observed for the hydrolysis of TAGs by the three mutants G237L, G237V and G237Y. Here, the three mutants discriminate sharply between fatty acids esters of a length up to C6 and those, which are longer. Starting with tricaprylin (C8) the hydrolytic activity of the mutants G237L, G237V and G237Y is strongly reduced for any triacylglyceride longer than this. Mutants G237L and G237V differentiate strongly between tricaprin (C6) and tricaprylin (C8). In contrast to this, mutant G237A discriminates triacylglycerides longer than C8.

FIG. 13 shows the chain length preference for four CAL-A variants, *i.e.*, G237A, G237L, G237V and G237Y as compared to wild-type CAL-A. Both the G237V and G237Y mutants demonstrated higher activity than wild-type CAL-A for C16 and C4 chain lengths. The activity values were normalized and the highest value of CAL-A (WT) was set to 1. While mutants G237L and G237V lead to a drastic distinction between $C_6$ and $C_8$ ester substrates, the mutants G237A and G237Y show very low residual activity for the hydrolysis of $C_8$ to $C_{10}$ substrates. Substrates longer than $C_{10}$ were not hydrolyzed by these mutants.

Table 9 shows a comparison of *trans*-preference of CAL-A and lipUMs for pNP C18:1Δ9*trans* and pNP C19:1Δ9*cis* as substrate determined as quotient "Trans over Cis" (ToC) for more than 50 clones each. The initial velocities of these reactions were measured spectrophotometrically in a microtiter plate format (MTP). The preference for *trans* vaccenic acid (C18:1 Δ11 *trans*) over oleic acid is slightly higher than for elaidic acid over oleic acid. The standard deviation, which includes systematic and other experimental errors, was plus or minus 20% for each of the measurements.

**TABLE 9**

**COMPARISON OF *TRANS*-PREFERENCE OF CAL-A AND LIPUMS**

| Trans over Cis preference (ToC) | CAL-A | LipUMs |
|---|---|---|
| pNP vaccinate vs pNP oleate | 3.0 ± 20% | n/a |
| pNP elaidate vs pNP oleate | 2.5 ± 20% | 1.7 ± 20% |

FIG. 14 shows the ToC values, as determined using elaidic acid p-nitrophenyl ester hydroylsis over oleic acid *p*-nitrophenyl ester hydrolysis of CAL-A normalized to random samples of CAL-A. The *trans* selectivity of the lipases was determined using *p*-NP elaidate (C18:1 Δ9 *trans*) or oleate (C18:1 Δ9 *cis)* synthesized chemically as described above. Oleate was chosen as it constitutes roughly 25% of PH soy oil and is the most abundant fatty acid therein. This diagram shows that the tested CAL-A cells show highly reproducible ToC values. All ToC values fall within the ±20 threshold area except one outlier.

FIG. 15 shows the residual activities of CAL-A normalized to random measurements of either hydrolysis activity of *p*-nitrophenyl-elaidic acid (E-activity) or *p*-nitrophenyl-oleic acid (O-activity) of CAL-A samples.

**FIG. 16** demonstrates the preference of CAL-A for pNP-esters with *trans*-fatty acids when transformed into *E. coli.* Five different $C_{18}$ fatty acid chains, *i.e.* stearic acid ($C_{18}$), vaccenic acid (C18V), elaidic acid (C18E), oleic acid (C18O), and linolenic acid (C18L), were used as substrates. CAL-A preferentially acted on the *trans* substrates, *i.e.* vaccenic acid and elaidic acid, rather than the *cis* substrates, *i.e.,* oleic acid and linolenic acid.

**EXAMPLE 6 - THE CHARACTERIZATION OF CAL-A VARIANTS**

Variants of CAL-A were cloned and functionally expressed in *E. coli* and *P. pastoris* cells to evaluate the variants for improved trans-selectivity over CAL-A, as described above.

*Hydrolysis and Ethanolyis Assay Using Partially Hydrogenated Soy Oil (PHSO)-* These procedures are the same as those described above.

*Determination of α-factors for trans fatty acid-selectivity in (PHSO) -* Competitive factors (α values) are used to describe the selectivity of an enzyme for different substrates, *e.g.*, selectivity for substrate A *vs*. substrate B. Fatty acid selectivity was determined as relative competitive factors (α-values), which are proportional to the specificity constants, $V_{max}/K_M$, for each fatty acid as described previously in Chen *et al.* (1982); Deleuze *et al.* (1987); Rangheard *et al.* (1989); and Borgdorf and Warwel (1999). Thus, a greater α-value indicates a greater reaction rate and selectivity for a particular fatty acid substrate. $C_{14}$ was used as the reference fatty acid substrate, because it was usually the fastest-reacting fatty acid, and was assigned an $\alpha$-value of 1.00.

The enzyme selectivity/specificity is a function of both substrates' binding and catalytic rate and, hence, is determined by the ratio of the specificity constants ($k_{cat}/K_M$). Therefore the competitive factor, $\alpha$, is defined as the ratio of different specificity constants. For example Rangheard *et al.* (1989) defined the $\alpha$-factor for the fatty acid selectivity of an enzyme as fellows:

$$\alpha = \frac{\frac{v_{AC1_X}}{K_{AC1_X}}}{\frac{v_{AC2_X}}{K_{AC2_X}}} = \frac{\log\left(\frac{AC1_X}{AC1_{X_0}}\right)}{\log\left(\frac{AC2_X}{AC2_{X_0}}\right)}$$

**(Eq. 2)**

From Equation 2, it is clear that the competitive factor is equal to the ratio of the reaction rates of two substrates when identical substrate concentrations ($ACI_X$ and $AC2_X$) are used. The definition of the E-value in kinetic resolution is very similar to Equation 2

$$E = \frac{\frac{v_A}{K_A}}{\frac{v_b}{K_B}} = \frac{\ln\left(\frac{A}{A_0}\right)}{\ln\left(\frac{B}{B_0}\right)}$$

**(Eq. 3)**

where $V_A$, and $V_B$, denote maximal velocities ($v_{max}$) of the fast- and slow-reacting enantiomers, respectively, and $K_A$ and $K_B$ denote Michaelis constants ($K_M$) of the fast- and slow-reacting enantiomers, respectively. Simpler methods to calculate selectivities may be thought of, such as using the initial slope of the graph or other initial velocity comparisons. However, these methods will not take competition of different substrates in the active site into account and therefore only describe apparent selectivity factors.

For the present embodiments, a modified $\alpha$-factor method was used that assumed that for the chemical equation, the bound trans-fatty acids ($trans_{bound}$) is in equilibrium with, or in some instances about equal to, the free trans-fatty acids ($trans_{free}$), for the time domain equation, the concentration of the initial $trans_{bound}$ ($[trans_{bound}]_0$) over time is in equilibrium with, or in some instances about equal to, the $trans_{bound}$ plus the $trans_{free}$, similarly the initial concentration of the bound non-trans-fatty acids ($[non\text{-}trans_{bound}]_0$ over time is in equilibrium with, or in some instances about equal to, the non-$trans_{bound}$ plus the non-$trans_{free}$, thus, the initial concentration of the trans-fatty acids is equal to the concentration of bound trans-fatty acids plus the concentration of free trans-fatty acids:

*chemical equation:* $trans_{bound} \rightleftharpoons trans_{free}$

*time domain equation:* $[trans_{bound}]_0 = trans_{bound} + trans_{free}$

$[non\text{-}trans_{bound}]_0 = non\text{-}trans_{bound} + non\text{-}trans_{free}$

*facts:* $[trans]_0 = [trans]_{bound} + [trans]_{free}$

Using these premises, Equation 1 can be re-written into Equation 4:

$$\alpha = \frac{\frac{v_{AC1_X}}{K_{AC1_X}}}{\frac{v_{AC2_X}}{K_{AC2_X}}} = \frac{\log\left(\frac{[trans]}{[trans]_0}\right)}{\log\left(\frac{[non-trans]}{[non-trans]_0}\right)}.$$

(Eq. 4)

The gas chromatography (GC) data provides relative percentages (% trans) rather than concentrations. Thus , the following presumptions were used:
*if:*

$$[trans]_0 \text{ is relative to } \%trans_0 \text{ or } [trans]_0 = X * \% trans_0$$

*then:*
The %trans from the GC (particularly from GC traces for the bound fraction) correlates into numbers that directly scale with actual concentration. For example, at 30% conversion, there will be a total of 30% free fraction. The percentages for *trans* and non-*trans* for the free fraction sums to 100%. For this testing, they must sum to 30%, which is accomplished by multiplying by c (conversion rate):

$$[trans]_{free} \text{ is relative to } \%trans_{free} * c \qquad \text{or} \qquad [trans]_{free} = X * \%trans_{free} * c$$

At 30% conversion, there will be a total of 70% bound fraction. The percentages for *trans* and non-*trans* for the bound fraction will sum to 100%. Therefore, it is needed to make them sum to 70%. So multiply by 1-c:

$$[trans]_{bound} \text{ is relative to } \%trans_{bound} * 1\text{-c} \qquad \text{or} \qquad [trans]_{bound} = X * \%trans_{bound} * 1\text{-c}$$

*so:*

$$\alpha = \frac{\log\left([trans]/[trans]_0\right)}{\log\left([non\text{-}trans]/[non\text{-}trans]_0\right)}$$

$$\alpha = \frac{\log\left(X * (\%trans_{bound} * 1\text{-c}) / X * (\%trans_0)\right)}{\log\left(X * (\%non\text{-}trans * 1\text{-c}) / X * (\%non\text{-}trans_0)\right)}$$

The X factors cancel, therefore:

$$\alpha = \frac{\log\left(\%trans_{bound} * 1\text{-c}\right) / \%trans_0}{\log\left(\%non\text{-}trans * 1\text{-c}\right) / \%non\text{-}trans_0}$$

(Eq. 5)

The X factors cancel because when a sample is submitted to the GC, a chromatogram that defines X for each fatty acid is provided (whether bound or free, they are now all FAME [fatty acid methyl esters]) in that sample, and only that sample. X will vary depending on the exact concentration of lipid in any given extract. For any given extraction, the percentage distribution of the peaks is identical whether 1 mg or 100 mg are submitted to the GC. Therefore, X can be cancelled.
When the calculated $\alpha$ factor becomes negative, it is indicating very high selectivities for the chosen comparison of substrates, *e.g.*, α (trans+sat)/cis< 0. To show this, the selectivity factor α by the method of Chen *et al.* (1982) was recalculated. While the Chen publication only takes into account values for the two different enantiomers, R and S, the behavior in pH soy is more complex. There multicompetitive reactions for the hydrolysis of different fatty acids take place. In order to simplify this further and make it applicable to the equation of Chen *et al.* (1982), we distinguish between three fractions: *cis* fatty acids, *trans* fatty acids and saturated fatty acids. Instead of using the content of the bound-fraction like in the modified Rangheard equation, the calculated released fatty fraction for calculating a modified eeP value was used (Equation 6):

$$eeP = [(\%trans + \%sat) - \%cis] / [(\%trans + \%sat) + \%sat] \qquad \textbf{(Eq. 6)}$$

This value (eeP) can now be used to determine a pseudo E-value for the complex fat mixture.

$$E = (v_1A/K_1A)/(v_1B/K_1B) = (\ln [(1-c(1 + eeP)])) / (\ln [(1-c(1-eeP)]) \qquad \textbf{(Eq. 7)}$$

This E-value equation yielded positive values, where the rational log function employed by Rangheard *et al.* (1989) and Borgdorf and Warwel (1999) will give negative values. Therefore, the negative numbers calculated by the modified Rangheard equation could be converted to high positive numbers using the modified equation from Chen *et al.* (1982) for calculating E-values. In kinetic resolution, E-values >100 indicate excellent selectivites but cannot be calculated precisely.

The data in Table 10 showed the hydrolysis/ethanolysis of partially hydrogenated plant oil (PH soy oil), as determined by the alpha factors (A and α) and compared to the performance of wild-type CAL-A (Ec_CalA_wt).

**TABLE 10**

| α-FACTORS FOR VARIANTS OF CAL-A | | | | |
|---|---|---|---|---|
| **Enzyme** | **Experiment** | **Multiplate** | **A Trans/(Cis+Sat)** | **α (Trans +Sat)(Cis)** |
| Background | Hydrolysis | 1 | No detection | |
| Background | Ethanolysis | 1 | 4 days <1% conv | |
| Geobacillus | Hydrolysis | 3 | 0.72 ± 0.09 | |
| Pp_CalA_wt | Hydrolysis | 3 | 3.50 ± 0.59 | 6.67 ± 2.85 |
| Pp_CalA_wt | Ethanolysis | 3 | 2.76 ± 0.05 | |
| Chirazyme L5 | Hydrolysis | 5 | 4.31 ± 0.59 | |
| Chirazyme L5 | Ethanolysis | 3 | 2.54 ± 0.04 | |
| Ec_CalA_wt | Hydrolysis | 3 | 3.62 ± 0.31 | 8.75 ±1.99 |
| Pp_CalA_L305N | Hydrolysis | 3 | 4.53 ± 1.21 | 9.56 ± 2.01 |
| Pp_CalA_L305N | Ethanolysis | 2 | 2.88 ± 0.03 | |
| Pp_CalA_L305F | Hydrolysis | 3 | 4.52 ± 0.25 | 8.74 ± 1.34 |
| Pp_CalA_L305F | Ethanolysis | 6 | 3.66 ± 0.10 | |
| Pp_CalA_A218N | Hydrolysis | 3 | 2.71 ± 0.73 | 3.88 ± 1.85 |
| Pp_CalA_A218N | Ethanolysis | 6 | 2.54 ± 0.11 | |
| Pp_CalA_F149D | Hydrolysis | 3 | 3.58 ± 1.09 | 12.53 ± 8.39 |
| Pp_CalA_F149D | Ethanolysis | 3 | 1 day <1% conv | |
| Ec_CalA_F149D | Hydrolysis | 3 | 4.64 ± 0.33 | 28.53 ± 10.54 |
| Ec_CalA_F222S | Hydrolysis | 3 | 4.46 ± 0.23 | 32.61 ± 15.09 |
| Ec_CalA_F222C | Hydrolysis | 3 | 4.6 ± 1.09 | 36.61 ± 35.75 |
| Ec_CalA_T221H | Hydrolysis | 3 | 9.77 ± 3.10 | >100 (outof range) |

(continued)

| α-FACTORS FOR VARIANTS OF CAL-A | | | | |
|---|---|---|---|---|
| **Enzyme** | **Experiment** | **Multiplate** | **A Trans/(Cis+Sat)** | **α (Trans +Sat)(Cis)** |
| Ec_CalA_I301H | Hydrolysis | 3 | 11.20 ± 0.99 | >100 (out of range) |
| Ec_CalA_I301H | Ethanolysis | 3 | 4 days <1% conv | |

As shown, in Table 10, the T221H and I301H lipase variants had the most improved *trans*-selectivity as compared to wild type CAL-A. Chirazyme L5 (Roche) is a commercial lyophilisate of CAL-A, which is used as a reference in these experiments. It would be expected that the most effective mutations to increase *trans*-selectivity would occur at the region of the *cis-/trans-* double bond in the fatty acid binding pocket. This, however, did not result in increased *trans*-selectivity and only resulted in the medium-chain FA selective variants. Surprisingly, it was found that the most successful (*trans*-selective) mutations occurred at different locations, especially at the terminal end of the fatty acid chain in the binding pocket. This could not be predicted by computer-modeling.

The data in Table 11 showed the hydrolysis and conversion percentages of partially hydrogenated soybean oil (PHSO) of various lipase variants. The PHSO contains around 20 different *trans-fatty* acids resembling ~18% of the total fatty acids. Thus, ToC-values determined with the *p*NP-esters may differ from the hydrolysis of this complex oil. Large-scale production of the six best CAL-A variants was performed using *Pichia pastoris* X33 or *E. coli* (DE3). The supernatant was then used in the hydrolysis of PHSO followed by gas chromatography analysis of the fatty acids that remained bound to glycerol and those that were hydrolysed. For evaluation of the performance of the CAL-A variants, the A and α factors were calculated. Thus, a high A factor reflects an enzyme with high preference for hydrolysis of *trans*-fatty acids.

**TABLE 11**

**CONVERSION AND HYDROLYSIS ACTIVITY USING DIFFERENT CAL-A VARIANTS**

| CAL-A variant | WT | L305N | F149D | F222S | F222C | T221H | I301H |
|---|---|---|---|---|---|---|---|
| Con. [%] | 22 | 14 | 11 | 16 | 20 | 5 | 12 |
| A[a] | 3.5 | 4.5 | 4.6 | 4.5 | 4.6 | 9.8 | 11.2 |
| α[b] | 6.7 | 9.6 | 28.5 | 32.2 | 36.6 | >100 | >100 |

[a] $\alpha_G$-factor for specificities $trans_{bound}/(cis_{bound}+sat_{bound})$; [b] $\alpha_G$-factor for specificities: $(trans_{bound}+sat_{bound})/cis_{bound}$; $trans_{bound}$: bound fraction of *trans* fatty acids; $cis_{bound}$: bound fraction of *cis* fatty acids; $sat_{bound}$: bound fraction of saturated fatty-acids in PHVO. All measurements were made in triplicate.

Table 11 shows that indeed all variants in the table were more selective than wild-type CAL-A with T221H and I301H being the most selective mutants. Furthermore, both variants gave an excellent α-factor > 100, meaning that *trans* and saturated fatty acids were preferentially hydrolyzed from partially hydrogenated vegetable oil. This provided an additional important health benefit to the resulting oil, as only the *cis*-fatty acids were enriched.

FIG. 17 shows the calculated percentage composition of *cis, trans* and saturated lipids in the released free fatty acid (FFA) fraction at 20% conversion for CAL-A and lipUMs variants. "Pp" indicates transformation of *P. pastoris* cells, "Ec" indicates transformation of *E. coli* cells, "CalA" indicates either wild-type (wt) CAL-A or a CAL-A variant, "UMs" indicates either the lipUMs sequence (wt) or a variant thereof. The composition of PH soy oil is shown as a comparison to initial starting conditions. As can be seen in Fig. 17, the percentage of *trans*-fatty acids for the CAL-A variants is comparable to that of wild-type CAL-A. The CAL-A variants and lipUMs variants can be used to modify the fatty acid profile of PH soy oil and especially to alter the *cis-* and *trans*-fatty acid content of a partially hydrogenated oil. The *trans*-fatty acids can be fully removed by the CAL-A mutants I301H, F149D and T221H, as indicated by the accumulation of *trans*-fatty acids in the released FFA fraction. The CAL-A mutant F222S, which was the best in the initial screen, also showed excellent selectivity for the removal of *trans*-fatty acid, as they accumulated in the released FFA fraction.

**REFERENCES**

Altschul, S.F., Gish, W., Miller, W., Myers, E.W., and Lipman, D.J. "Basic local alignment search tool," J. Mol. Biol., 215:403-410 (1990).

Anthonsen, H.W., Baptista, A., Drablos, F., Martel, P., Petersen, S.B., Sebastiao, M., and Vaz, L. "Lipases and esterases: a review of their sequences, structure and evolution," Biotechnol. Annu. Rev., 1:315-371 (1995).

Aparna, G., Chatterjee, A., Sonti, R.V., and Sankaranarayanan, R. "A cell wall-degrading esterase of Xanthomonas oryzae requires a unique substrate recognition module for pathogenesis on rice," Plant Cell, 21:1860-1873 (2009).

Badings, H. T., and De Jong, C., "Glass capillary gas chromatography of fatty acid methyl esters. A study of conditions for the quantitative analysis of short- and long-chain fatty acids in lipids," J. Chromatog., 279:493-506 (1983).

Beer, H.D., McCarthy, J.E., Bornscheuer, U.T., and Schmid, R.D. "Cloning, expression, characterization and role of the leader sequence of a lipase from Rhizopus oryzae. Biochim. Biophys. Acta, 1399:173-180 (1998).

Beer, H.D., Wohlfahrt, G., Schmid, R.D., and McCarthy, J.E. "The folding and activity of the extracellular lipase of Rhizopus oryzae are modulated by a prosequence," Biochem. J. 319(Pt 2):351-359 (1996).

Bendtsen, J.D., Nielsen, H., von Heijne, G., and Brunak, S. "Improved prediction of signal peptides: SignalP 3.0," J. Mol. Biol., 340:783-795 (2004).

Berman, H.M., Westbrook, J.D., Gabanyi, M.J., Tao, W., Shah, R., Kouranov, A., Schwede, T., Arnold, K., Kiefer, F., Bordoli, L., et al., "The protein structure initiative structural genomics knowledgebase," Nucl. Acids Res., 37:D365-368 (2009).

Borgdorf, R., and Warwel, S. "Substrate selectivity of various lipases in the esterification of cis- and trans-9-octadecenoic acid," Appl. Microbiol. Biotechnol., 51:480-485 (1999).

Bornscheuer, U.T., and Kazlauskas, R.J. "Hydrolases in Organic Synthesis - Regio- and Stereoselective Biotransformations, 2nd ed. Wiley-VCH, Weinheim (2005).

Brunke, S., and Hube, B. "MfLIP1, a gene encoding an extracellular lipase of the lipid-dependent fungus Malassezia furfur," Microbiology, 152:547-554 (2006).

de Maria, P.D., Carboni-Oerlemans, C., Tuin, B., Bargeman, G., van der Meer, A., and van Gemert, R. "Biotechnological applications of Candida antarctica lipase A: State-of-the-art,". J. Mol. Catal. B-Enzymat., 37:36-46 (2005).

Dumon-Seignovert, L., Cariot, G., and Vuillard, L. "The toxicity of recombinant proteins in Escherichia coli: a comparison of overexpression in BL21(DE3), C41(DE3), and C43(DE3)," Protein Expr. Purif., 37:203-206 (2004).

Emanuelsson, O., Nielsen, H., Brunak, S., and von Heijne, G. "Predicting subcellular localization of proteins based on their N-terminal amino acid sequence," J. Mol. Biol., 300:1005-1016 (2000).

Ericsson, D.J., Kasrayan, A., Johanssonl, P., Bergfors, T., Sandstrom, A.G., Backvall, J.E., and Mowbray, S.L. "X-ray structure of Candida antarctica lipase a shows A novel lid structure and a likely mode of interfacial activation," J. Mol. Biol., 376:109-119 (2008).

Hanahan, D. "Studies on transformation of Escherichia coli with plasmids," J. Mol. Biol., 166:557-580 (1983).

Hegde, R.S., and Bernstein, H.D. "The surprising complexity of signal sequences," Trends Biochem. Sci., 31:563-571 (2006).

Hoegh, I.S.P., T. Halkier, M.T. Hansen. "Two lipases from Candida antarctica: Cloning and expression in Aspergillus oryzae," Can. J. Bot., 73(S1):S869-S875 (1995).

Holm, L., and Sander, C. "Protein structure comparison by alignment of distance matrices," J. Mol. Biol., 233:123-138 (1993).

Holmquist, M. "Alpha/Beta-hydrolase fold enzymes: structures, functions and mechanisms," Curr. Protein Pept. Sci., 1:209-235 (2000).

Kakugawa, K., Shobayashi, M., Suzuki, O., and Miyakawa, T. "Cloning, characterization, and expression of cDNA encoding a lipase from Kurtzmanomyces sp. I-11," Biosci. Biotechnol. Biochem., 66:1328-1336 (2002a).

Kakugawa, K., Shobayashi, M., Suzuki, O., and Miyakawa, T. "Purification and characterization of a lipase from the glycolipid-producing yeast Kurtzmanomyces sp. I-11," Biosci. Biotechnol. Biochem., 66:978-985 (2002b).

Kasrayan, A., Bocola, M., Sandstrom, A.G., Laven, G., and Backvall, J.E. "Prediction of the Candida antarctica lipase A protein structure by comparative modeling and site-directed mutagenesis," Chembiochem, 8:1409-1415 (2007).

Kirk, O., and Christensen, M.W. " Lipases from Candida antarctica: Unique biocatalysts from a unique origin," Organ. Process Res. Develop., 6:446-451 (2002).

Kourist, R., Brundiek, H., and Bournscheuer, U.T. "Protein engineering and Discovery of lipases," Eur. J. Lipid Sci. Technol., 112:64-74 (2010).

Krishna, S.H., Persson, M., and Bornscheuer, U.T. "Enantioselective transesteri cation of a tertiary alcohol by lipase A from," Tetrahedron, 13:2693-2696 (2002).

Krogh, A., Larsson, B., von Heijne, G., and Sonnhammer, E.L. "Predicting transmembrane protein topology with a hidden Markov model: application to complete genomes," J. Mol. Biol., 305:567-580 (2001).

Laemmli, U.K. "Cleavage of structural proteins during the assembly of the head of bacteriophage T4," Nature, 227:680-685 (1970).

Li, X.G., and Kanerva, L.T. "Lipases in beta-dipeptide synthesis in organic solvents," Org. Lett., 8(24):5593-5596 (2006).

Miroux, B., and Walker, J.E. "Over-production of proteins in Escherichia coli: mutant hosts that allow synthesis of some membrane proteins and globular proteins at high levels," J. Mol. Biol., 260:289-298 (1996).

Ollis, D.L., Cheah, E., Cygler, M., Dijkstra, B., Frolow, F., Franken, S.M., Harel, M., Remington, S.J., Silman, I., and Schrag, J. "The α/β hydrolase fold," Protein Eng., 5:197-211 (1992).

Pavelka, A., Chovancova, E., and Damborsky, J. "HotSpot Wizard: a web server for identification of hot spots in protein engineering," Nucl. Acids Res., 37:W376-383 (2009).

Pfeffer, J., Richter, S., Nieveler, J., Rachid, C.-e.H., Rhlid, B., Schmid, R.D., and Rusnak, M. "High yield expression of Lipase A from Candida antarctica in the methylotrophic yeast Pichia pastoris and its purification and characterisation," Appl. Microbiol.,: 72(5):931-938 (2006).

Pfeffer, J., Rusnak, M., Hansen, C.E., Rhlid, R.B., Schmid, R.D., and Maurer, S.C. "Functional expression of lipase A from Candida antarctica in Escherichia coli - A prerequisite for high-throughput screening and directed evolution," J. Mol. Catal. B-Enzym., 45:62-67 (2007).

Rangheard, M.S., Langrand, G., Triantaphylides, C., and Baratti, J.." Multi-competitive enzymatic reactions in organic media: a simple test for the determination of lipase fatty acid specificity," Biochim. Biophys. Acta, 1004:20-28 (1989).

Reetz, M.T., Kahakeaw, D., and Lohmer, R. "Addressing the numbers problem in directed evolution," Chembiochem, 9:1797-1804 (2008).

Schmidt, M., Barbayianni, E., Fotakopoulou, I., Ho, M., Constantinou-kokotou, V., Bornscheuer, U.T., and Kokotos, G. "Enzymatic removal of carboxyl protecting groups. 1. Cleavage of the tert-butyl moiety," J. Org. Chem., 70(9):3737-3740 (2005).

Sezonov, G., Joseleau-Petit, D., and D'Ari, R. "Escherichia coli physiology in Luria-Bertani broth," J. Bacteriol., 189:8746-8749 (2007).

Soumanou, M. M., Bornscheuer, U. T., Menge, U, and Schmid R. D., "Synthesis of structured triglycerides from peanut oil with immobilized lipase," J. Amer. Oil Chem. Soc., 74:427-433 (1997).

Soumanou, M. M., Edorh, A. P., and Bornscheuer, U. T. "Activity and stability of immobilized lipases in lipase-catalyzed modification of peanut oil," Oleagineux, Corps Gras, Lipides, 11:6 464-468 (2004).

von Heijne, G. "Signal sequences. The limits of variation," J. Mol. Biol., 184:99-105 (1985).

Wagner, S., Klepsch, M.M., Schlegel, S., Appel, A., Draheim, R., Tarry, M., Hogbom, M., van Wijk, K.J., Slotboom, D.J., Persson, J.O., et al., "Tuning Escherichia coli for membrane protein overexpression. Proc. Natl. Acad. Sci. USA, 105:14371-14376 (2008).

Warwel, S., and Borgdorf, R. "Substrate selectivity of lipases in the esterification of cis/trans-isomers and positional isomers of conjugated linoleic acid (CLA)," Biotechnol. Lett., 22:1151-1155 (2000).

Warwel, S., Borgdorf, R., and Bruhl, L. "Substrate selectivity of lipases in the esterification of oleic acid, linoleic acid, linolenic acid and their all-trans-isomers and in the transesterification of cis/trans-isomers of linoleic acid methyl ester," Biotechnol. Lett., 21: 431-436 (1999).

Winkler, U.K., and Stuckmann, M. "Glycogen, hyaluronate, and some other polysaccharides greatly enhance the formation of exolipase by Serratia marcescens," J. Bacteriol., 138:663-670 (1979).

All of the compositions and methods disclosed and claimed herein can be made and executed without undue experimentation in light of the present disclosure. While the compositions and methods of this invention have been described in terms of exemplary embodiments, it will be apparent to those of skill in the art that variations may be applied to the composition, methods and in the steps or in the sequence of steps of the method described herein without departing from the scope of the invention. More specifically, it will be apparent that certain agents that are both chemically- and physiologically-related may be substituted for the agents described herein while the same or similar results would be achieved. All such similar substitutes and modifications apparent to those of ordinary skill in the art are deemed to be within the scope of the invention as defined by the appended claims. Accordingly, the exclusive rights sought to be patented are as described in the claims below.

This invention may be combined in many different forms and should not be construed as limited to the embodiments set forth herein; rather theses embodiments are provided so that this disclosure will fully convey the invention to those skilled in the art. Many modifications and other embodiments will come to mind in one skilled in the art to which this invention pertains having the benefit of the teachings presented in the foregoing description. Although specific terms are employed, they are used as in the art unless otherwise indicated.

## Claims

1. A lipase variant comprising an amino acid sequence of a starting microbial lipase that has been substituted in at least one amino acid at a position that corresponds to amino acid residue 170, 171, 239, 242, 243, 254, 258, 262, 269, 322, or 326, of SEQ ID NO:11, wherein the substitution confers to the lipase variant a) an increase in hydrolytic or ethanolytic activity in the presence of at least a first trans-fatty acid moiety, when compared to that of the unsubstituted starting microbial lipase; b) an increased preference for catalyzing fatty acid moieties having a carbon chain length greater than or equal to six ($\geq C_6$), when compared to that of the un-substituted starting microbial lipase; or c) a combination thereof; wherein the starting microbial lipase is obtained from *Candida antarctica, Ustilago maydis, Kurtzmanomyces species 1-11, or Sporisorium relianum;* and wherein, when the starting microbial lipase has been substituted in at least one amino acid at a position that corresponds to amino acid residue 242, 254, 258, 170 or 171, the at least one amino acid substitution is: a phenylalanine-to-threonine substitution at amino acid residue 170 (F170T); a phenylalanine-to-asparagine substitution at amino acid residue 170 (F170N); a phenylalanine-to-valine substitution at amino acid residue 170 (F170V); a threonine-to-isoleucine substitution at amino acid residue 242 (T242I), a threonine-to-histidine substitution at amino acid residue 242 (T242H); a phenylalanine-to-arginine substitution at amino acid residue 254 (F254R); a glycine-to-alanine substitution at amino acid residue 258 (G258A); a glycine-to-valine substitution at amino acid residue 258 (G258V); a glycine-to-arginine substitution at amino acid residue 258 (G258R); a glycine-to-tryptophan substitution at amino acid residue 258 (G258W); a glycine-to-tyrosine substitution at amino acid residue 258 (G258Y); an isoleucine-to-lysine substitution at amino acid residue 171 (I171K); an isoleucine-to-threonine substitution at amino acid residue 171 (I171T); an isoleucine-to-glutamic acid substitution at amino acid residue 171 (I171E); and/or an isoleucine-to-valine substitution at amino acid residue 171 (I171V).

2. The lipase variant of claim 1, wherein the substitution of at least one amino acid confers to the variant an about 5-fold increase or an about 10-fold increase in hydrolytic or ethanolytic activity in the presence of an at least a first *trans*-fatty acid moiety, when compared to that of the un-substituted starting microbial lipase.

3. The lipase variant of any preceding claim, wherein the one or more amino acid substitutions is a phenylalanine-to-serine substitution, a phenylalanine-to-cysteine substitution, a phenylalanine-to-aspartic acid substitution, a phenylalanine-to-glycine substitution, a phenylalanine-to-methionine substitution, a phenylalanine-to-threonine substitution, a phenylalanine-to-asparagine substitution, a phenylalanine-to-valine substitution, an isoleucine-to-histidine substitution, an isoleucine-to-arginine substitution, an isoleucine-to-alanine substitution, a threonine-to-isoleucine substitution, a threonine-to-histidine substitution, a leucine-to-phenylalanine substitution, a leucine-to-asparagine substitution, a leucine-to-tyrosine substitution, a leucine-to-cysteine substitution, a leucine-to-glutamine substitution, a phenylalanine-to-arginine substitution, a leucine-to-arginine substitution, an alanine-to-asparagine substitution, a glycine-to-alanine substitution, a glycine-to-leucine substitution, a glycine-to-valine substitution, a glycine-to-tyrosine substitution, a glycine-to-arginine substitution, a glycine-to-tryptophan substitution, an isoleucine-to-lysine substitution, an isoleucine-to-threonine substitution, an isoleucine-to-glutamic acid substitution, an isoleucine-to-valine substitution, a leucine-to-alanine substitution, a methionine-to-valine substitution, or a leucine-to-alanine substitution, or any combination thereof;
optionally wherein the one or more amino acid substitutions is phenylalanine-to-serine substitution at amino acid residue 243 (F243S), a phenylalanine-to-cysteine substitution at amino acid residue 243 (F243C), a phenylalanine-to-aspartic acid substitution at amino acid residue 170 (F170D), a phenylalanine-to-glycine substitution at amino acid residue 170 (F170G), a phenylalanine-to-methionine substitution at amino acid residue 170 (F170M), an isoleucine-to-histidine substitution at amino acid residue 322 (I322H) or at amino acid residue 171 (I171H), an isoleucine-to-arginine substitution at amino acid residue 322 (I322R) or at amino acid residue 171 (I171R), an isoleucine-to-alanine substitution at amino acid residue 322 (I322A), a leucine-to-phenylalanine substitution at amino acid residue 326 (L326F), a leucine-to-asparagine substitution at amino acid residue 326 (L326N) or at amino acid residue 262 (L262N), a leucine-to-tyrosine substitution at amino acid residue 326 (L326Y), a leucine-to-cysteine substitution at amino acid residue 326 (L326C), a leucine-to-glutamine substitution at amino acid residue 326 (L326Q), an alanine-to-asparagine substitution at amino acid residue 149 (A149N), a leucine-to-alanine substitution at amino acid residue 262 (L262A), a methionine-to-valine substitution at amino acid residue 269 (M269V), or a leucine-to-alanine substitution at amino acid residue 326 (L326A), or any combination thereof.

4. The lipase variant of any preceding claim, that has been substituted in at least two amino acids corresponding to two or more of amino acid resides 170, 171, 239, 242, 243, 254, 258, 262, 269, 322, or 326, of SEQ ID NO:11.

5. The lipase variant of any preceding claim, formulated with one or more stabilizers, one or more detergents, one or more enzymes, one or more surfactants, one or more buffers, one or more enzyme substrates, or any combination thereof.

6. The lipase variant of any preceding claim, formulated with one or more enzyme substrates that comprised a lipid, a fatty acid, a sterol, a wax, an oil, a triglyceride, an ester, a carboxylic acid moiety, or any combination thereof.

7.  The lipase variant of any preceding claim, substantially bound or substantially chemically cross-linked to a matrix, a column, a fiber, a filter, a resin, a gel, a bead, or any combination thereof.

8.  The lipase variant of any preceding claim, formulated with one or more additional enzymes selected from the group consisting of one or more lipases, one or more esterases, one or more lyases, one or more deproteinases, one or more phosphatases, one or more dehydrogenases, one or more transglutaminases, one or more oxidases, or any combination thereof.

9.  An isolated polynucleotide encoding the lipase variant of any one of claims 1 to 8.

10.  An expression vector comprising an isolated polynucleotide that encodes the lipase variant of any one of claims 1 to 8, optionally wherein the expression vector is codon-optimized for expression in a bacterial or non-basidiomycetous yeast cell.

11.  A microbial host cell transformed with the isolated polynucleotide of claim 9, or the expression vector of claim 10; optionally wherein the microbial host cell is defined as an *E. coli* or a *P. pastoris* host cell.

12.  A method for producing a lipase variant, comprising (a) culturing a population of microbial host cells that encodes the lipase variant, of any one of claims 1 to 8, under conditions conducive to the expression and secretion of the lipase variant, and (b) recovering the expressed lipase variant from the population of host cells or the culture medium.

13.  A method of reducing or eliminating one or more *trans*-unsaturated fatty acid compounds or one or more medium- or long-chain ($\geq C_6$) fatty acid moieties from a substrate, comprising contacting the substrate with an effective amount of a lipase variant in accordance with any one of claims 1 to 8, for a time sufficient to hydrolyze or esterify at least a portion of the substrate thereby reducing or eliminating the one or more *trans*-unsaturated fatty acid compounds or the one or more medium- or long-chain ($\geq C_6$) fatty acid moieties from the substrate;
optionally further comprising removing the lipase variant from the composition after the one or more *trans*-unsaturated fatty acid compounds or the one or more medium- or long-chain ($\geq C_6$) fatty acid moieties has been substantially reduced or eliminated from the substrate.

14.  The method of claim 13, wherein the substrate comprises an edible lipid, an edible fat, an edible fatty acid, an edible sterol, an edible wax, an edible oil, or an edible triglyceride, or any combination thereof.

15.  A lipase variant comprising an amino acid sequence of a starting microbial lipase that has been substituted in at least one amino acid of SEQ ID NO:11, wherein the substitution is: F243S, F243C, F170D, F170G, F170M, F170T, F170N, F170V, I322H, I322R, I322A, T242I, T242H, L326F, L326N, L326Y, L326C, L326Q, F254R, L246R, A239N, G258A, G258V, G258Y, G258R, G258W, I171K, I171H, I171R, I171T, I171E, I171V, L262A, L262N, M269V and L326A, or a combination thereof where the substitutions do not overlap with one another.

**Patentansprüche**

1.  Lipasevariante, die eine Aminosäuresequenz einer mikrobiellen Anfangslipase umfasst, die in wenigstens einer Aminosäure an einer Position substituiert ist, die dem Aminosäurerest 170, 171, 239, 242, 243, 254, 258, 262, 269, 322 oder 326 von SEQ ID Nr. 11 entspricht, wobei die Substitution der Lipasevariante Folgendes verleiht: a) eine Zunahme der hydrolytischen oder ethanolytischen Aktivität in Anwesenheit von wenigstens einem ersten *trans*-Fettsäureanteil im Vergleich zu dem der unsubstituierten mikrobiellen Anfangslipase; b) eine erhöhte Präferenz zum Katalysieren von Fettsäureanteilen mit einer Kohlenstoffkettenlänge von gleich oder größer als sechs ($\geq C_6$) im Vergleich zu der der unsubstituierten mikrobiellen Anfangslipase; oder c) eine Kombination davon; wobei die mikrobielle Anfangslipase erhalten wird von *Candida antarctica, Ustilago maydis, Kurtzmanomyces Spezies 1-11 oder Sporisorium relianum;* und wobei, wenn die mikrobielle Anfangslipase in wenigstens einer Aminosäure an einer Position substituiert wurde, die dem Aminosäurerest 242, 254, 258, 170 oder 171 entspricht, die wenigstens eine Aminosäuresubstituition wie folgt lautet: eine Phenylalanin-zu-Threonin-Substition an Aminosäurerest 170 (F170T); eine Phenylalanin-zu-Asparagin-Substitution an Aminosäurerest 170 (F170N); eine Phenylalanin-zu-Valin-Substitution an Aminosäurerest 170 (F170V); eine Threonin-zu-Isoleucin-Substitution an Aminosäurerest 242 (T242I), eine Threonin-zu-Histidin-Substitution an Aminosäurerest 242 (T242H); eine Phenylalanin-zu-Arginin-Substitution an Aminosäurerest 254 (F254R); eine Glycin-zu-Alanin-Substitution an Aminosäurerest 258 (G258A); eine Glycin-zu-Valin-Substitution an Aminosäurerest 258 (G258V); eine Glycin-zu-Arginin-Substitution an Aminosäurerest 258 (G258R); eine Glycin-zu-Tryptophan-Substitution an Aminosäurerest 258 (G258W); eine Glycin-zu-Tyrosin-Substitution an Aminosäurerest 258 (G258Y); eine Isoleucin-zu-Lysin-Substitution an Aminosäurerest 171 (I171K); eine Isoleucin-zu-Threonin-Substitution an Aminosäurerest 171 (I171T); eine Isoleucin-zu-Glutaminsäure-Substitution an Aminosäurerest 171 (I171E); und/oder eine Isoleucin-zu-Valin-Substitution an Aminosäurerest 171 (I171V).

2. Lipasevariante nach Anspruch 1, wobei die Substitution von wenigstens einer Aminosäure der Variante eine etwa 5fache Zunahme oder eine etwa 10fache Zunahme an hydrolytischer oder ethanolytischer Aktivität in Anwesenheit von wenigstens einem ersten *trans*-Fettsäureanteil im Vergleich zu der der unsubstituierten mikrobiellen Anfangslipase verleiht.

3. Lipasevariante nach einem vorherigen Anspruch, wobei die ein oder mehreren Aminosäuresubstitutionen Folgende sind: eine Phenylalanin-zu-Serin-Substitution, eine Phenylalanin-zu-Cystein-Substitution, eine Phenylalanin-zu-Asparaginsäure-Substitution, eine Phenylalanin-zu-Glycin-Substitution, eine Phenylalanin-zu-Methionin-Substitution, eine Phenylalanin-zu-Threonin-Substitution, eine Phenylalanin-zu-Asparagin-Substitution, eine Phenylalanin-zu-Valin-Substitution, eine Isoleucin-zu-Histidin-Substitution, eine Isoleucin-zu-Arginin-Substitution, eine Isoleucin-zu-Alanin-Substitution, eine Threonin-zu-Isoleucin-Substitution, eine Threonin-zu-Histidin-Substitution, eine Leucin-zu-Phenylalanin-Substitution, eine Leucin-zu-Asparagin-Substitution, eine Leucin-zu-Tyrosin-Substitution, eine Leucin-zu-Cystein-Substitution, eine Leucin-zu-Glutamin-Substitution, eine Phenylalanin-zu-Arginin-Substitution, eine Leucin-zu-Arginin-Substitution, eine Alanin-zu-Asparagin-Substitution, eine Glycin-zu-Alanin-Substitution, eine Glycin-zu-Leucin-Substitution, eine Glycin-zu-Valin-Substitution, eine Glycin-zu-Tyrosin-Substitution, eine Glycin-zu-Arginin-Substitution, eine Glycin-zu-Tryptophan-Substitution, eine Isoleucin-zu-Lysin-Substitution, eine Isoleucin-zu-Threonin-Substitution, eine Isoleucin-zu-Glutaminsäure-Substitution, eine Isoleucin-zu-Valin-Substitution, eine Leucin-zu-Alanin-Substitution, eine Methionin-zu-Valin-Substitution oder eine Leucin-zu-Alanin-Substitution oder eine beliebige Kombination davon;

   wobei die ein oder mehreren Aminosäuresubstitutionen optional wie folgt sind: eine Phenylalanin-zu-Serin-Substitution an Aminosäurerest 243 (F243S), eine Phenylalanin-zu-Cystein-Substitution an Aminosäurerest 243 (F243C), eine Phenylalanin-zu-Asparaginsäure-Substitution an Aminosäurerest 170 (F170D), eine Phenylalanin-zu-Glycin-Substition an Aminosäurerest 170 (F170G), eine Phenylalanin-zu-Methionin-Substitution an Aminosäurerest 170 (F170M), eine Isoleucin-zu-Histidin-Substitution an Aminosäurerest 322 (I322H) oder an Aminosäurerest 171 (I171H), eine Isoleucin-zu-Arginin-Substitution an Aminosäurerest 322 (I322R) oder an Aminosäurerest 171 (I171R), eine Isoleucin-zu-Alanin-Substitution an Aminosäurerest 322 (I322A), eine Leucin-zu-Phenylalanin-Substitution an Aminosäurerest 326 (L326F), eine Leucin-zu-Asparagin-Substitution an Aminosäurerest 326 (L326N) oder an Aminosäurerest 262 (L262N), eine Leucin-zu-Tyrosin-Substitution an Aminosäurerest 326 (L326Y), eine Leucin-zu-Cystein-Substitution an Aminosäurerest 326 (L326C), eine Leucin-zu-Glutamin-Substitution an Aminosäurerest 326 (L326Q), eine Alanin-zu-Asparagin-Substitution an Aminosäurerest 149 (A149N), eine Leucin-zu-Alanin-Substitution an Aminosäurerest 262 (L262A), eine Methionin-zu-Valin-Substitution an Aminosäurerest 269 (M269V) oder eine Leucin-zu-Alanin-Substitution an Aminosäurerest 326 (L326A) oder eine beliebige Kombination davon.

4. Lipasevariante nach einem vorherigen Anspruch, die in wenigstens zwei Aminosäuren entsprechend zwei oder mehr von Aminosäureresten 170, 171, 239, 242, 243, 254, 258, 262, 269, 322 oder 326 von SEQ ID Nr. 11 substituiert wurde.

5. Lipasevariante nach einem vorherigen Anspruch, formuliert mit einem oder mehreren Stabilisatoren, einem oder mehreren Detergenzien, einem oder mehreren Enzymen, einem oder mehreren Tensiden, einem oder mehreren Puffern, einem oder mehreren Enzymsubstraten oder einer beliebigen Kombination davon.

6. Lipasevariante nach einem vorherigen Anspruch, formuliert mit einem oder mehreren Enzymsubstraten bestehend aus einem Lipid, einer Fettsäure, einem Sterol, einem Wachs, einem Öl, einem Triglycerid, einem Ester, einem Carbonsäureanteil oder einer beliebigen Kombination davon.

7. Lipasevariante nach einem vorherigen Anspruch, im Wesentlichen gebunden oder im Wesentlichen chemisch vernetzt mit einer/einem Matrix, Säule, Faser, Filter, Harz, Gel, Korn oder einer beliebigen Kombination davon.

8. Lipasevariante nach einem vorherigen Anspruch, formuliert mit einem oder mehreren zusätzlichen Enzymen, ausgewählt aus der Gruppe bestehend aus einer oder mehreren Lipasen, einer oder mehreren Esterasen, einer oder mehreren Lyasen, einer oder mehreren Deproteinasen, einer oder mehreren Phosphatasen, einer oder mehreren Dehydrogenasen, einer oder mehreren Transglutaminasen, einer oder mehreren Oxidasen oder einer beliebigen Kombination davon.

9. Isoliertes Polynukleotid, das die Lipasevariante nach einem der Ansprüche 1 bis 8 kodiert.

10. Expressionsvektor, der ein isoliertes Polynukleotid umfasst, das die Lipasevariante nach einem der Ansprüche 1 bis 8 kodiert,

   wobei der Expressionsvektor optional zur Expression in einer Bakterien- oder Non-Basidiomyceten-Hefezelle kodonoptimiert ist.

11. Mikrobielle Wirtszelle, transformiert mit dem isolierten Polynukleotid nach Anspruch 9 oder dem Expressionsvektor von Anspruch 10;

   wobei die mikrobielle Wirtszelle optional als *E. coli* oder als *P. pastoris* Wirtszelle definiert ist.

12. Verfahren zum Erzeugen einer Lipasevariante, das Folgendes beinhaltet: (a) Kultivieren einer Population von mikrobiellen Wirtszellen, die die Lipasevariante kodiert, nach einem der Ansprüche 1 bis 8, unter Bedingungen, die zur Expression und Sekretion der Lipasevariante führen, und (b) Zurückgewinnen der exprimierten Lipasevariante von der Population von Wirtszellen oder dem Kulturmedium.

13. Verfahren zum Reduzieren oder Eliminieren von einer oder mehreren trans-ungesättigten Fettsäureverbindungen oder einem oder mehreren mittel- oder langkettigen ($\geq C_6$) Fettsäureanteilen von einem Substrat, das das Kontaktieren des Substrats mit einer wirksamen Menge einer Lipasevariante nach einem der Ansprüche 1 bis 8 für eine Zeit beinhaltet, die ausreicht, um wenigstens einen Teil des Substrats zu hydrolysieren oder zu verestern, um dadurch die ein oder mehreren trans-ungesättigten Fettsäureverbindungen oder die ein oder mehreren mittel- oder langkettigen ($\geq C_6$) Fettsäureanteile von dem Substrat zu reduzieren oder zu eliminieren;

   das ferner optional das Entfernen der Lipasevariante aus der Zusammensetzung beinhaltet, nachdem die ein oder mehreren *trans*-ungesättigten Fettsäureverbindungen oder die ein oder mehreren mittel- oder langkettigen ($\geq C_6$) Fettsäureanteile aus dem Substrat im Wesentlichen reduziert oder eliminiert wurden.

14. Verfahren nach Anspruch 13, wobei das Substrat ein essbares Lipid, ein essbares Fett, eine essbare Fettsäure, ein essbares Sterol, ein essbares Wachs, ein essbares Öl oder ein essbares Triglycerid oder eine beliebige Kombination davon umfasst.

15. Lipasevariante, die eine Aminosäuresequenz einer mikrobiellen Anfangslipase umfasst, die in wenigstens einer Aminosäure von SEQ ID Nr. 11 substituiert wurde, wobei die Substitution wie folgt ist: F243S, F243C, F170D, F170G, F170M, F170T, F170N, F170V, I322H, I322R, I322A, T242I, T242H, L326F, L326N, L326Y, L326C, L326Q, F254R, L246R, A239N, G258A, G258V, G258Y, G258R, G258W, I171K, I171H, I171R, I171T, I171E, I171V, L262A, L262N, M269V und L326A, oder eine Kombination davon, wobei die Substitionen einander nicht überlappen.

## Revendications

1. Une variante de lipase comprenant une séquence d'acides aminés d'une lipase microbienne de départ qui a été substituée dans au moins un acide aminé à une position qui correspond au résidu d'acide aminé 170, 171, 239, 242, 243, 254, 258, 262, 269, 322 ou 326 de SEQ ID NO : 11, dans laquelle la substitution confère à la variante de lipase a) une augmentation de l'activité hydrolytique ou éthanolytique en présence d'au moins une première fraction d'acide gras trans, lorsqu'elle est comparée à celle de la lipase microbienne de départ non substituée ; b) une préférence accrue pour la catalyse des fractions d'acide gras ayant une longueur de chaîne de carbone égale ou supérieure à six ($\geq$C6), lorsqu'elle est comparée à celle de la lipase microbienne de départ non substituée ; ou c) une combinaison de celles-ci ; dans laquelle la lipase microbienne de départ est obtenue de *Candida antarctica*, *Ustilago maydis*, *Kurtzmanomyces species 1-11* ou *Sporisorium relianum* ; et dans laquelle, lorsque la lipase microbienne de départ a été substituée dans au moins un acide aminé à une position qui correspond au résidu d'acide aminé 242, 254, 258, 170, 171, la au moins une substitution d'un acide aminé est : une substitution de la phénylalanine par la thréonine au niveau du résidu d'acide aminé 170 (F170T) ; une substitution de la phénylalanine par l'asparagine au niveau du résidu d'acide aminé 170 (F170N) ; une substitution de la phénylalanine par la valine au niveau du résidu d'acide aminé 170 (F170V) ; une substitution de la thréonine par l'isoleucine au niveau du résidu d'acide aminé 242 (T42I), une substitution de la thréonine par l'histidine au niveau du résidu d'acide aminé 242 (T242H) ; une substitution de la phénylalanine par l'arginine au niveau du résidu d'acide aminé 254 (F254R) ; une substitution de la glycine par l'alanine au niveau du résidu d'acide aminé 258 (G258A) ; une substitution de la glycine par la valine au niveau du résidu d'acide aminé 258 (G258V) ; une substitution de la glycine par l'arginine au niveau du résidu d'acide aminé 258 (G258R) ; une substitution de la glycine par le tryptophane au niveau du résidu d'acide aminé 258 (G258W) ; une substitution de la glycine par la tyrosine au niveau du résidu d'acide aminé 258 (G258Y) ; une substitution de l'isoleucine par la lysine au niveau du résidu d'acide aminé 171 (I171K), une substitution de l'isoleucine par la thréonine au niveau du résidu d'acide aminé 171 (I171T) ; une substitution de l'isoleucine par l'acide glutamique au niveau du résidu d'acide aminé 171 (I171E) ; et/ou une substitution de l'isoleucine par la valine au niveau du résidu d'acide aminé 171 (I171V).

2. La variante de lipase selon la revendication 1, dans laquelle la substitution d'au moins un acide aminé confère à la variante une augmentation d'environ 5 fois ou une augmentation d'environ 10 fois de l'activité hydrolytique ou éthanolytique en présence d'au moins une première fraction d'acide gras *trans,* lorsqu'elle est comparée à celle de la lipase microbienne de départ non substituée.

3. La variante de lipase selon l'une quelconque des revendications précédentes, dans laquelle la ou les substitutions d'acides aminés est une substitution de la phénylalanine par la serine, une substitution de la phénylalanine par la cystéine, une substitution de la phénylalanine par l'acide aspartique, une substitution de la phénylalanine par la glycine, une substitution de la phénylalanine par la méthionine, une substitution de la phénylalanine par la thréonine, une substitution de la phénylalanine par l'asparagine, une substitution de la phénylalanine par la valine, une substitution de l'isoleucine par l'histidine, une substitution de l'isoleucine par l'arginine, une substitution de l'isoleucine par l'alanine, une substitution de la thréonine par l'isoleucine, une substitution de la thréonine par l'histidine, une substitution de la leucine par la phénylalanine, une substitution de la leucine par l'asparagine, une substitution de la leucine par la tyrosine, une substitution de la leucine par la cystéine, une substitution de la leucine par la glutamine, une substitution de la phénylalanine par l'arginine, une substitution de la leucine par l'arginine, une substitution de l'alanine par l'asparagine, une substitution de la glycine par l'alanine, une substitution de la glycine par la leucine, une substitution de la glycine par la valine, une substitution de la glycine par la tyrosine, une substitution de la glycine par l'arginine, une substitution de la glycine par le tryptophane, une substitution de l'isoleucine par la lysine, une substitution de l'isoleucine par la thréonine, une substitution de l'isoleucine par l'acide glutamique, une substitution de l'isoleucine par la valine, une substitution de la leucine par l'alanine, une substitution de la méthionine par la valine, ou une substitution de la leucine par l'alanine, ou une combinaisons de celles-ci.

   en option dans laquelle la ou les substitutions d'acides aminés est une substitution de la phénylalanine par la serine au niveau du résidu d'acide aminé 243 (F243S), une substitution de la phénylalanine par la cystéine au niveau du résidu d'acide aminé 243 (F243C), une substitution de la phénylalanine par l'acide aspartique au niveau du résidu d'acide aminé 170 (F170D), une substitution de la phénylalanine par la glycine au niveau du résidu d'acide aminé 170 (F170G), une substitution de la phénylalanine par la méthionine au niveau du résidu d'acide aminé 170 (F170M), une substitution de l'isoleucine par l'histidine au niveau du résidu d'acide aminé 322 (I322H) ou au niveau du résidu d'acide aminé 171 (I171H), une substitution de l'isoleucine par l'arginine au niveau du résidu d'acide aminé 322 (I322R) ou au niveau du résidu d'acide aminé 171 (I171R), une substitution de l'isoleucine par l'alanine au niveau du résidu d'acide aminé 322 (I322A), une substitution de la leucine par la phénylalanine au niveau du résidu d'acide aminé 326 (L326F), une substitution de la leucine par l'asparagine au niveau du résidu d'acide aminé 326 (L326N) ou au niveau du résidu d'acide aminé 262 (L262N), une substitution de la leucine par la tyrosine au niveau du résidu d'acide aminé 326 (L326Y), une substitution de la leucine par la cystéine au niveau du résidu d'acide aminé 326 (L326C), une substitution de la leucine par la glutamine au niveau du résidu d'acide aminé 326 (L326Q), une substitution de l'alanine par l'asparagine au niveau du résidu d'acide aminé 149 (A149N), une substitution de la leucine par l'alanine au niveau du résidu d'acide aminé 262 (L262A), une substitution de la méthionine par la valine au niveau du résidu d'acide aminé 269 (M269V) ou une substitution de la leucine par l'alanine au niveau du résidu d'acide aminé 326 (L326A) ou une combinaison de celles-ci.

4. La variante de lipase selon l'une quelconque des revendications précédentes, qui a été substituée dans au moins deux acides aminés correspondant à au moins deux résidus d'acides aminés 170, 171, 239, 242, 243, 254, 258, 262, 269, 322 ou 326 de SEQ ID NO : 11.

5. La variante de lipase selon l'une quelconque des revendications précédentes, formulée avec un ou plusieurs stabilisants, un ou plusieurs détergents, un ou plusieurs enzymes, un ou plusieurs surfactants, un ou plusieurs tampons, un ou plusieurs substrats enzymatiques ou une combinaison de ceux-ci.

6. La variante de lipase selon l'une quelconque des revendications précédentes, formulée avec un ou plusieurs substrats enzymatiques qui comprennent un lipide, un acide gras, un stérol, une cire, une huile, un triglycéride, un ester, une fraction d'acide carboxylique ou une combinaison de ceux-ci.

7. La variante de lipase selon l'une quelconque des revendications précédentes, sensiblement liée ou sensiblement réticulée chimiquement à une matrice, une colonne, une fibre, un filtre, une résine, un gel, une perle ou une combinaison de ceux-ci.

8. La variante de lipase selon l'une quelconque des revendications précédentes, formulée avec un ou plusieurs enzymes additionnels sélectionnés dans le groupe constitué par une ou plusieurs lipases, une ou plusieurs estérases, une ou plusieurs lyases, une ou plusieurs déprotéinases, une ou plusieurs phosphatases, une ou plusieurs déhydrogénases, une ou plusieurs transglutaminases, une ou plusieurs oxydases ou une combinaison de celles-ci.

9. Un polynucléotide isolé codant la variante de lipase selon l'une des revendications 1 à 8.

10. Un vecteur d'expression comprenant un polynucléotide isolé qui code la variante de lipase selon l'une des revendications 1 à 8.
en option dans lequel le vecteur d'expression est à codons optimisés pour l'expression dans une cellule de levure bactérienne ou non-basidiomycète.

11. Une cellule hôte microbienne transformée par le polynucléotide isolé selon la revendication 9 ou le vecteur d'expression selon la revendication 10 ;
en option dans laquelle la cellule hôte microbienne est définie comme étant une cellule hôte *E. coli* ou *P. pastoris*.

12. Un procédé pour produire une variante de lipase, comprenant (a) la culture d'une population de cellules hôtes microbiennes qui code la variante de lipase, selon l'une des revendications 1 à 8, dans des conditions propices à l'expression et à la sécrétion de la variante de lipase, et (b) la récupération de la variante de lipase exprimée de la population de cellules hôtes ou du milieu de culture.

13. Un procédé pour la réduction ou l'élimination d'un ou plusieurs composés d'acides gras *trans*-insaturés ou d'une ou plusieurs fractions d'acide gras à chaîne moyenne ou longue (≥C6) d'un substrat, comprenant la mise en contact du substrat avec une quantité efficace d'une variante de lipase selon l'une des revendications 1 à 8, pendant une durée suffisante pour hydrolyser ou estérifier au moins une partie du substrat et ainsi réduire ou éliminer un ou plusieurs des composés d'acide gras *trans*-insaturés ou une ou plusieurs fractions d'acide gras à chaîne moyenne ou longue (≥C6) du substrat ;
en option comprenant en outre le retrait de la variante de lipase de la composition après qu'un ou plusieurs des composés d'acide gras *trans*-insaturés ou qu'une ou plusieurs fractions d'acide gras à chaîne moyenne ou longue (≥C6) a été sensiblement réduit ou éliminé du substrat.

14. Le procédé selon la revendication 13, dans laquelle le substrat comprend un lipide comestible, une matière grasse comestible, un acide gras comestible, un stérol comestible, une cire comestible, une huile comestible ou un triglycéride comestible ou une de leurs combinaisons.

15. Une variante de lipase comprenant une séquence d'acides aminés d'une lipase microbienne de départ qui a été substituée dans au moins un acide aminé de SEQ ID NO : 11, dans laquelle la substitution est : F243S, F243C, F170D, F170G, F170M, F170T, F170N, F170V, I322H, I322R, I322A, T242I, T242H, L326F, L326N, L326Y, L326C, L326Q, F254R, L246R, A239N, G258A, G258V, G258Y, G258R, G258W, I171K, I171H, I171R, I171T, I171E, I171V, L262A, L262N, M269V et L326A, ou une de leurs combinaisons où les substitutions ne se chevauchent pas.

**FIG. 1A**

**FIG. 1B**

**FIG. 1C**

FIG. 2

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

Calculated percentage composition of *cis, trans* and sat lipids in released (FFA) fraction

**FIG. 17**

**FIG. 18**

**FIG. 19**

**FIG. 20**

**FIG. 21**

**FIG. 22**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61561067 A
- US 61480862 B

- WO 9401541 A
- WO 2008040738 A

**Non-patent literature cited in the description**

- **SANDSTROM et al.** *Protein engineering and Design,* 2009, vol. 22 (7), 413-420
- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410
- **ANTHONSEN, H.W. ; BAPTISTA, A. ; DRABLOS, F. ; MARTEL, P. ; PETERSEN, S.B. ; SEBASTIAO, M. ; VAZ, L.** Lipases and esterases: a review of their sequences, structure and evolution. *Biotechnol. Annu. Rev.,* 1995, vol. 1, 315-371
- **APARNA, G. ; CHATTERJEE, A. ; SONTI, R.V. ; SANKARANARAYANAN, R.** A cell wall-degrading esterase of Xanthomonas oryzae requires a unique substrate recognition module for pathogenesis on rice. *Plant Cell,* 2009, vol. 21, 1860-1873
- **BADINGS, H. T. ; DE JONG, C.** Glass capillary gas chromatography of fatty acid methyl esters. A study of conditions for the quantitative analysis of short- and long-chain fatty acids in lipids. *J. Chromatog.,* 1983, vol. 279, 493-506
- **BEER, H.D. ; MCCARTHY, J.E. ; BORNSCHEUER, U.T. ; SCHMID, R.D.** Cloning, expression, characterization and role of the leader sequence of a lipase from Rhizopus oryzae. *Biochim. Biophys. Acta,* 1998, vol. 1399, 173-180
- **BEER, H.D. ; WOHLFAHRT, G. ; SCHMID, R.D. ; MCCARTHY, J.E.** The folding and activity of the extracellular lipase of Rhizopus oryzae are modulated by a prosequence. *Biochem. J.,* 1996, vol. 319, 351-359
- **BENDTSEN, J.D. ; NIELSEN, H. ; VON HEIJNE, G. ; BRUNAK, S.** Improved prediction of signal peptides: SignalP 3.0. *J. Mol. Biol.,* 2004, vol. 340, 783-795
- **BERMAN, H.M. ; WESTBROOK, J.D. ; GABANYI, M.J. ; TAO, W. ; SHAH, R. ; KOURANOV, A. ; SCHWEDE, T. ; ARNOLD, K. ; KIEFER, F. ; BORDOLI, L. et al.** The protein structure initiative structural genomics knowledgebase. *Nucl. Acids Res.,* 2009, vol. 37, 365-368

- **BORGDORF, R. ; WARWEL, S.** Substrate selectivity of various lipases in the esterification of cis- and trans-9-octadecenoic acid. *Appl. Microbiol. Biotechnol.,* 1999, vol. 51, 480-485
- **BORNSCHEUER, U.T. ; KAZLAUSKAS, R.J.** Hydrolases in Organic Synthesis - Regio- and Stereoselective Biotransformations. Wiley-VCH, 2005
- **BRUNKE, S. ; HUBE, B.** MfLIP1, a gene encoding an extracellular lipase of the lipid-dependent fungus Malassezia furfur. *Microbiology,* 2006, vol. 152, 547-554
- **DE MARIA, P.D. ; CARBONI-OERLEMANS, C. ; TUIN, B. ; BARGEMAN, G. ; VAN DER MEER, A. ; VAN GEMERT, R.** Biotechnological applications of Candida antarctica lipase A: State-of-the-art. *J. Mol. Catal. B-Enzymat.,* 2005, vol. 37, 36-46
- **DUMON-SEIGNOVERT, L. ; CARIOT, G. ; VUILLARD, L.** The toxicity of recombinant proteins in Escherichia coli: a comparison of overexpression in BL21(DE3), C41(DE3), and C43(DE3). *Protein Expr. Purif.,* 2004, vol. 37, 203-206
- **EMANUELSSON, O. ; NIELSEN, H. ; BRUNAK, S. ; VON HEIJNE, G.** Predicting subcellular localization of proteins based on their N-terminal amino acid sequence. *J. Mol. Biol.,* 2000, vol. 300, 1005-1016
- **ERICSSON, D.J. ; KASRAYAN, A. ; JOHANSSONL, P. ; BERGFORS, T. ; SANDSTROM, A.G. ; BACKVALL, J.E. ; MOWBRAY, S.L.** X-ray structure of Candida antarctica lipase a shows A novel lid structure and a likely mode of interfacial activation. *J. Mol. Biol.,* 2008, vol. 376, 109-119
- **HANAHAN, D.** Studies on transformation of Escherichia coli with plasmids. *J. Mol. Biol.,* 1983, vol. 166, 557-580
- **HEGDE, R.S. ; BERNSTEIN, H.D.** The surprising complexity of signal sequences. *Trends Biochem. Sci.,* 2006, vol. 31, 563-571
- **HOEGH, I.S.P. ; T. HALKIER ; M.T. HANSEN.** Two lipases from Candida antarctica: Cloning and expression in Aspergillus oryzae. *Can. J. Bot.,* 1995, vol. 73 (S1), S869-S875

- **HOLM, L. ; SANDER, C.** Protein structure comparison by alignment of distance matrices. *J. Mol. Biol.,* 1993, vol. 233, 123-138
- **HOLMQUIST, M.** Alpha/Beta-hydrolase fold enzymes: structures, functions and mechanisms. *Curr. Protein Pept. Sci.,* 2000, vol. 1, 209-235
- **KAKUGAWA, K. ; SHOBAYASHI, M. ; SUZUKI, O. ; MIYAKAWA, T.** Cloning, characterization, and expression of cDNA encoding a lipase from Kurtzmanomyces sp. I-11. *Biosci. Biotechnol. Biochem.,* 2002, vol. 66, 1328-1336
- **KAKUGAWA, K. ; SHOBAYASHI, M. ; SUZUKI, O. ; MIYAKAWA, T.** Purification and characterization of a lipase from the glycolipid-producing yeast Kurtzmanomyces sp. I-11. *Biosci. Biotechnol. Biochem.,* 2002, vol. 66, 978-985
- **KASRAYAN, A. ; BOCOLA, M. ; SANDSTROM, A.G. ; LAVEN, G. ; BACKVALL, J.E.** Prediction of the Candida antarctica lipase A protein structure by comparative modeling and site-directed mutagenesis. *Chembiochem,* 2007, vol. 8, 1409-1415
- **KIRK, O. ; CHRISTENSEN, M.W.** Lipases from Candida antarctica: Unique biocatalysts from a unique origin. *Organ. Process Res. Develop.,* 2002, vol. 6, 446-451
- **KOURIST, R. ; BRUNDIEK, H. ; BOURNSCHEUER, U.T.** Protein engineering and Discovery of lipases. *Eur. J. Lipid Sci. Technol.,* 2010, vol. 112, 64-74
- **KRISHNA, S.H. ; PERSSON, M. ; BORNSCHEUER, U.T.** Enantioselective transesteri cation of a tertiary alcohol by lipase A from. *Tetrahedron,* 2002, vol. 13, 2693-2696
- **KROGH, A. ; LARSSON, B. ; VON HEIJNE, G. ; SONNHAMMER, E.L.** Predicting transmembrane protein topology with a hidden Markov model: application to complete genomes. *J. Mol. Biol.,* 2001, vol. 305, 567-580
- **LAEMMLI, U.K.** Cleavage of structural proteins during the assembly of the head of bacteriophage T4. *Nature,* 1970, vol. 227, 680-685
- **LI, X.G. ; KANERVA, L.T.** Lipases in beta-dipeptide synthesis in organic solvents. *Org. Lett.,* 2006, vol. 8 (24), 5593-5596
- **MIROUX, B. ; WALKER, J.E.** Over-production of proteins in Escherichia coli: mutant hosts that allow synthesis of some membrane proteins and globular proteins at high levels. *J. Mol. Biol.,* 1996, vol. 260, 289-298
- **OLLIS, D.L. ; CHEAH, E. ; CYGLER, M. ; DIJKSTRA, B. ; FROLOW, F. ; FRANKEN, S.M. ; HAREL, M. ; REMINGTON, S.J. ; SILMAN, I. ; SCHRAG, J.** The $\alpha/\beta$ hydrolase fold. *Protein Eng.,* 1992, vol. 5, 197-211
- **PAVELKA, A. ; CHOVANCOVA, E. ; DAMBORSKY, J.** HotSpot Wizard: a web server for identification of hot spots in protein engineering. *Nucl. Acids Res.,* 2009, vol. 37, 376-383

- **PFEFFER, J. ; RICHTER, S. ; NIEVELER, J. ; RACHID, C.-E.H. ; RHLID, B. ; SCHMID, R.D. ; RUSNAK, M.** High yield expression of Lipase A from Candida antarctica in the methylotrophic yeast Pichia pastoris and its purification and characterisation. *Appl. Microbiol.,* 2006, vol. 72 (5), 931-938
- **PFEFFER, J. ; RUSNAK, M. ; HANSEN, C.E. ; RHLID, R.B. ; SCHMID, R.D. ; MAURER, S.C.** Functional expression of lipase A from Candida antarctica in Escherichia coli - A prerequisite for high-throughput screening and directed evolution. *J. Mol. Catal. B-Enzym.,* 2007, vol. 45, 62-67
- **RANGHEARD, M.S. ; LANGRAND, G. ; TRIANTAPHYLIDES, C. ; BARATTI, J.** Multi-competitive enzymatic reactions in organic media: a simple test for the determination of lipase fatty acid specificity. *Biochim. Biophys. Acta,* 1989, vol. 1004, 20-28
- **REETZ, M.T. ; KAHAKEAW, D. ; LOHMER, R.** Addressing the numbers problem in directed evolution. *Chembiochem,* 2008, vol. 9, 1797-1804
- **SCHMIDT, M. ; BARBAYIANNI, E. ; FOTAKOPOULOU, I. ; HO, M. ; CONSTANTINOU-KOKOTOU, V. ; BORNSCHEUER, U.T. ; KOKOTOS, G.** Enzymatic removal of carboxyl protecting groups. 1. Cleavage of the tert-butyl moiety. *J. Org. Chem.,* 2005, vol. 70 (9), 3737-3740
- **SEZONOV, G. ; JOSELEAU-PETIT, D. ; D'ARI, R.** Escherichia coli physiology in Luria-Bertani broth. *J. Bacteriol.,* 2007, vol. 189, 8746-8749
- **SOUMANOU, M. M. ; BORNSCHEUER, U. T. ; MENGE, U ; SCHMID R. D.** Synthesis of structured triglycerides from peanut oil with immobilized lipase. *J. Amer. Oil Chem. Soc.,* 1997, vol. 74, 427-433
- **SOUMANOU, M. M. ; EDORH, A. P. ; BORNSCHEUER, U. T.** Activity and stability of immobilized lipases in lipase-catalyzed modification of peanut oil. *Oleagineux, Corps Gras, Lipides,* 2004, vol. 11 (6), 464-468
- **VON HEIJNE, G.** Signal sequences. The limits of variation. *J. Mol. Biol.,* 1985, vol. 184, 99-105
- **WAGNER, S. ; KLEPSCH, M.M. ; SCHLEGEL, S. ; APPEL, A. ; DRAHEIM, R. ; TARRY, M. ; HOGBOM, M. ; VAN WIJK, K.J. ; SLOTBOOM, D.J. ; PERSSON, J.O. et al.** Tuning Escherichia coli for membrane protein overexpression. *Proc. Natl. Acad. Sci. USA,* 2008, vol. 105, 14371-14376
- **WARWEL, S. ; BORGDORF, R.** Substrate selectivity of lipases in the esterification of cis/trans-isomers and positional isomers of conjugated linoleic acid (CLA). *Biotechnol. Lett.,* 2000, vol. 22, 1151-1155
- **WARWEL, S. ; BORGDORF, R. ; BRUHL, L.** Substrate selectivity of lipases in the esterification of oleic acid, linoleic acid, linolenic acid and their all-trans-isomers and in the transesterification of cis/trans-isomers of linoleic acid methyl ester. *Biotechnol. Lett.,* 1999, vol. 21, 431-436

- **WINKLER, U.K. ; STUCKMANN, M.** Glycogen, hyaluronate, and some other polysaccharides greatly enhance the formation of exolipase by Serratia marcescens. *J. Bacteriol.,* 1979, vol. 138, 663-670